(19) Europäisches Patentamt
European Patent Office
Office européen des brevets

(11) EP 2 229 378 B1

(12) # EUROPÄISCHE PATENTSCHRIFT

(45) Veröffentlichungstag und Bekanntmachung des Hinweises auf die Patenterteilung:
**10.08.2011 Patentblatt 2011/32**

(21) Anmeldenummer: **08858404.0**

(22) Anmeldetag: **12.12.2008**

(51) Int Cl.:
***C07D 401/14*** (2006.01) ***C09D 11/00*** (2006.01)
***H01L 31/042*** (2006.01)

(86) Internationale Anmeldenummer:
**PCT/EP2008/067416**

(87) Internationale Veröffentlichungsnummer:
**WO 2009/074675 (18.06.2009 Gazette 2009/25)**

(54) **FLUORESZIERENDE, HETEROZYKLISCH ANNELLIERTE PERYLENE**

FLUORESCENT, HETEROCYCLIC ANNELLATED PERYLENE

PÉRYLÈNES CONDENSÉS HÉTÉROCYCLIQUES ET FLUORESCENTS

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **12.12.2007 DE 102007059683**
**10.12.2008 DE 102008061452**

(43) Veröffentlichungstag der Anmeldung:
**22.09.2010 Patentblatt 2010/38**

(73) Patentinhaber: **BASF SE**
**67056 Ludwigshafen (DE)**

(72) Erfinder:
- **LANGHALS, Heinz**
**85521 Ottobrunn (DE)**
- **KINZEL, Simon**
**80687 München (DE)**
- **OBERMEIER, Andreas**
**85232 Oberachern (DE)**

(56) Entgegenhaltungen:
- **H. LANGHALS, OSWALD KROTZ: "Chirale bichromophore Silicone: Ordnungsprinzipien von Struktureinheiten in komplexen Molekülen" ANGEWANDTE CHEMIE, Bd. 118, Nr. 27, 2006, Seiten 4555-4558, XP002514868 ISSN: 1521-3757 (ONLINE); ISSN: 0044-8249 (PRINT) in der Anmeldung erwähnt**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 229 378 B1

## Beschreibung

Durch die Verknappung von fossilen Energieträgern und deren Kohlendioxid-Emission, die über den Treibhauseffekt zu einer globalen Erwärmung führt, besteht in der Technik ein zunehmendes Interesse an alternativen Energiequellen, deren Nutzung sich nicht negativ auf die Umwelt auswirkt. Von diesen ist die Solarenergie besonders attraktiv, weil sie für menschliche Dimensionen unbegrenzt erscheint. Das Hauptproblem ist aber andererseits die geringe Energiedichte dieser Quelle, das für die technische Nutzung ausgesprochen gravierend ist. Das Problem kann grundsätzlich durch lichtkonzentrierende Systeme gelöst werden. Wenn man dabei auch die in den gemäßigten Breiten wichtige diffuse Solarstrahlung nutzen will, werden Systeme mit einer nichtlinearen Optik benötigt. Hier ist insbesondere der Fluoreszenz-Solarkollektor [Nachr. Chem. Tech. Lab. 1980, 28, 716-718] zu nennen, der aus einer planparallelen Platte aus hoch lichtbrechendem Material besteht, die mit einem Fluoreszenzfarbstoff eingefärbt ist. Für den kurzwellig sichtbaren Bereich stehen einige Farbmittel, wie z. B. die Perylenfarbmittel (1), zur Verfügung, die mit hohen Quantenausbeuten fluoreszieren. Demgegenüber stellt der langwellig sichtbare und NIR-Bereich das größte Problem dar.

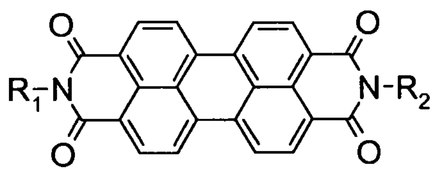

(1)

| 1 | $R_1=R_2$ |
|---|---|
| a | $CH(n\text{-}C_6H_{13})_2$ |
| b | $CH(iso\text{-}C_3H_7)_2$ |

Die Aufgabe der Erfindung war die Entwicklung eines breitbandig absorbierenden Fluoreszenzfarbmittels, der im langwellig sichtbaren Bereich fluoresziert, um ihn für Anwendungen wie Lichtsammelsystemen, beispielsweise in Fluoreszenz-Solarkollektoren, einsetzen zu können.

Die Lichtechtheit der Perylentetracarbonsäurebisimide (1) [Helv. Chim. Acta. 2005, 88, 1309-1343; Heterocycles 1995, 40, 477-500] und ihre hohen Fluoreszenzquantenausbeuten macht die Verbindungsklasse für Solaranwendungen, wie z. B. im Fluoreszenz-Solarkollektor, ausgesprochen interessant. Mit $\lambda_{max}$ von ca. 525 nm kann jedoch nur der kurzwellig sichtbare Bereich der Solarstrahlung durch (1) genutzt werden. Es gibt einen ganze Reihe von Ansätzen, die Lichtabsorption der Perylenbisimide in den längerwelligen Bereich zu verschieben. So hat z. B. der Austausch der Carbonylgruppen in (1) gegen Ketimino-Gruppen zu einer deutlichen bathochromen Verschiebung der Absorption geführt [J. Prakt. Chem. 1997, 339, 597-602; Liebigs Ann. Chem. 1995, 481- 486; Chem. Ber. 1983, 116, 3524-3528]. Nach Adachi [Pure Appl. Chem. 1996, 68, 1441- 1442] ist dabei allerdings der nach diesem Konzept erreichbare bathochrome Shift begrenzt.

$$\text{Do.- } \pi \text{ -Ac.- } \pi \text{ -Do. (a)}$$

$$\text{Ac.- } \pi \text{ -Do.- } \pi \text{ -Ac. (b)}$$

Als Alternative ist für eine langwellige Verschiebung der Perylenkern in den Positionen 1,6,7 und 12 mit Donor-Gruppen substituiert worden [Forschungsber. - Bundesminist. Forsch. Technol., Technol. Forsch. Entwickl. 1984, BMFT-FB-T 84-164; Chem. Abstr. 1985, 102, 150903; Vestn. Khar'kov. Politekh. Inst. 1969, 41, 21-26; Chem. Abstr. 1971, 75, 7375; Tetrahedron Lett. 1999, 40, 7047-7050; Eur. J. Org. Chem. 2000, 365-380] (diese Positionen werden in der Literatur häufig als ‚Bay-Region' bezeichnet - dieser Ausdruck wird hier nicht verwendet, denn er erweckt den unzutreffenden Eindruck, dass hier eine sterisch besonders leicht zugängliche Struktur vorliegt; an diesen Positionen ist aber der sterische Druck besonders groß, weil eine Häufung von Wasserstoffatomen vorliegt, die bei der üblichen Strichschreibweise der Formeln nicht zum Ausdruck kommt).

Der Chromophor der Perylenbisimide kann als inverses König'sches Farbstoff-System aufgefasst werden; im normalen Farbstoff-System nach Gleichung (a) [Prakt. Chem. 1926, 112, 1-36] sind zwei terminale Donor- Gruppen über $\pi$-Systeme mit einem zentralen Acceptor verknüpft. Bei inversen Systemen sind entsprechend Gleichung (b) Donor und Acceptorgruppen vertauscht. Bei den Perylenbisimiden bilden die Carbonylgruppen diese terminalen Acceptor-Gruppen, und die Donor-Gruppen im Zentrum fehlen [Helv. Chim. Acta. 2005, 88, 1309-1343]. Dementsprechend bewirken Donor-Gruppen in diesen Positionen eine bathochrome Verschiebung der Absorption, die bei dem Einsatz von α-Effekt-Donorgruppen [Dyes Pigm. 2003, 59, 109-116] bis in den NIR-Bereich hineinreicht. Bisher sind diesen Positionen nur fünf- und sechsgliedrige Ringe ankondensiert worden. Es ist zu fragen, in wieweit andere Ringgrößen zu Farbmitteln mit interessanten, langwellig absorbierenden Farbmitteln führen.

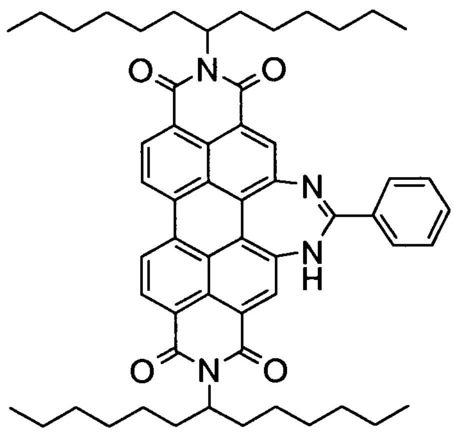

(2a)

Wir haben auf den Farbmittel (1a) [Chem. Ber. 1988, 121, 225-230] eine Mischung von Natriumamid und Benzonitril unter Luftsauerstoff bei einer Temperatur von 160˚C einwirken lassen und dabei erstaunlicherweise direkt einen Ringschluss zu einem siebengliedrigen Ring unter Bildung von (2a) erzielt, bei dem ein Siebenring mit zwei Stickstoffatomen an (1a) ankondensiert ist; siehe Abbildung 1. Bei einen Überschuss der Reagenz-Mischung erfolgt die Reaktion auch doppelt unter Bildung der Substanzen (3a). Diese Reaktion ist nicht singulär auf das Substrat (1a) und Benzonitril beschränkt, sondern ist überraschend universell. So konnte die Reaktion auch am Beispiel des komplizierter aufgebauten Ausgangsmaterials (1 b) [Angew. Chem. 2006, 118, 4555-4561; Angew. Chem. Int. Ed. Engl. 2006, 45, 4444-4447] unter Bildung von (2b) und (3b) verifiziert werden. Diverse andere aromatische Nitrile konnten umgesetzt werden, wie z.B. 4-Brombenzonitril zu (2c) und (3c), 4-Methoxybenzonitil zu (2d) und (3d), und es ist sogar die Umsetzung von Nitrilen mehrkerniger Aromaten möglich, wie die Reaktion von 2-Naphthonitril zu (2e) und (3e) zeigt. Die Reaktion kann in Substanz oder in Lösung durchgeführt werden. Allerdings muss das Solvens hinreichend stabil unter den stark alkalischen Reaktionsbedingungen sein, wie z.B. 1,2-Dimethoxyethan. Die Nitrile lassen sich überraschenderweise durch die Carbonsäureamide, wie z. B. Benzamid ersetzen - die Ausbeuten an (2) und (3) werden dadurch nicht nennenswert beeinflusst. Die starke Base Natriumamid kann durch festes Kaliumhydroxid ersetzt werden; Natriumhydrid liefert deutlich schlechtere Ergebnisse. Für den Ablauf der Reaktion ist der Zutritt von Luftsauerstoff erforderlich, denn unter Argon-Schutzatmosphäre konnte keinerlei Umsetzung nachgewiesen werden.

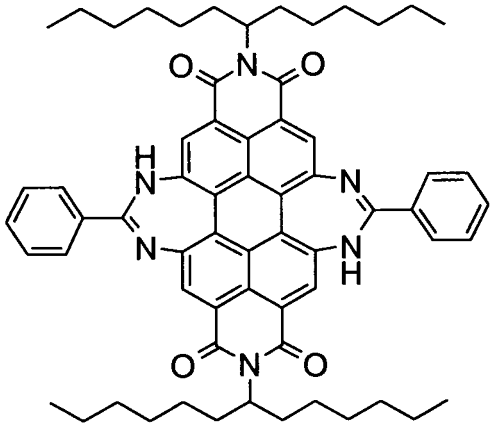

(3a)

Die Substanzen (2) und (3) enthalten jeweils an einem bzw. zwei Stickstoffatomen acide Protonen. Man kann daher fragen, ob diese Positionen nach einer Deprotonierung mit Elektrophilen umgesetzt werden können. Zwar liegen

solche Anionen auch spontan in den Protonierungsgleichgewichten vor, günstigere Ergebnisse haben wir aber nach einer Deprotonierung unter Verwendung starker Basen erwartet. Wir haben die Substanzen (2) und (3) jeweils mit Natriumhydrid als typische starke Base deprotoniert und dann mit Methyliodid als Elektrophil umgesetzt. Eine Reaktion erfolgte unter diesen Bedingungen erstaunlich glatt unter Bildung der Methylderivate.

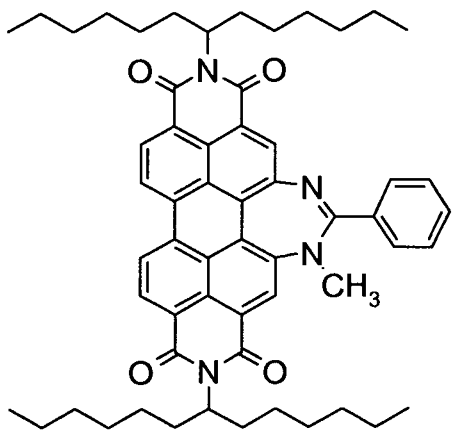

(4a)

Die UV/Vis-spektroskopischen Eigenschaften der neuen Farbmittel-Klassen sind ebenso überraschend, wie deren einfache Synthese. Die UV/Vis-Absorption von (2) ist in Abbildung 2 dargestellt - man beobachtet gegenüber (1a) eine erhebliche bathochrome Farbverschiebung. Erstaunlicher ist noch die ausgeprägte, rote Fluoreszenz der Substanz, deren Quantenausbeute nahe bei 100% liegt. Dies ist insofern bemerkenswert, da in der Literatur spekuliert wird, dass im langwellig sichtbaren Spektralbereich wegen einer Kopplung mit C-H-Schwingungsobertönen keine hohe Fluoreszenzquantenausbeuten möglich sind. Die Fluoreszenz von (2) widerlegt überraschend dieses Postulat. Die Substanz ist ausgesprochen lichtecht und eignet sich auch für Anwendungen unter direkter Einwirkung des Sonnenlichts. Die neue Substanz (2) hat gegenüber den oben erwähnten, donorsubstituierten Perylenderivaten den Vorteil, dass nicht nur eine bathochrome Verschiebung der längstwelligen Absorption erfolgt ist, sondern darüber hinaus im sichtbaren und UV-Bereich weitere Banden hinzukommen, welche der neuen heterocyclischen Struktur zuzuschreiben sind. Diese neuen Banden bewirken, dass von der längstwelligen Absorption bis in der UV-Bereich Licht absorbiert wird, so dass ein Breitband-Absorber vorliegt. Solche Breitbandabsorber sind für jede Art von Solaranwendungen von besonderem Interesse, insbesondere für Lichtsammelsysteme, die damit einen entsprechend großen Ausschnitt aus der Solarstrahlung nutzen können.

Beim neuen heterocyclischen Farbmittel (3) mit zwei siebengliedrigen Ringen tritt eine noch stärkere bathochrome Verschiebung der Absorption auf, so dass man bereits grüne Lösungen erhält; siehe Abbildung 3. Hier liegt ein extremer Breitbandabsorber vor, der kontinuierlich vom UV- bis in den NIR-Bereich Licht aufnehmen kann. Auch dieser Farbmittel fluoresziert mit einer nahe bei 100% liegenden Quantenausbeute. Da der überwiegende Teil der Fluoreszenz im langwellig sichtbaren Bereich auftritt mit einer bereits absinkenden Augenempfindlichkeit und große Teile des Spektrums im NIR liegen, erscheint die tiefrote Fluoreszenz nicht mehr so ausgeprägt, wie die von (2).

Mit starken Basen wie DBU lassen sich die Farbmittel (2) und (3) deprotonieren - dies führt zu einem überraschend starken bathochromen Shift der Absorption und der Fluoreszenz - zudem ist die Fluoreszenz erstaunlich intensiv; siehe Abbildung 4. Diese Farbmittel können dadurch als NIR-Farbmittel eingesetzt werden: Hier ist an die üblichen Anwendungen als unsichtbarer Fluoreszenzmarker oder als Kontrastmittel in der Medizin zu denken, da in diesem Bereich das Gewebe nur wenig Licht absorbiert.

Es wäre für diverse praktische Anwendungen der Farbmittel interessant, nur die Fluoreszenz von (2) oder (3) langwellig zu verschieben und die Lichtabsorption spektral zu belassen. Es ist zu fragen, ob dies durch eine schwache Base bewirkt werden kann. Wir haben deshalb den Farbmittel (2) mit der schwachen Base Piperidin gemischt und einen erstaunlich großen Stokes-Shift erzielt; siehe Abb. 5. Offensichtlich führt die optische Anregung des Farbmittels zu einer genügend großen Aciditätssteigerung der N-H-Gruppe, dass diese durch die schwache Base deprotoniert werden kann und so der erstaunlich großen Stokes-Shift auftritt. Für die Vergrößerung des Stokes-Shifts wird der ESPT-Mechanismus nach Abb. 5 verantwortlich gemacht, bei dem der Grundzustand des Farbmittels Licht absorbiert und dadurch optisch angeregt wird. Hierdurch erfolgt eine Deprotonierung des Stickstoffs unter Erhaltung der optischen Anregung. Die deprotonierte Spezies fluoresziert entsprechend langwellig verschoben, wie bei der Fluoreszenz des Anions. Der durch die Fluoreszenz erreichte Grundzustand ist dann ebenfalls deprotoniert und geht durch Protonierung wieder in den Ausgangszustand zurück; erstaunlich ist bei dem Kreisprozess, dass er auch in nichtwässrigen Medien derart effizient

bei den langen Wellenlängen erfolgt, während der erste von Förster und Weller gefundene ESPT-Prozess im kurzwelligen Spektralbereich und in wässrigem Medium erfolgte, in dem die Protonenübertragungen sehr schnell sind. Das Resultat, ein Fluoreszenzfarbmittel mit extrem großem Stokes-Shift im langwelligen Spektralbereich, ist für viele Anwendungen ausgesprochen interessant, da kein Fluoreszenzlicht wieder reabsorbiert wird, wie dies bei den konventionellen Fluoreszenzfarbmitteln erfolgt. Dies ist insbesondere für Anwendungen als Laserfarbmittel oder als Farbmittel für Fluoreszenz-Solarkollektoren wichtig.

Noch extremer und überraschender wird die Situation beim Farbmittel (3), denn hier wird die Emission bis weit in den NIR-Bereich verschoben - in dieser Eigenschaft sind die erfindungsgemässen Farbmittel bekannten Materialien überlegen.

Die Substanzen (2) und (3), insbesondere die letztere, sind für Fluoreszenz-Solarkollektoren ausgesprochen interessant, denn es liegt eine Kombination aus breitbandiger Absorption im Sichtbaren, einer starken Fluoreszenz im NIR-Bereich vor; ein Eindruck davon vermittelt ein Vergleich mit dem AM1 Sonnenspektrum nach Abbildung 6. Verwendet man einen Stapel übereinanderliegender Platten, dann kann das Solarspektrum in einzelnen Bereiche zerlegt und einzeln genutzt werden. Über den ESPT-Mechanismus lässt sich zudem ein großer Stokes-Shift erzielen, durch den Absorptions- und Fluoreszenzspektrum spektral getrennt werden, so dass eine Reabsorption des Fluoreszenzlichts im Fluoreszenz-Solarkollektor unterdrückt wird. Da die Farbmittel zudem ausgesprochen lichtecht sind, liegt ein ideales Material für den Fluoreszenz-Solarkollektor vor. Bei der Anwendung als Laserfarbmittel ist die breite Lichtabsorption von Interesse, da man in diesem Fall mit breitbandig emittiernden Lichtquellen wie Blitzlampen optisch anregen kann, da die Farbmittel durch ihre breitbandige Absorption einen großen Teil des Lichts aufnehmen können und auf den spektral schmaleren Bereich der Fluoreszenz umwandeln.

Farbmittel wie (2) oder (3) können in der Photovoltaik direkt in organischen Solarzellen eingesetzt werden oder auch in Elektrolyt-Systemen, wie z. B. der Grätzel-Zelle [Angew. Chem. 2007, 119, 8510-8514]. Schließlich sind Farbmittel des Chromophors (2) in den Fällen interessant, in denen man eine Abdunkelung wünscht, ohne die Infrarotstrahlung zu schwächen; diese Farbmittel sind deshalb hier besonders geeignet, weil sie das sichtbare Licht komplett absorbieren können, das Infrarotlicht aber nicht schwächen. Dies ist für passiv arbeitende Solarwärmesysteme von Bedeutung.

Die langwellige und breitbandige Absorption der Farbmittel des Chromophors (3) ist dann von Interesse, wenn man beispielsweise eine Tönung von Scheiben wünscht, wie eine Tönung zum Wärmeschutz gegen Lichtstrahlung. Im Gegensatz zu einer Tönung mit einer allein lichtabsorbierenden Substanz wird das absorbierte Licht nicht einfach in Wärme umgewandelt wird, sondern wieder als Fluoreszenzlicht abgestrahlt. Hierdurch wird der Absorber erheblich schwächer aufgeheizt, als wenn ein üblicher, nicht fluoreszierender Farbmittel eingesetzt würde.

Die neuen Farbmittel (2) und (3) lassen sich auch als Pigment- oder Textilfarbstoffe in konventioneller Technologie einsetzen. Hierbei ist ihre langwellige Lichtabsorption interessant, durch die besondere Farbeffekte erzielt werden können.

Die Erfindung betrifft daher folgende Gegenstände:

1. Diazepinoperylenbisimide der allgemeinen Formel

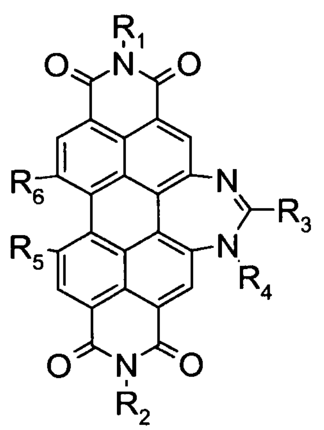

(5),

in denen die Reste $R_1$ bis $R_6$ gleich oder verschieden voneinander sein können und unabhängig voneinander Wasserstoff oder lineare Alkylreste mit mindestens einem und höchstens 37 C-Atome bedeuten, bei denen eine bis 10 $CH_2$-Einheiten unabhängig voneinander ersetzt sein können durch jeweils Carbonylgruppen, Sauerstoffatome, Schwefelatome, Selenatome, Telluratome, cis- oder trans-CH=CH-Gruppen, bei der eine CH-Einheit auch durch ein Stickstoffatom ersetzt sein kann, acetylenische C≡C-Gruppen, 1,2-, 1,3- oder 1,4-substituierten Phenylreste,

2,3-, 2,4-, 2,5-, 2,6-, 3,4- oder 3,5-disubstituierte Pyridinreste, 2,3-, 2,4-, 2,5- oder 3,4-disubstituierte Thiophenreste, 1,2-, 1,3-, 1,4-, 1,5-, 1,6-, 1,7-, 1,8-, 2,3-, 2,6- oder 2,7- disubstituierte Naphthalinreste, bei denen ein oder zwei CH-Gruppen durch Stickstoffatome ersetzt sein können, 1,2-, 1,3-, 1,4-, 1,5-, 1,6-, 1,7-, 1,8-, 1,9-, 1,10-, 2,3-, 2,6-, 2,7-, 2,9-, 2,10- oder 9,10-disubstituierte Anthracenreste, bei denen ein oder zwei CH-Gruppen durch Stickstoffatome ersetzt sein können. Bis zu 12 einzelne Wasserstoffatome der $CH_2$-Gruppen können jeweils unabhängig voneinander auch an gleichen C-Atomen ersetzt sein durch die Halogene Fluor, Chlor, Brom oder Iod oder die Cyanogruppe oder eine lineare Alkylkette mit bis zu 18 C-Atomen, bei der eine bis 6 $CH_2$-Einheiten unabhängig voneinander ersetzt sein können durch Carbonylgruppen, Sauerstoffatome, Schwefelatome, Selenatome, Telluratome, cis- oder trans-CH=CH-Gruppen, bei denen eine CH-Einheit auch durch ein Stickstoffatom ersetzt sein kann, acetylenische C≡C-Gruppen, 1,2-, 1,3- oder 1,4-substituierte Phenylreste, 2,3-, 2,4-, 2,5-, 2,6-, 3,4- oder 3,5-disubstituierte Pyridinreste, 2,3-, 2,4-, 2,5- oder 3,4-disubstituierter Thiophenreste, 1,2-, 1,3-, 1,4-, 1,5-, 1,6-, 1,7-, 1,8-, 2,3-, 2,6- oder 2,7-disubstituierte Naphthalinreste, bei dem ein oder zwei Kohlenstoffatome durch Stickstoffatome ersetzt sein 15 können, 1,2-, 1,3-, 1,4-, 1,5-, 1,6-, 1,7-, 1,8-, 1,9-, 1,10-, 2,3-, 2,6-, 2,7-, 2,9-, 2,10- oder 9,10- disubstituierte Anthracenreste, bei denen ein oder zwei Kohlenstoffatome durch Stickstoffatome ersetzt sein können. Bis zu 12 einzelne Wasserstoffatome der $CH_2$-Gruppen der Alkylreste können jeweils unabhängig voneinander auch an gleichen C-Atomen ersetzt sein durch die Halogene Fluor, Chlor, Brom oder Iod oder oder Cyanogruppen oder lineare Alkylketten mit bis zu 18 C-Atomen, bei denen eine bis 6 $CH_2$-Einheiten unabhängig voneinander ersetzt sein können durch Carbonylgruppen, Sauerstoffatome, Schwefelatome, Selenatome, Telluratome, cis- oder trans-CH=CH-Gruppen, bei der eine CH-Einheit auch durch ein Stickstoffatom ersetzt sein kann, acetylenische C≡C-Gruppen 1,2-, 1,3- oder 1,4-substituierte Phenylreste, 2,3-, 2,4-, 2,5-, 2,6-, 3,4- oder 3,5-disubstituierte Pyridinreste, 2,3-, 2,4-, 2,5- oder 3,4-disubstituierte Thiophenreste, 1,2-, 1,3-, 1,4-, 1,5-, 1,6-, 1,7-, 1,8-, 2,3-, 2,6- oder 2,7-disubstituierte Naphthalinreste, bei denen ein oder zwei Kohlenstoffatome durch Stickstoffatome ersetzt sein können, 1,2-, 1,3-, 1,4-, 1,5-, 1,6-, 1,7-, 1,8-, 1,9-, 1,10-, 2,3-, 2,6-, 2,7-, 2,9-, 2,10- oder 9,10-disubstituierte Anthracenreste, bei denen ein oder zwei Kohlenstoffatome durch Stickstoffatome ersetzt sein können. Statt Substituenten zu tragen können die freien Valenzen der Methingruppen bzw. der quartären C-Atome paarweise verknüpft werden, so dass Ringe entstehen, wie z. B. Cyclohexanringe. Die Reste $R_1$ bis $R_{16}$ können außerdem unabhängig voneinander die Halogenatome F, Cl, Br oder I bedeuten. Sind $R_1$ bis $R_6$ Kohlenwasserstoffreste, beispielsweise unsubstituierte oder substituierte, unverzweigte oder verzweigte, gegebenenfalls mono- oder polycyclische Kohlenwasserstoffreste, so enthalten diese insbesondere von 1 bis 60, bevorzugt von 1 bis 37, besonders bevorzugt von 1 bis 18 Kohlenstoffatome. Dies gilt insbesondere für $R_1$ und $R_2$. $R_3$ ist bevorzugt unsubstituiertes oder durch 1 bis 5 gleichen oder unterschiedlichen Substituenten substituiertes Phenyl. $R_4$ ist bevorzugt unsubstituiertes oder einfach oder mehrfach durch gegebenenfalls gleichen oder unterschiedlichen Substituenten substituiertes $C_1$-$C_8$Alkyl oder $C_3$-$C_8$Cyclolkyl. $R_5$ und $R_6$ sind bevorzugt H.

2. Bisdiazepinoperylenbisimide der allgemeinen Formel

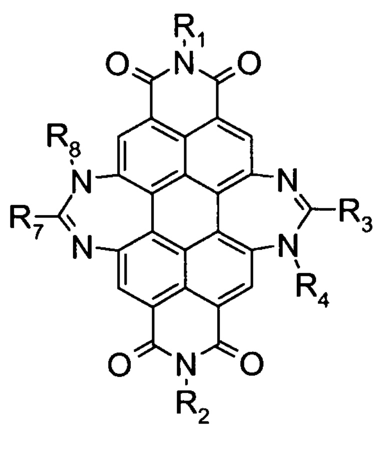 (6),

in denen alle Reste $R_1$ bis $R_8$ die gleiche Bedeutung haben, wie in Formel (5) für $R_1$ bis $R_6$ offenbart. Sind $R_1$ bis $R_8$ Kohlenwasserstoffreste, beispielsweise unsubstituierte oder substituierte, unverzweigte oder verzweigte, gegebenenfalls mono- oder polycyclische Kohlenwasserstoffreste, so enthalten diese insbesondere von 1 bis 60, bevorzugt von 1 bis 37, besonders bevorzugt von 1 bis 18 Kohlenstoffatome. Dies gilt insbesondere für $R_1$ und $R_2$. $R_3$ und $R_7$ sind bevorzugt unabhängig voneinander unsubstituiertes oder durch 1 bis 5 gleichen oder unterschiedlichen Substituenten substituiertes Phenyl. $R_4$ und $R_8$ sind bevorzugt unabhängig voneinander unsubstituiertes

oder einfach oder mehrfach durch gegebenenfalls gleichen oder unterschiedlichen Substituenten substituiertes $C_1$-$C_8$Alkyl oder $C_3$-$C_8$Cyclolkyl.

3. Verfahren, dadurch gekennzeichnet, dass als Ausgangsmaterialien für die Darstellung von (5) und/oder (6) Perylentetracarbonsäurebisimide, aromatische Nitrile und starke Basen verwendet werden. Beispiele für starke Basen sind Natriumamid, Kaliumhydroxid, Kaliumhydrid und 16 Natriumhydrid. Beispiele für aromatische Nitrile (Arylnitrile) sind Benzonitril, 1- oder 2- Naphthonitril, 4-Brombenzonitril und 4-Methoxybenzonitril.

4. Verfahren, dadurch gekennzeichnet, dass die Synthese der Verbindungen (5) oder (6) in Gegenwart von Sauerstoff erfolgt, bevorzugt Luftsauerstoff.

5. Verfahren, dadurch gekennzeichnet, dass die Reaktionspartner Perylentetracarbonsäurebisimid, Arylnitril und Base in Substanz erfolgt, bevorzugt bei erhöhter Temperatur, beispielsweise bei Temperaturen zwischen 100 und 200˚C, bevorzugt bei 160˚C; hierbei wird das Arylnitril äquimolekular oder im Überschuss eingesetzt, bevorzugt in einem zweifachen Überschuss - es können aber auch größere Überschüsse eingesetzt werden. Beispiele für sind Benzonitril , 1- oder 2-Naphthonitril, 4-Brombenzonitril und 4-Methoxybenzonitril.

6. Verfahren, dadurch gekennzeichnet, dass die Reaktionspartner unter Verwendung von Lösungsmitteln umgesetzt werden. Beispiele für Lösungsmittel sind Ethylenglycodimethylether (Glyme) oder Diethylenglycoldimethylether (Diglyme).

7. Verfahren, dadurch gekennzeichnet, dass die Farbmittel (5) mit $R_4$=H und (6) mit $R_5$ oder $R_6$ = H oder $R_5$ und $R_6$ = H zu den Farbmitteln (5) mit $R_4$=Alkyl und (6) mit $R_5$ oder $R_6$ = Alkyl oder $R_5$ und $R_6$ = Alkylreste alkyliert, bevorzugt methyliert werden. Beispiele für Alkylierungsmittel sind Alkylhalogenide mit den betreffenden Resten, wie z. B. Alkylchloride, Alkylbromide und Alkyliodide oder mono- und Dialkylsulfate wie Dimethylsulfat oder Alkyl/arylsulfonate wie Methyltosylat. Bevorzugte Medien für solche Alkylierungen sind dipolaraprotische Lösungsmittel wie DMSO, DMF, N-Methylpyrrolidon (NMP), Tetramethylharnstoff, DMPU, DMEU oder Sulfolan.

8. Verfahren, dadurch gekennzeichnet, dass die Farbmittel (5) mit $R_4$=H oder (6) mit $R_4$ oder $R_8$ = H oder $R_4$ und $R_8$ = H unter Zusatz von schwachen Basen zur Erzeugung von großen Stokes-Shifts nach dem ESPT-Mechanismus eingesetzt werden. Beispiele für schwache Basen sind Amine wie Piperidin.

9. Verwendung der Substanzen (5) oder (6) als Farbmittel, bevorzugt als Pigmente.

10. Verwendung der Substanzen (5) oder (6) als Farbmittel, bevorzugt als Pigmente für Leimfarben und verwandte Farben wie Aquarell-Farben und Wasserfarben und Farben für Tintenstrahldrucker, Papierfarben, Druckfarben, Tinten und Tuschen und andere Farben für Mal- und Schreib-Zwecke und in Anstrichstoffen.

11. Verwendung der Substanzen (5) oder (6) als Farbmittel, bevorzugt als Pigmente in Lacken. Bevorzugte Lacke sind Kunstharz Lacke wie Acryl- oder VinylHarze, Polyesterlacke, Novolacke, Nitrocellulose-Lacke (Nitrolacke) oder auch Naturstoffe wie Zaponlack, Schellack oder Qi-Lack (Japanlack bzw. Chinalack oder ostasiatischer Lack).

12. Verwendung der Farbmittel (5) oder (6) in Datenspeichern, bevorzugt in optischen Speichern. Beispiele sind Systeme wie die CD- oder DVD-Disk.

13. Verwendung der Substanzen (5) oder (6) als Fluoreszenzfarbmittel.

14. Verwendung der Substanzen (5) oder (6) in OLEDS (organischen Leuchtdioden).

15. Verwendung der Substanzen (5) oder (6) in photovoltaischen Anlagen.

16. Anwendung der Farbmittel (5) oder (6) zur Masse-Färbung von Polymeren. Beispiele sind Materialien aus Polyvinylchlorid, Polyvinylidenchlorid, Polyacrylsäure, Polyacrylamid, Polyvinylbutyral, Polyvinylpyridin, Celluloseacetat, Nitrocellulose, Polycarbonaten, Polyamiden, Polyurethanen, Polyimiden, Polybenzimidazolen, Melaminharzen, Silikonen wie Polydimethylsiloxan, Polyestern, Polyethern, Polystyrol, Polydivinylbenzol, Polyvinyltoluol, Polyvinylbenzylchlorid, Polymethylmethacrylat, Polyethylen, Polypropylen, Polyvinylacetat, Polyacrylnitril, Polyacrolein, Polybutadien, Polychlorbutadien oder Polyisopren bzw. die Copolymeren der genannten Monomeren.

17. Anwendung der Farbmittel (5) oder (6) zur Färbung von Naturstoffen. Beispiele sind Papier, Holz, Stroh, oder natürliche Fasermaterialien wie Baumwolle, Jute, Sisal, Hanf, Flachs oder und deren Umwandlungsprodukte wie z. B. die Viskosefaser, Nitratseide oder Kupferrayon (Reyon).

18. Anwendung der Farbmittel (5) oder (6) als Beizenfarbmittel, z. B. zur Färbung von Naturstoffen. Beispiele sind Papier, Holz, Stroh, oder natürliche Fasermaterialien wie Baumwolle, Jute, Sisal, Hanf, Flachs oder deren Umwandlungsprodukte wie z.B. die Viskosefaser, Nitratseide oder Kupferrayon (Reyon). Bevorzugte Salze zum beizen sind Aluminium-, Chrom- und Eisensalze.

19. Anwendung der Farbmittel (5) oder (6) als Farbmittel, z. B. zur Färbung von Farben, Lacken und anderen Anstrichsstoffen, Papierfarben, Druckfarben, Tinten und andere Farben für Mal- und Schreib-Zwecke.

20. Anwendung der Farbmittel (5) oder (6) als Pigmente in der Elektrophotographie: z. B. für Trockenkopiersysteme (Xerox-Verfahren) und Laserdrucker ("Non-Impact-Printing").

21. Anwendung der Farbmittel (5) oder (6) für Sicherheitsmarkierungs-Zwecke, wobei die große chemische und photochemische Beständigkeit und ggf. auch die Fluoreszenz der Substanzen von Bedeutung ist. Bevorzugt ist dies für Schecks, Scheckkarten, Geldscheine, Coupons, Dokumente, Ausweispapiere und dergleichen, bei denen ein besonderer, unverkennbarer Farbeindruck erzielt werden soll.

22. Anwendung der Farbmittel (5) oder (6) als Zusatz zu anderen Farben, bei denen eine bestimmte Farbnuance erzielt werden soll, bevorzugt sind besonders leuchtende Farbtöne.

23. Anwendung der Farbmittel (5) oder (6) zum Markieren von Gegenständen zum maschinellen Erkennen dieser Gegenstände über die Fluoreszenz, bevorzugt ist die maschinelle Erkennung von Gegenständen zum Sortieren, z.B. auch für das Recycling von Kunststoffen.

24. Anwendung der Farbmittel (5) oder (6) als Fluoreszenzfarbmittel für maschinenlesbare Markierungen, bevorzugt sind alphanumerische Aufdrucke oder Barcodes.

25. Anwendung der Farbmittel (5) oder (6) zur Frequenzumsetzung von Licht, z. B. um aus kurzwelligem Licht längerwelliges, sichtbares Licht zu machen.

26. Anwendung der Farbmittel (5) oder (6) in Anzeigeelementen für vielerlei Anzeige-, Hinweis- und Markierungszwecke, z. B. passive Anzeigeelemente, Hinweis- und Verkehrszeichen, wie Ampeln.

27. Anwendung der Farbmittel (5) oder (6) in Tintenstrahldruckern in homogener Lösung als fluoreszierende Tinte.

28. Anwendung der Farbmittel (5) oder (6) als Ausgangsmaterial für supraleitende organische Materialien.

29. Anwendung der Farbmittel (5) oder (6) für Feststoff-Fluoreszenz-Markierungen.

30. Anwendung der Farbmittel (5) oder (6) für dekorative Zwecke.

31. Anwendung der Farbmittel (5) oder (6) für künstlerische Zwecke.

32. Anwendung der Farbmittel (5) oder (6) zu Tracer-Zwecken, z. B. in der Biochemie, Medizin, Technik und Naturwissenschaft. Hierbei können die Farbmittel kovalent mit Substraten verknüpft sein oder über Nebenvalenzen wie Wasserstoffbrückenbindungen oder hydrophobe Wechselwirkungen (Adsorption).

33. Anwendung der Farbmittel (5) oder (6) als Fluoreszenzfarbmittel in hochempfindlichen Nachweisverfahren.

34. Anwendung der Farbmittel (5) oder (6) als Fluoreszenzfarbmittel in Szintillatoren.

35. Anwendung der Farbmittel (5) oder (6) als Farbmittel oder Fluoreszenzfarbmittel in optischen Lichtsammelsystemen.

36. Anwendung der Farbmittel (5) oder (6) als Farbmittel oder Fluoreszenzfarbmittel in Fluoreszenz-Solarkollektoren.

37. Anwendung der Farbmittel (5) oder (6) als Farbmittel oder Fluoreszenzfarbmittel in Fluoreszenz-aktivierten Displays.

38. Anwendung der Farbmittel (5) oder (6) als Farbmittel oder Fluoreszenzfarbmittel in Kaltlichtquellen zur lichtinduzierten Polymerisation zur Darstellung von Kunststoffen.

39. Anwendung der Farbmittel (5) oder (6) als Farbmittel oder Fluoreszenzfarbmittel zur Materialprüfung, z. B. bei der Herstellung von Halbleiterschaltungen.

40. Anwendung der Farbmittel (5) oder (6) als Farbmittel oder Fluoreszenzfarbmittel zur Untersuchung von Mikrostrukturen von integrierten Halbleiterbauteilen.

41. Anwendung der Farbmittel (5) oder (6) als Farbmittel oder Fluoreszenzfarbmittel in Photoleitern.

42. Anwendung der Farbmittel (5) oder (6) als Farbmittel oder Fluoreszenzfarbmittel in fotografischen Verfahren.

43. Anwendung der Farbmittel (5) oder (6) als Farbmittel oder Fluoreszenzfarbmittel in Anzeige-, Beleuchtungs- oder Bildwandlersystemen, bei denen die Anregung durch Elektronen, Ionen oder UV-Strahlung erfolgt, z. B. in Fluoreszenzanzeigen, Braunschen Röhren oder in Leuchtstoffröhren.

44. Anwendung der Farbmittel (5) oder (6) als Farbmittel oder Fluoreszenzfarbmittel als Teil einer integrierten Halbleiterschaltung, die Farbmittel als solche oder in Verbindung mit anderen Halbleitern z. B. in Form einer Epitaxie.

45. Anwendung der Farbmittel (5) oder (6) als Farbmittel oder Fluoreszenzfarbmittel in Chemilumineszenzsystemen, z. B. in Chemilumineszenz-Leuchtstäben, in Lumineszenzimmunoassays oder anderen Lumineszenznachweisverfahren.

46. Anwendung der Farbmittel (5) oder (6) als Farbmittel oder Fluoreszenzfarbmittel als Signalfarben, bevorzugt zum optischen Hervorheben von Schriftzügen und Zeichnungen oder anderen graphischen Produkten, zum Kennzeichnen von Schildern und anderen Gegenständen, bei denen ein besonderer optischer Farbeindruck erreicht werden soll.

47. Anwendung der Farbmittel (5) oder (6) als Farbmittel oder Fluoreszenzfarbmittel in Farbstoff-Lasern, bevorzugt als Fluoreszenzfarbmittel zur Erzeugung von Laserstrahlen.

48. Anwendung der Farbmittel (5) oder (6) als Farbmittel in Farbstoff-Lasern als Q-Switch Schalter.

49. Anwendung der Farbmittel (5) oder (6) als aktive Substanzen für eine nichtlineare Optik, z. B. für die Frequenzverdopplung und die Frequenzverdreifachung von Laserlicht.

50. Anwendung der Farbmittel (5) oder (6) als Rheologieverbesserer.

51. Anwendung der Farbmittel (5) oder (6) zur Dichtigkeitsprüfung geschlossener Systeme.

52. Verfahren zur Anregung von Fluoreszenz im Bereich von 500 bis 1000 nm, dadurch gekennzeichnet, dass ein Farbmittel (5) oder (6) mit einer elektromagnetischen Strahlung der Wellenlänge von 250 bis 600 nm, bevorzugt mit sichtbares Licht der Wellenlänge von 400 bis 600 nm, bestrahlt wird. Die dadurch erzeugte Fluoreszenz kann beispielsweise zur Erzeugung von Strom oder Wärme, oder zur Vollziehung einer chemischen Reaktion verwendet werden.

53. Verfahren zur Detektion von Fluoreszenz im Bereich von 500 bis 1000 nm, dadurch gekennzeichnet, dass die Fluoreszenz durch Bestrahlung eines Farbmittels (5) oder (6) mit einer elektromagnetischen Strahlung der Wellenlänge von 250 bis 600 nm, bevorzugt sichtbares Licht der Wellenlänge von 400 bis 600 nm, angeregt wird. Die detektierte Fluoreszenz kann teilweise oder ganz aufgefangen werden und in analoge oder digitale Signale oder in Energie umgewandelt werden.
Die zuvor unter den Punkten 3. bis 53. offenbarten Verfahren und Verwendungen gelten auch für die hiernach in Punkten 54. bis 60. offenbarten weiteren isomeren Strukturen, welche auf gleiche Art und Weise zugänglich sind.

54. Imidazoloperylenbisimide der allgemeinen Formel

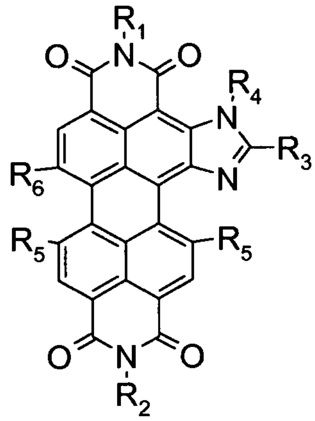

,

worin die Reste $R_1$ bis $R_6$ die für Formel (5) oder (6) genannte Bedeutung haben.

55. Imidazoloperylenbisimide der allgemeinen Formel

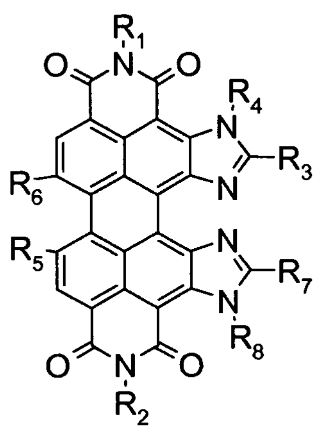

,

worin die Reste $R_1$ bis $R_8$ die für Formel (5) oder (6) genannte Bedeutung haben.

56. Imidazoloperylenbisimide der allgemeinen Formel

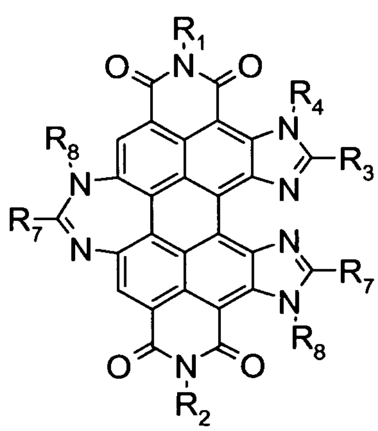

,

worin die Reste $R_1$ bis $R_5$ die für Formel (5) oder (6) genannte Bedeutung haben.

57. Imidazoloperylenbisimide der allgemeinen Formel

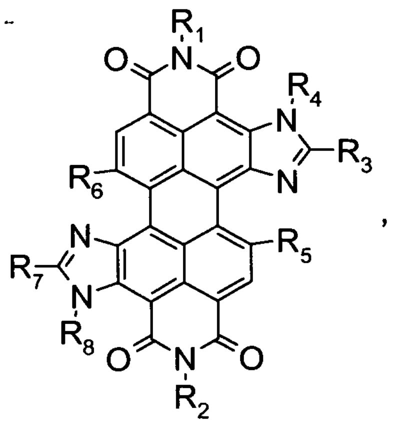

,

worin die Reste $R_1$ bis $R_8$ die für Formel (5) oder (6) genannte Bedeutung haben.

58. Imidazoloperylenbisimide der allgemeinen Formel

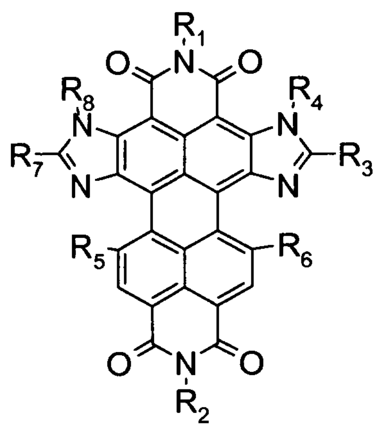

,

worin die Reste $R_1$ bis $R_8$ die für Formel (5) oder (6) genannte Bedeutung haben.

59. Imidazoloperylenbisimide der allgemeinen Formel

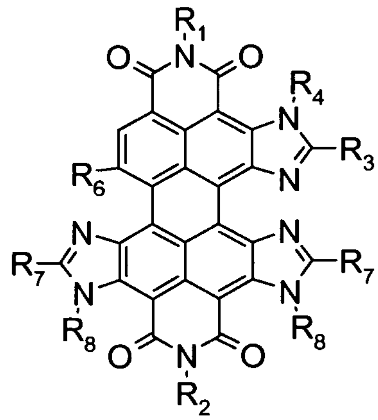

,

worin die Reste $R_1$ bis $R_8$ die für Formel (5) oder (6) genannte Bedeutung haben.60. Imidazoloperylenbisimide der allgemeinen
Formel

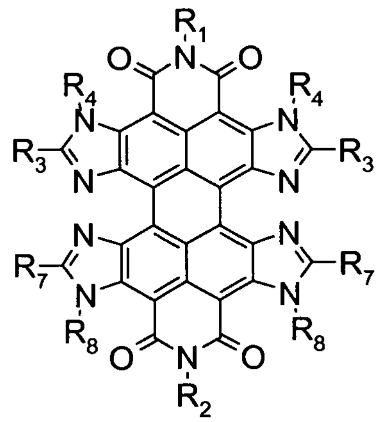

,

worin die Reste $R_1$ bis $R_8$ die für Formel (5) oder (6) genannte Bedeutung haben.

Abb. 1 zeigt das Syntheseschema der Diazepinoperylentetracarbonsäure-bisimide.

Abb. 2 zeigt die UV/Vis Absorptions- (dicke Linie, links) und Fluoreszenzspektren (dicke Linie rechts) von (2a) in Chloroform im Vergleich zum Absorptionsspektrum von (1a) (dünne Linie).

Abb. 3 zeigt die UV/Vis Absorptions- (dicke Linie, links) und Fluoreszenzspektren (dicke Linie rechts) von (3a) in Chloroform im Vergleich zum Absorptionsspektrum von (1a) (dünne Linie).

Abb. 4 zeigt die UV/Vis Absorptions- (dicke Linie, links) und Fluoreszenzspektren (dicke Linie rechts) von (2a) in Chloroform unter Zusatz von DBU im Vergleich zum Absorptionsspektrum von (1a) (dünne Linie).

Abb. 5 zeigt den ESPT-Mechanismus von 2a mit dem Absorptionsspektrum (dicke Linie links), dem Fluoreszentspektrum (dicke Linie rechts) und dem Fluoreszenzanregungsspektrum von (2a) bei 694 nm in Chloroform/Piperidin 3,1:1.

Abb. 6 zeigt das Breitband-Absorptions- und Fluoreszenzspektrum des Farbmittels gemäss Beispiel 25 in Chloroform.

Abb. 7 zeigt eine Übersicht über die UV/Vis-Absorptionsspektren in Chloroform. Die Maxima entsprechen von links nach rechts der Verbindung (1a), der Verbindung gemäss Beispiel 18, der Verbindung gemäss Beispiel 22, der

Verbindung gemäss Beispiel 25, der deprotonierten Form der Verbindung gemäss Beispiel 18 und dem Fluoreszenzspektrum der deprotonierten Form der Verbindung gemäss Beispiel 18 im Vergleich zum AM1 Sonennspektrum (verrauschte obere Linie).

Im vorangegangenen Text wurden hauptsächlich nur die Strukturen (1) bis (6) diskutiert. Es ist jedoch zu erwähnen, dass die Strukturen der erfindungsgemässen Farbmittel nicht mit absoluter Sicherheit geklärt werden konnten. Strukturen (2) bis (6) widerspiegeln daher nur eine von mehreren möglichen Interpretationen der experimentellen analytischen Daten. Die heute zur Verfügung stehenden Methoden erlauben noch keine vollständig zufriedenstellende Zuordnung der Struktur. Gesichert sind jedoch deren Teilstrukturen, die wie folgt dargestellt werden können:

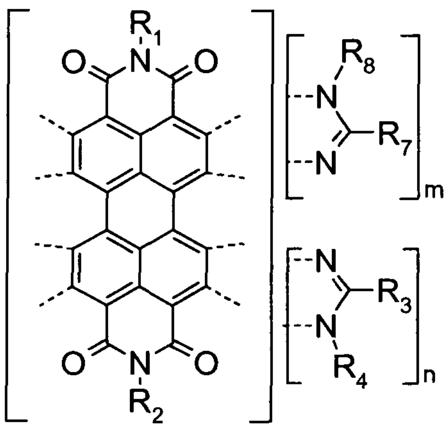 (X).

In Formel (X) sind m gleich 0, 1 oder 2 und n gleich 1 oder 2. Die Substituenten sind selbstverständlich die gleichen, wie zuvor offenbart. Deren genaue Definition ist nochmals in Anspruch 1 offenbart.

Aus den nachfolgenden Beispielen ergeben sich aber auch deutliche Hinweise auf folgende zusätzliche Strukturen, gegebenenfalls inklusive deren Isomere bezüglich der genauen Position von $R_4$ und/oder $R_8$ an den zwei N-Atomen der Imidazol-Ringe (beziehungsweise Tautomere, wenn $R_4$ und/oder $R_8$ gleich H sind):

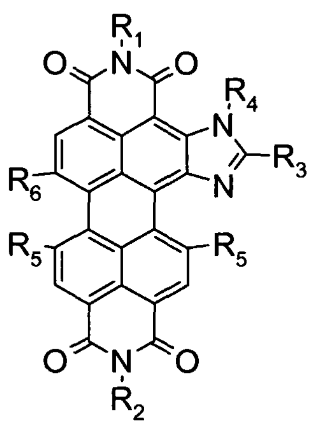 (7), 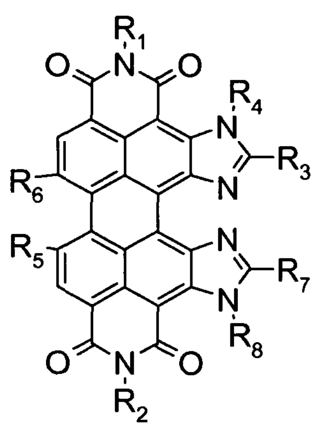 (8), 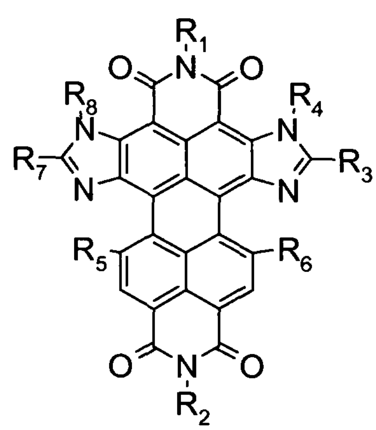 (9),

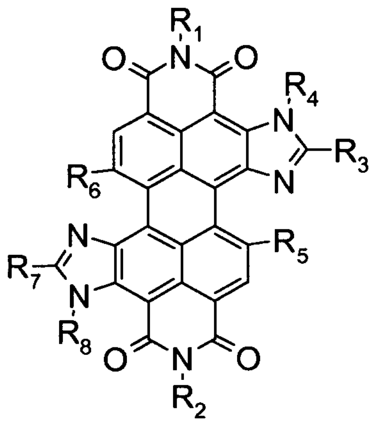 (10), 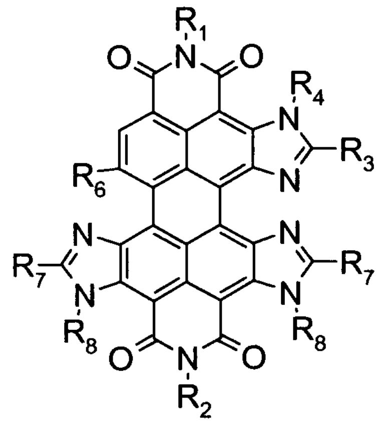 (11),

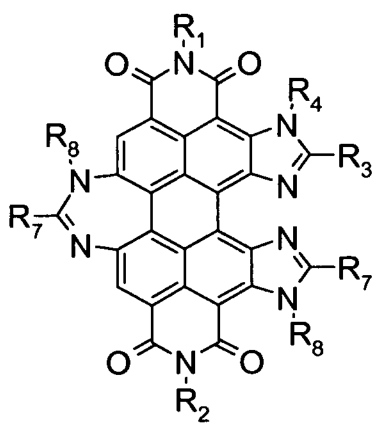 (12) oder 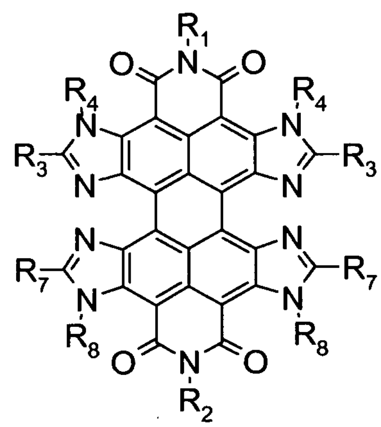 (13).

Die erfindungsgemässen Verbindungen können alternativ auch durch die Kondensation eines Amid-substituierten Perylendiimids in Gegenwart einer starken Base (wie zuvor, beispielsweise Natriumamid), gegebenenfalls in Gegenwart eines Lösungsmittels, erhalten werden.

Die nachfolgenden Beispiele erläutern die Erfindung, ohne deren Umfang einzuschränken (wo nicht anders angegeben, handelt es sich bei "%" immer um Gewichts-%):

<u>Allgemeines:</u>

IR-Spektren: Perkin Elmer 1420 Ratio Recording Infrared Spektrometer, FT 1000; UV/Vis-Spektren: Varian Cary 5000 und Bruins Omega 20; Fluoreszenzspektren: Perkin Elmer FS 3000 (totalkorrigiert); NMR-Spektroskopie: Varian Vnmrs 600 (600 MHz); Massenspektrometrie: Finnigan MAT 95.

<u>Beispiel</u> 1: 2,10-Bis(1-hexylheptyl)-6-phenyl[1,3]diazepino[4',5',6':4,5]phenanthro-[2,1,10-*def*::7,8,9-*d'e'f'*]diisochinolin-1,3,9,11(2*H*,5*H*,9*H*,11*H*)-tetraon (2a) und 2,9-Bis(1-hexylheptyl)-bis-[1,3]diazepino[4',5',6':1,12;4",5",6":6,7]perylo[3,4-*cd*:9,10-*c'd'*]dipyridin-1,3,9,11(2*H*,5*H*,10*H*,13*H*)-tetraon (3a). *N,N'*-Bis(1-hexylheptyl)-perylen-3,4:9,10-tetracarbonsäure-3,4:9,10-bisimid ((1a), 1,00 g, 1,32 mMol) und $NaNH_2$(1,00 g, 25,6 mMol) werden in Benzonitril (250 mL) suspendiert, 3 h auf 165°C erhitzt (Blaufärbung), abkühlen lassen und mit einem 1:1-Gemisch aus 2N-HCl und $CHCl_3$ (300 mL) ausgeschüttelt. Die organische Phase wurde getrocknet ($MgSO_4$), im Vakuum bis zur Trockene eingedampft (entfernen von überschüssigem Benzonitril), in $CHCl_3$ aufgenommen, filtriert und säulenchromatographisch (Kieselgel, $CHCl_3$) gereinigt. Es werden ein Einfach-Addukt und ein Doppeladdukt von Benzonitril erhalten. 1. Fraktion: 2,9-Bis(1-hexylheptyl)- bis-[1,3]diazepino[4',5',6':1,12;4",5",6":6,7]perylo[3,4-*cd*:9,10-*c'd'*]dipyridin-1,3,9,11(2*H*,5*H*,10*H*,13*H*)-tetraon (3a). Ausb.: 40 mg (4%) schwarzer Farbmittel; Schmp. >300°C; $R_f$(Kieselgel, Chloroform) = 0,88; IR (ATR): $\tilde{\nu}$ = 3316,7 m, 2950,3 m, 2920,1 s, 2852,0 m, 1672,9 s, 1616,9 s, 1586,9 s, 1532,3 w, 1487,0 w, 1453,1 w, 1436,5 w, 1401,4 w, 1339,6 m, 1332,0 s, 1299,4 w, 1267,0 w, 1232,4 w, 1184,3 w, 1109,1 w, 1058,6 m, 1000,6 m, 955,5w, 895,7 w, 867,9 w, 815,0 w, 779,6 w, 758,2 m, 731,6 w, 687,6 w $cm^{-1}$; $^1$H-NMR (600 MHz, $CDCl_3$, 25°C), δ = 0,82-0,89 (m, 12 H, $CH_3$), 1,26-1,47 (m, 32 H, $CH_2$), 1,91-2,06 (dm, 4 H, β-$CH_2$), 2,28-2,43 (m, 4 H, β-$CH_2$), 5,22-5,36 (m, 2 H, CH-N), 7,65-7,67 (m, 3 H, $CH_{aryl}$), 8,33-8,38 (m, 2 H, $CH_{aryl}$), 8,72-8,85 (m, 6 H, $H_{pery}$), 10,95 (d, 1 H, $^3J$= 8,0Hz), 11,55 ppm (s, 1 H, N-

H); $^{13}$C-NMR (151 MHz, $CDCl_3$, 25˚C): δ = 14,1, 22,6, 27,0, 29,3, 31,8, 32,4, 54,6, 104,8, 121,2, 122,7, 123,5, 126,5, 126,6, 127,7, 128,1, 129,1, 129,4, 130,4, 130,6, 131,1, 132,2, 134,7, 134,9, 135,2, 135,2, 138,7, 39,0, 139,2, 143,9, 157,3, 163,9, 164,9, 165,7 ppm; UV/Vis ($CHCl_3$): $\lambda_{max}$ ($E_{rel}$) = 388,6 (0,07), 413,0 (0,12), 452,6 (0,14), 481,0 (0,15), 514,4 (0,14), 547,2 (0,11), 589,6 (0,42), 640,6 (1,00); Fluoreszenz ($CHCl_3$): $\lambda_{max}$ ($I_{rel}$) = 650,8 (1,00), 712,3 nm, (0,32), Fluoreszenzquantenausbeute ($CHCl_3$, $\lambda_{exc}$= 472 nm, $E_{472nm}$= 0,149 $cm^{-1}$, Referenz: S13 mit 1,00): 1,00; MS ($DEI^+$/ 70 eV): *m/z* (%) = 990 (25) [$M^+$+ 4H], 989 (63) [$M^+$+ 3H], 988 (91) [$M^+$+ 4H], 806 (19), 805 (20), 624 (21), 623 (66), 622 (100), 55 (11); HMRS ($C_{64}H_{71}N_6O_4$): Ber, *m/z:* 987,554; Gef, *m/z:* 987,552, Δ = -2 mmu. 2. Fraktion: Ausb. 409 mg (36 %) 2,10-Bis(1-hexylheptyl)-6- phenyl[1,3]diazepino-[4',5',6':4,5]phenanthro[2,1,10-*def*::7,8,9-*d'e'f'*]diisochinolin-1,3,9,11 (2*H*,5*H*,9*H*,-11*H*)-tetraon (2a) als metallisch glänzender, violetter Farbmittel, Schmp. >300˚C, $R_f$ (Kieselgel, Chloroform) = 0,85, IR (ATR): ṽ = 3411,7 m, 2954,8 m, 2923,5 s, 2855,2 m, 1689,1 s, 1656,0 s, 1640,3 s, 1623,0 s, 1591,7 s, 1534,8 w, 1486,6 w, 1455,4 w, 1432,6 w, 1396,1 w, 1375,6 w, 1342,4 s, 1332,0 s, 1305,6 m, 1257,4 m, 1219,9 w, 1179,1 w, 1090,9 m, 1054,9 m, 1016,0 m, 871,0 w, 807,6 m, 796,5 m, 748,1 m, 727,0 w, 684,0 w $cm^{-1}$; $^1$H -NMR (600 MHz, $CDCl_3$, 25˚C): δ = 0,81-0,85 (m, 12H, $CH_3$), 1,23-1,40 (m, 32H, $CH_2$), 1,86-1,97 (m, 4H, β-$CH_2$), 2,24-2,37 (m, 4H, β-$CH_2$), 5,18- 5,30 (m, 2H, CH-N), 7,67-7,69 (m, 3H, $CH_{aryl}$), 8,33-8,35 (m, 2H, $CH_{aryl}$), 8,56-8,77 (m, 6H, $H_{pery}$), 10,74 (d, 1H, $^3J$= 8,2Hz), 11,52 ppm (s, 1H, N-H), $^{13}$C-NMR (151 MHz, $CDCl_3$, 25˚C): δ= 14,1, 22,6, 27,0, 29,3, 31,8, 32,4, 54,6, 104,8, 121,2, 122,7, 123,5, 126,5, 126,6, 127,7, 128,1, 129,1, 129,4, 130,4, 130,6, 131,1, 132,2, 134,7, 134,9, 135,2, 135,2, 138,7, 39,0, 139,2, 143,9, 157,3, 163,9, 164,9, 165,7 ppm; UV/Vis ($CHCl_3$): $\lambda_{max}$ ($E_{rel}$) = 378,6 (9020), 396,9 (9210), 439,4 (13400), 463,6 (14700), 504,6 (15900), 541,9 (48100), 586,6 nm (92000); Fluoreszenz ($CHCl_3$): $\lambda_{max}$ ($I_{rel}$) = 599,3 (1,00), 651,5 (0,43); Fluoreszenzquantenausbeute ($CHCl_3$, $\lambda_{exc}$= 541 nm, $E_{541nm}$ = 0,322 $cm^{-1}$, Referenz: (1a) mit Φ = 1,00): 1,00; HMRS ($C_{57}H_{67}N_4O_4$): Ber, *m/z:* 871,516; Gef, *m/z:* 871,517; Δ = 1 mmu; $C_{57}H_{67}N_4O_4$ (871,2): ber. C 78,59 %, H 7,64, N 6,43 %; gef. C 78,49, H 7,78, N 6,55 %.

Beispiel 2: 2,10-Bis(1-hexylheptyl)-6-(2-naphthyl)[1,3]diazepino[4',5',6':4,5]-phenanthro[2,1,1 0-*def*:: 7,8,9-*d'e'f'*]diisochinolin-1,3,9,11 (2*H*,5*H*,9*H*,11*H*)-tetraon (2e). *N*,*N'*-Bis(1-hexylheptyl)-perylen-3,4:9,10-tetracarbonsäure-3,4:9,10-bisimid ((1a), 200 mg, 0,264 mMol), $NaNH_2$(1,00 g, 5,12 mMol) und 2-Naphthonitril (8 g) wurden wie im Beispiel 1 umgesetzt und aufgearbeitet. Ausbeute: 54 mg (22 %) violetter Farbmittel, Schmp. >300˚C; $R_f$(Kieselgel, Chloroform) = 0,90; IR (ATR): ṽ = 3409,1 m, 2953,2 m, 2922,7 s, 2853,1 m, 1690,6 s, 1655,7 s, 1640,2 s, 1624,2 s, 1607,6 w, 1591,4 s, 1527,4 w, 1507,9 w, 1458,7 w, 1432,3 w, 1411,5 w, 1379,2 w, 1343,4 s, 1343,4 m, 1248,2 m, 1220,4 w, 1179,01 10 w, 1120,0 wm, 974,1 m, 872,0 w, 852,3 m, 810,3 m, 749,3 m, 727,9 w, 632,3 w, 581,2 w $cm^{-1}$; $^1$HNMR (300 MHz, $CDCl_3$, 25˚C): δ = 0,81-0,87 (m, 12H, $CH_3$), 1,27-1,50 (m, 32H, $CH_2$), 1,89-2,04 (m, 4H, β-$CH_2$), 2,26-2,44 (m, 4H, β-$CH_2$), 5,20-5,34 (m, 2H, CH-N), 7,52-7,64 (m, 2H, $CH_{aryl}$), 7,81-8,00 (m, 3H, $CH_{aryl}$), 8,32-8,37 (m, 2H,$CH_{aryl}$), 8,46-8,72 (m, 6H, $H_{pery}$), 10,61 (d, 1H, $^3J$=8,2Hz), 11,54 ppm (s, 1H, N-H); $^{13}$C-NMR (151 MHz, $CDCl_3$, 25˚C): δ = 14,1, 22,6, 27,0, 29,3, 31,8, 32,5, 54,6, 120,9, 122,2, 122,4, 123,0, 123,3, 124,0, 124,8, 125,2 126,4, 126,4, 127,2, 127,6, 128,0 128,3, 128,9, 129,0, 129,2, 130,1, 130,4, 130,8, 139,9, 132,9, 134,5, 134,6, 134,62, 139,1, 143,8, 157,1, 163,7,164,7, 164,8 ppm; UV/Vis ($CHCl_3$): $\lambda_{max}$ ($E_{rel}$) = 384,4 (0,14), 451,8 (0,20), 476,6 (0,23), 507,5 (0,19), 545,6 (0,54), 591,2 (1,00); Fluoreszenz ($CHCl_3$): $\lambda_{max}$ ($I_{rel}$) = 603,0 (1,00), 655,5 (0,46); Fluoreszenzquantenausbeute ($CHCl_3$, $\lambda_{exc}$ = 472 nm, $E_{472\ nm}$ = 0,159 $cm^{-1}$, Referenz: (1a) mit Φ = 1,00): 1,00; HMRS ($C_{61}H_{68}N_4O_4$): ber. *m/z:* 920,524, gef. *m/z* 920,522; Δ = -0,002.

Beispiel 3: 2,10-Bis(1-hexylheptyl)-6-(4-bromphenyl)[1,3]diazepino[4',5',6':4,5]-phenanthro[2,1,10-*def*:: 7,8,9-*d'e'f'*]diisochinolin-1,3,9,11(2*H*,5*H*,9*H*,11*H*)-tetraon (2c). *N*,*N'*-Bis(1-hexylheptyl)-perylen-3,4:9,10-tetracarbonsäure-3,4:9,10-bisimid ((1a), 100 mg, 0,264 mMol), $NaNH_2$(1,00 g, 2,56 mmol) und 4-Brombenzonitril (10 g) wurden wie im Beispiel 1 umgesetzt und aufgearbeitet. Ausbeute 97 mg (77 %) violetter Farbmittel; Schmp. >300˚C; $R_f$(Kieselgel, Chloroform) = 0,80, IR (ATR): ṽ = 3407,7 m, 2952,5 m, 2923,3 s, 2854,8 m, 1690,2 s, 1641,2 s, 1623,1 s, 1591,6 s, 1531,7 w, 1481,0 w, 1467,0 w, 1434,22,6 w, 1411,3 w, 1387,0 w, 1343,7 s, 1331,0 s, 1255,4 m, 1219,6 w, 1179,8 w, 1119,8 w, 1071,9 w, 1008,7 m, 871,3 w, 809,0 m, 772,0 w, 748,5 m, 724,1 w, 596,2 w $cm^{-1}$; $^1$HNMR (600 MHz, $CDCl_3$, 25˚C): δ = 0,81-0,89 (m, 12H, $CH_3$), 1,22-1,39 (m, 32H, $CH_2$), 1,87-1,97 (m, 4H, β-$CH_2$), 2,25-2,36 (m, 4H, β-$CH_2$), 5,18-5,31 (m, 2H, CH-N), 7,81-7,83 (m, 2H, $CH_{aryl}$), 8,23-8,25 (m, 2H, $CH_{aryl}$), 8,65-8,84 (m, 6H, $H_{pery}$), 10,78 (d, 1H, $^3J$=8,2Hz), 11,52 ppm (s, 1H, N-H); $^{13}$C-NMR (151 MHz, $CDCl_3$, 25˚C): δ = 14,1, 22,6, 27,0, 29,3, 31,8, 32,4, 54,6, 121,4, 122,8, 123,7, 126,7, 126,7, 127,0, 127,2, 129,1, 129,2, 132,8, 143,9 ppm; UV/Vis ($CHCl_3$): $\lambda_{max}$ (ε) = 378,9 (9540), 399,0 (8870), 441,3 (14100), 465,0 (15400), 505,8 (15000), 542,9 (46600), 587,5 (89400); Fluoreszenz ($CHCl_3$): $\lambda_{max}$ ($I_{rel}$) = 599,8 (1,00), 653,5 (0,42) ; Fluoreszenzquantenausbeute ($CHCl_3$, $\lambda_{exc}$ = 495 nm, $E_{495nm}$ = 0,053 $cm^{-1}$, Referenz: (1 a) mit Φ = 1,00): 1,00, HMRS ($C_{57}H_{65}BrN_4O_4$): ber. *m/z* 950,431, gef. *m/z*: 950,429, Δ = -2 mmu; $C_{57}H_{65}BrN_4O_4$ (950,1): ber. C 72,06, H 6,90, N 5,90; gef. C 71,79, H 6,73, N 5,88.

Beispiel 4: 10-Bis[1-(1-methylethyl)-2-methylpropyl]-6- phenyl[1,3]diazepino[4',5'-,6':4,5]phenanthro [2,1,10-*def*::7,8,9-*d'e'f'*]diisochinolin-1,3,9,11(2*H*,5*H*,9*H*,11*H*)-tetraon (2b). *N*,*N'*-Bis[1-(1-methylethyl)-2-methylpropyl] perylen-3,4:9,10-tetracarbonsäure-3,4:9,10-bisimid ((1b), 1,30 g, 1,85 mMol), $NaNH_2$(1,30 g, 33,3 mMol) und Benzonitril (250 g) wurden wie im Beispiel 1 umgesetzt und aufgearbeitet. Ausbeute 670 mg (43%) metallisch glänzender, violetter Farbmittel; Schmp. >300˚C; $R_f$(Kieselgel, Chloroform) = 0,25; IR (ATR): ṽ = 3407,6 m, 2962,1 m, 2923,5 m, 2872,3 m, 1698,7 m, 1683,5 m, 1659,4 m, 1639,5 s, 1628,5 s, 1608,2 m, 1591,9 s, 1566,2 w, 1532,8 w, 1487,6 w, 1457,0 w, 1433,1

w, 1411,6 w, 1382,6 w, 1332,0 s, 1306,2 m, 1252,1 m, 1212,4 w, 1167,3 w, 1123,5 w, 1100,3 w, 1052,2 w,924,4 w, 904,0 w, 872,0 w, 844,1 w, 811,9 m, 775,6 w, 751,3 m, 684,0 w, 585,4 w $cm^{-1}$; $^1$HNMR (600 MHz, $CDCl_3$, 25˚C): δ = 0,97-1,01 (m, 12 H, $CH_3$), 1,14-1,18 (m, 12 H, $CH_3$), 2,72-2,83 (m, 4 H, β-CH), 4,77-4,88 (m, 2 H, CH-N), 7,67-7,69 (m, 3 H, $CH_{aryl}$), 8,36-8,38 (m, 2 H, $CH_{aryl}$), 8,62-8,84 (m, 6 H, $H_{pery}$), 10,79-10,83 (m, 1 H, $CH_{pery}$), 11,57-11,59 ppm (m, 1 H, N-H) ; $^{13}$C-NMR (151 MHz, $CDCl_3$, 25˚C): δ = 20,7, 21,9, 22,0, 29,2, 29,4, 65,0, 65,1, 121,2, 121,3, 122,3, 122,4, 122,7, 122,8, 123,0, 123,1, 123,6, 125,1, 125,2, 125,3, 125,4, 126,6, 126,7, 127,7, 127,8, 128,2, 129,2, 129,4, 129,5, 130,6, 130,7, 131,3, 131,9, 132,2, 132,6, 134,8, 134,9,135,0, 135,3, 139,1, 139,4, 144,0, 157,4, 164,2, 164,4, 164,5, 165,4, 165,5, 166,3 ppm; UV/Vis ($CHCl_3$): $\lambda_{max}(E_{rel})$ = 379,7 (0,10), 397,5 (0,10), 439,1 (0,15), 463,5 (0,17), 504,4 (0,19), 542,2 (0,52), 586,7 (1,00); Fluoreszenz ($CHCl_3$): $\lambda_{max}$ ($I_{rel}$) = 599,0 (1,00), 650,3 (0,43); Fluoreszenzquantenausbeute ($CHCl_3$, $\lambda_{exc}$ = 495 nm, $E_{495\ nm}$ = 0,053 $cm^{-1}$, Referenz: (1a) mit Φ = 1,00): 1,00; HMRS ($C_{45}H_{42}N_4O_4$): ber. *m*/*z*: 702,320, gef. *m*/*z*: 702,319, Δ = -0,001; $C_{45}H_{42}N_4O_4$ (702,8): ber. C 76,90, H 6,02, N 7,97; gef. C 76,55, H 5,91, N 7,94.

Beispiel 5: 2,10-Bis(1-hexylheptyl)-6-(4-methoxyphenyl)[1,3]diazepino[4',5',6':4,5]-phenanthro[2,1,10-*def*::7,8,9-*d'e'f'*]diisochinolin-1,3,9,11(2*H*,5*H*,9*H*,11*H*)-tetraon (2d). *N*,*N*'-Bis(1-hexylheptyl)-perylen-3,4:9,10-tetracarbonsäure-3,4:9,10-bisimid ((1a), 500 mg, 0,660 mMol), $NaNH_2$(500 mg, 12,8 mMol) und 4-Methoxybenzonitril (20 g) wurden wie im Beispiel 1 umgesetzt und aufgearbeitet. Ausbeute 137 mg (23%) metallisch glänzender, violetter Farbmittel; Schmp. >300˚C; $R_f$(Kieselgel, Chloroform) = 0,80; IR (ATR): $\tilde{\nu}$ = 3411,8 m, 2951,8 m, 2921,6 s, 2853,3 m, 1687,4 s, 1654,2 s, 1638,8 s, 1622,0 s, 1608,2 s, 1591,2 s, 1489,7 w, 1466,1 w, 1433,9 w, 1411,8 w, 1393,2 w, 1373,5 w, 1342,3 s, 1329,9 s, 1301,5 m, 1250,6 s, 1219,4 w, 1172,3 w, 1120,0 w, 1058,3 w, 1029,0 w, 840,2 w, 832,2 m, 809,0 m, 749,2 m, 726,8 w $cm^{-1}$; $^1$HNMR (300 MHz, $CDCl_3$, 25˚C): δ = 0,81-0,85 (m, 12 H, $CH_3$), 1,23-1,40 (m, 32 H, $CH_2$), 1,86-1,97 (m, 4 H, β-$CH_2$), 2,24-2,37 (m, 4 H, β-$CH_2$), 3,99 (s, 3 H, $CH_3$), 5,18-5,30 (m, 2 H, CH-N), 7,14 (d, 2 H, $^3J$=8,8 Hz, $CH_{aryl}$), 8,22 (d, 2 H, $^3J$= 8,7 Hz, -$CH_{aryl}$), 8,38-8,68 (m, 6 H, $H_{pery}$), 10,57 (d, 1 H, $^3J$= 8,1 Hz), 11,30 ppm (s, 1H, N-H) ; UV/Vis ($CHCl_3$): $\lambda_{max}(\varepsilon)$ = 380,0 (7610), 399,0 (7490), 465,1 (15700), 482,1 (19800), 511,8 (16300), 548,1 (41100), 592,7 (73500); Fluoreszenzquantenausbeute ($CHCl_3$, $\lambda_{exc}$ = 465 nm, $E_{465nm}$ = 0,00434 $cm^{-1}$, Referenz: (1a) mit Φ = 1,00): 1,00); HMRS ($C_{58}H_{69}N_4O_5$): ber. *m*/*z*: 901,528; gef. *m*/*z*: 901,530, Δ = 2 mmu; $C_{58}H_{69}N_4O_5$ (901,2): ber. C 77,30, H 7,61, N 6,22; gef. C 77,34, H 7,78, N 6,09 %.

Beispiel 6: 2,10-Bis(1-hexylheptyl)-5-methyl-6-phenyl[1,3]diazepino[4',5',6':4,5]-phenanthro[2,1,10-*def*::7,8,9-*d'e'f'*]diisochinolin-1,3,9,11(2*H*,5*H*,9*H*,11*H*)-tetraon (4a). 2,10-Bis(1-hexylheptyl)-6-phenyl[1,3]diazepino[4',5',6':4,5]phenanthro[2,1,10-*def*::7,8,9-*d'e'f'*]diisochinolin-1,3,9,11(2*H*,5*H*,9*H*,11*H*)-tetraon (2a) gemäss Beispiel 1 (100 mg, 0,115 mMol) wurde in THF (30 mL) gelöst und mit wässriger KOH (10%, 2,5 mL) versetzt, bis zur Blaufärbung gerührt, tropfenweise mit Dimethylsulfat (0,3 ml, Vorsicht: toxisch) versetzt, 3 Std. bei Raumtemperatur gerührt, mit Wasser versetzt (100 mL), abgesaugt, mit Methanol gewaschen und säulenchromatographisch gereinigt. Ausbeute 90 mg (88 %) violetter Farbmittel, Schmp. > 300˚C, $R_f$(Kieselgel, Chloroform) = 0,80; IR (ATR): $\tilde{\nu}$ = 2952,8 m, 2922,3 s, 2854,5 m, 1688,8 s, 1645,9 s, 1588,8 s, 1528,5 w, 1486,3 w, 1462,8 w, 1424,1 w, 1404,1 w, 1332,2 s, 1253,5 m, 1219,9 w, 1179,2 w, 1099,5 w, 1023,2 m, 1016,0 w, 871,3 w, 808,0 m, 772,5 w, 746,0 m, 700,3 w, 600,0 w $cm^{-1}$; $^1$HNMR (300 MHz, $CDCl_3$, 25˚C): δ = 0,81-0,84 (m, 12 H, $CH_3$), 1,23-1,39 (m, 32 H, $CH_2$), 1,87-1,93 (m, 4H, β-$CH_2$), 2,26-2,33 (m, 4 H, β-$CH_2$), 4,01 (s, 3 H, $CH_3$) 5,19-5,24 (m, 2 H, CH-N), 7,67-7,72 (m, 3 H, $CH_{aryl}$), 8,09-8,10 (m, 2 H, $CH_{aryl}$), 8,56-8,72 (m, 6 H, $H_{pery}$), 10,74 ppm (d, 1 H, $^3J$=8,2Hz) $^{13}$C-NMR (151 MHz, $CDCl_3$, 25˚C): δ = 14,0, 22,6, 27,0, 29,3, 31,8, 32,4, 39,0, 54,6, 121,1, 121,1, 122,4, 122,4, 123,4, 126,7, 127,7, 129,0, 129,0, 129,1, 130,4, 131,0, 131,4, 134,6, 144,2, 163,0, 163,9, 165,0 ppm; UV/Vis ($CHCl_3$): $\lambda_{max}(E_{rel})$ = 377,5 (0,11), 394,5 (0,11), 437,6 (0,13), 502,9 (0,18), 540,2 (0,53), 584,8 (1,00); Fluoreszenz ($CHCl_3$): $\lambda_{max}$ ($I_{rel}$) = 599,0 (1,00), 650,0 (0,44); Fluoreszenzquantenausbeute ($CHCl_3$, $\lambda_{exc}$ = 490 nm, $E_{490\ nm}$ = 0,0094 $cm^{-1}$, Referenz: (1a) mit Φ = 1,00): 1,00; HMRS ($C_{58}H_{68}N_4O_4$): ber. *m*/*z*: 884,524; gef. *m*/*z*: 884,522; Δ = -0,002.

Beispiel 7: 2,10-Bis[1-(1-methylethyl)-2-methylpropyl]-5-methyl-6- phenyl[1,3]di-azepino[4',5',6':4,5]phenanthro[2,1,10-*def*:7,8,9-*d'e'f'*]diisochinolin- 1,3,9,11(2*H*,-5*H*,9*H*,11*H*)-tetraon (4b). 2,10-Bis[1-(1-methylethyl)-2-methylpropyl]-6- phenyl-[1,3]diazepino[4',5',6':4,5]phenanthro[2,1,10-*def*:7,8,9-*d'e'f'*]diisochinolin- 1,3,9,11-(2*H*,5*H*,9*H*,11*H*)-tetraon (2b) gemäss Beispiel 4 (500 mg, 0,711 mMol) wurden analog zu Beispiel 6 umgesetzt und aufgearbeitet, Ausbeute 450 mg (88%) metallisch glänzender, violetter Farbmittel. Schmp. >300˚C; $R_f$(Kieselgel, $CHCl_3$) = 0,22; HMRS ($C_{46}H_{44}N_4O_4$): ber. *m/z:* 716,335; gef. *m/z:* 716,335, Δ = 0,000 mmu.

Beispiel 8: 8,15-Bis-(1-hexylheptyl)-phenanthra[2,1,10-*def*:7,8,9-*d'e'f'*]-2,5-diphenyl-1,6,10,15-tetrahydro-imidizo[4,5-h:4',5'-h']diisochinolin-7,9,14,16-tetraon. *N*,*N*'-Bis(1-hexylheptyl)perylen-3,4:9,10-tetracarbonsäure-3,4:9,10-bisimid ((1a), 1.00 g, 1.32 mmol) und $NaNH_2$ (1.00 g, 25.6 mmol) wurden in Benzonitril (250 mL) suspendiert, auf 165˚C erhitzt (Blaufärbung), nach 3 h wieder abgekühlt, mit einem 1:1-Gemisch aus 2 N wässriger HCl und $CHCl_3$ (300 mL) ausgeschüttelt, über Magnesiumsulfat getrocknet, mit dem Rotationsverdampfer bei 12 mbar und dann zum Entfernen von Benzonitril in Feinvakuum eingedampft, in $CHCl_3$ aufgenommen, filtriert und säulenchromatographisch über Kieselgel mit Chloroform als Eluent gereinigt. 1. grüne Fraktion: 2,9-Bis(1-hexylheptyl)-bis-[1,3]diazepino-[4',5',6':1,12;4'',5'',6'':6,7]perylo[3,4-*cd*:9,10-*c'd'*]dipyridin-1,3,9,11(2*H*,5*H*,10*H*,13*H*)-tetraon. Ausbeute 40 mg (4 %) schwarzer Farbstoff, Schmp.: >250˚C. $R_f$(Kieselgel, Chloroform) = 0.88. 2. Fraktion: 2,10-Bis(1-hexylheptyl)-6-phenyl[1,3]-diazepino[4',5',6':4,5]phenanthro[2,1,10-*def*::7,8,9-*d'e'f'*]diisochinolin-1,3,9,11-(2*H*,5*H*,9*H*,11*H*)-tetraon. Ausb. 409 mg (36 %) metallisch

glänzender, violetter Farbstoff der Formel (8). Schmp. >250°C. $R_f$ (Kieselgel, Chloroform) = 0.85. 3. Fraktion: 8,15-Bis-(1-hexylheptyl)-phenanthra[2,1,10-*def*:7,8,9-*d'e'f'*]-2,5-diphenyl-1,6,10,15-tetrahydro-imidizo[4,5-h:4',5'-h']diisochinolin-7,9,14,16-tetraon. Ausb. 180 mg (18 %) grün-schwarzer Farbstoff, Schmp. >300°C. $R_f$(Kieselgel, Chloroform) = 0.23. IR (ATR): $\tilde{\nu}$ = 3387.6 m, 2952.9 m, 2920.8 s, 2853.6 m, 1685.1 m, 1639.5 s, 1593.9 s, 1582.7 s, 1545.9 w, 1486.1 w, 1456.3 w, 1429.2 w, 1402.4 w, 1381.5 w, 1349.0 m, 1325.2 m, 1304.9 m, 1290.2 m, 1242.9 s, 1173.7 m, 1127.5 w, 1026.1 w, 974.3 w, 879.7 w, 839.5 w, 810.7 m, 774.0 w, 753.0 w, 728.1 w, 701.4 w, 684.8, 624.9 w $cm^{-1}$. $^1$H-NMR (600 MHz, $CDCl_3$, 25°C): $\delta$ = 0.83 (t, 12 H, $^3J$ = 7.0 Hz, $CH_3$), 1.24-1.42 (m, 32 H, $CH_2$), 1.93-2.02 (m, 4 H, $\beta$-$CH_2$), 2.28-2.39 (m, 4 H, $\beta$-$CH_2$), 5.26-5.33 (m, 2 H, CH-N), 7.49-7.54 (m, 4 H, $CH_{aryl}$), 7.65-7.69 (m, 2 H, $CH_{aryl}$), 8.20-8.80 (m, 4 H, $CH_{aryl}$), 8.69-8.80 (m, 4 H, $H_{perylen}$), 18.13 ppm (s, 1 H, N-H). $^{13}$C-NMR (150 MHz, $CDCl_3$, 25°C): $\delta$ = 14.1, 22.6, 27.2, 29.3, 29.4, 31.8, 32.5, 54.6, 54.9, 121.8, 124.4, 127.3, 128.6, 129.3, 132.2, 139.3, 163.9, 164.9 ppm. UV/Vis ($CHCl_3$): $\lambda_{max}$ ($E_{rel}$) = 390.0 (0.15), 435.0 (0.23), 486.0 (0.11), 524.0 (0.12), 555.0 (0.17), 598.0 (0.52), 651.0 (1.00). Fluoreszenz ($CHCl_3$): $\lambda_{max}$ ($I_{rel}$) = 670.0 (1.00), 735.1 (0.33) nm. Fluoreszenzquantenausb. ($CHCl_3$, $\lambda_{exc}$ = 598 nm, $E_{589\ nm}$ = 0.0249 $cm^{-1}$, Referenz: (1a) mit 1.00): 0.80. MS (DEI$^+$ / 70 eV): *m*/*z* (%) = 989.5 (9), 988.5 (37) [$M^+$ + 2H], 987.5 (82) [$M^+$ + H], 986.5 (100) [$M^+$], 805.4 (13), 624.1 (31), 623.1 (65), 622.1 (32), 594.1 (23). $C_{64}H_{70}N_6O_4$ (987.3): Ber. C 77.86, H 7.15, N 8.51; Gef. C 77.83, H 7.08 N 8.47.

Beispiel 9: 2,10-Bis(1-hexylheptyl)-6-(2-bromphenyl)[1,3]diazepino[4',5',6':4,5]-phenanthro[2,1,10-*def*::7,8,9-*d'e'f'*]diisochinolin-1,3,9,11(2*H*,5*H*,9*H*,11*H*)-tetraon. *N*,*N*'-Bis(1-hexylheptyl)perylen-3,4:9,10-tetracarbonsäure-3,4:9,10-bisimid (150 mg, 0.199 mmol) und $NaNH_2$ (150 mg, 3.85 mmol) werden in o-Bromobenzonitril (10 g) suspendiert und analog zu 2,10-Bis(1-hexylheptyl)-6-phenyl[1,3]diazepino[4',5',6':-4,5]phenanthro[2,1,10-*def*::7,8,9-*d'e'f'*]diisochinolin-1,3,9,11(2*H*,5*H*,9*H*,11*H*)-tetraon umgesetzt und aufgearbeitet und säulenchromatographisch gereinigt (Kieselgel, Chloroform und *iso*-Hexan (3:1)). Ausb. 91 mg (48.4 %) violetter Farbstoff der Formel (8), Schmp. >250°C. $R_f$(Kieselgel, Chloroform) = 0.80. $^1$H-NMR (300 MHz, $CDCl_3$, 25°C): $\delta$ = 0.80-0.93 (m, 12 H, $CH_3$), 1.18-1.48 (m, 32 H, $CH_2$), 1.83-2.02 (m, 4 H, $\beta$-$CH_2$), 2.20-2.43 (m, 4 H, $\beta$-$CH_2$), 5.15-5.35 (m, 2 H, CH-N), 7.52 (t, 1 H, $^3J$=7.6 Hz, $CH_{aryl}$), 7.69 (t, 1 H, $^3J$=7.6 Hz, $CH_{aryl}$), 7.87 (d, 1 H, $^3J$=7.6 Hz, $CH_{aryl}$), 8.60.8.88 (m, 6 H, $CH_{perylen}$), 10.79 (d, 1 H, $^3J$ =7.8 Hz, $CH_{perylen}$), 12.41 ppm (s, 1 H, N-H). UV/Vis ($CHCl_3$): $\lambda_{max}$ ($\varepsilon$) = 380.3 (9280), 397.1 (10070), 438.8 (13350), 461.2 (13030), 505.5 (16430), 542.9 (47470), 588.1 (86950). Fluoreszenz ($CHCl_3$): $\lambda_{max}$ ($I_{rel}$) = 601.5 (1.00), 654.1 (0.45). Fluoreszenzquantenausb. ($CHCl_3$, $\lambda_{exc}$ = 542 nm, $E_{542\ nm}$ = 0.0097 cm-1, Referenz: (1a) mit 1.00): 1.00. HRMS ($C_{57}H_{65}{}^{79}BrN_4O_4$): Ber. *m*/*z* =948.4189; Gef. *m*/*z* = 948.4154, $\Delta$ = -0.0035. $C_{57}H_{65}BrN_4O_4$ (950.1): Ber. C 72.06, H 6.90, N 5.90; Gef. C 71.91, H 6.76, N 5.67.

Beispiel 10: 2,10-Bis(1-hexylheptyl)-6-(3-bromphenyl)[1,3]diazepino[4',5',6':4,5]-phenanthro[2,1,10-*def*::7,8,9-*d'e'f'*]diisochinolin-1,3,9,11(2*H*,5*H*,9*H*,11*H*)-tetraon. *N*,*N*'-Bis(1-hexylheptyl)perylen-3,4:9,10-tetracarbonsäure-3,4:9,10-bisimid (200 mg, 0.265 mmol) und $NaNH_2$ (200 mg, 5.13 mmol) wurden in m-Bromobenzonitril (10 g) suspendiert und analog zu 2,10-Bis(1-hexylheptyl)-6-phenyl[1,3]diazepino[4',5',6':-4,5]phenanthro[2,1,10-*def*::7,8,9-*d'e'f'*]diisochinolin-1,3,9,11(2*H*,5*H*,9*H*,11*H*)-tetraon umgesetzt und aufgearbeitet und säulenchromatographisch gereinigt (Kieselgel, Chloroform/*iso*-Hexan 3:1). Ausb 107 mg (42.4%) violetter Farbstoff der Formel (8), Schmp. >250°C. $R_f$(Kieselgel, Chloroform) = 0.80. $^1$H-NMR (400 MHz, $CDCl_3$, 25°C): $\delta$ = 0.74-0.87 (m, 12 H, $CH_3$), 1.13-1.45 (m, 32 H, $CH_2$), 1.82-2.00 (m, 4 H, $\beta$-$CH_2$), 2.20-2.37 (m, 4 H, $\beta$-$CH_2$), 5.14-5.31 (m, 2 H, CH-N), 7.53 (t, 1 H, $^3J$=7.9 Hz, $CH_{aryl}$), 7.77 (ddd, 1 H, $^3J$=8.0 Hz, $^4J$=1.8 Hz, $^4J$=0.8 Hz, $CH_{aryl}$), 8.25 (d, 1 H, $^3J$=7.6 Hz, $CH_{aryl}$), 8.50-8.81 (m, 6H, $CH_{perylen}$), 10.64 (d, 1 H, $^3J$=8.2 Hz, $CH_{perylen}$), 11.48 ppm (s, 1 H, N-H). $^{13}$C-NMR (150 MHz, $CDCl_3$, 25°C): $\delta$ = 14.3, 22.8, 27.2, 29.5, 31.8, 32.0, 32.7, 54.9, 121.5, 123.0, 123.8, 123.8, 125.7, 125.8, 126.5, 126.8, 129.9, 129.3, 1230.4, 130.6, 131.0, 131.2, 132.6, 134.8, 135.2, 135.5, 139.0, 139.2, 143.7, 155.8, 164.0, 165.0 ppm. UV/Vis ($CHCl_3$): $\lambda_{max}$ ($E_{rel}$) = 378.6 (0.10), 399.1 (0.10), 437.2 (0.13), 461.4 (0.13), 504.6 (0.16), 542.7 (0.51), 587.4 (1.00). Fluoreszenz ($CHCl_3$): $\lambda_{max}$ ($I_{rel}$) = 595.8 (1.00), 648.8 (0.42). Fluoreszenzquantenausbeute ($CHCl_3$, $\lambda_{exc}$ = 542 nm, $E_{542\ nm}$ = 0.0068 $cm^{-1}$, Referenz: (1a) mit 1.00): 0.99. HRMS ($C_{57}H_{65}{}^{79}BrN_4O_4$): Ber. *m*/*z* = 948.4189; Gef. *m*/*z* = 948.4177, $\Delta$ = -0.0012.

Beispiel 11: 2,10-Bis-(1-hexylheptyl)-6-(4-dimethylaminophenyl)[1,3]diazepino-[4',5',6'-d",e",f"]phenanthra [2,1,10-*def*:7,8,9-*d'e'f'*]-2,5,9,11-tetrahydro-diisochinolin-1,3,9,11-tetraon. *N*,*N*'-Bis(1-hexylheptyl)perylen-3,4:9,10-tetracarbonsäure-3,4:9,10-bisimid (200 mg, 0.26 mmol), $NaNH_2$ (200 mg, 5.13 mmol) wurden in 4-Dimethylaminobenzonitril (10 g) suspendiert und analog zu 2,10-Bis(1-hexylheptyl)-6-phenyl[1,3]diazepino[4',5',6':4,5]phenanthro [2,1,10-*def*::7,8,9-*d'e'f'*]diisochinolin-1,3,9,11(2*H*,5*H*,9*H*,11*H*)-tetraon umgesetzt und aufgearbeitet und säulenchromatographisch (Kieselgel, $CHCl_3$) gereinigt. Der Farbstoff der Formel (8) wird in Chloroform gelöst und mit Methanol gefällt. Ausb. 23 mg (12 %) metallisch glänzender, violetter Farbstoff, Schmp. >300°C. $R_f$(Kieselgel, Chloroform) = 0.70. IR (ATR): $\tilde{\nu}$ = 3416.2 m, 3096.0 w, 2952.8 s, 2921.7 s, 2853.8 s, 2364.3 w, 1691.1 m, 1639.8 s, 1626.6 s, 1607.3 s, 1590.8 s, 1493.2 m, 1470.3 m, 1434.2 m, 1413.5 m, 1365.8 m, 1343.8 s, 1306.2 m, 1255.9 s, 1219.9 m, 1196.3 m, 1121.0 m, 1103.5 m, 1058.3 m, 1038.2 w, 949.8 w, 871.3 w, 809.8 m, 751.4 w, 619.1 w, 581.6 w $cm^{-1}$. $^1$H-NMR (600 MHz, $CDCl_3$, 25°C): $\delta$ = 0.82-0.87 (m, 12 H, $CH_3$), 1.25-1.46 (m, 32 H, $CH_2$), 1.88-1.99 (m, 4 H, $\beta$-$CH_2$), 2.25-2.35 (m, 4 H, $\beta$-$CH_2$), 3.17 (s, 6 H, $NCH_3$), 5.21-5.31 (m, 2 H, CH-N), 6.87 (d, 2 H, $^3J$ =8.2Hz, $CH_{aryl}$), 8.15 (d, 2 H, $^3J$ =8.2Hz, $CH_{aryl}$), 8.46-8.73 (m, 5 H, $H_{perylen}$), 10.65 (d, 1 H, $^3J$=8.1Hz), 11.25 ppm (s, 1H, N-H). $^{13}$C-NMR (150 MHz, $CDCl_3$, 25°C): $\delta$ = 14.1, 14.1, 22.6, 27.0, 29.3, 31.8, 31.8, 32.5, 40.4, 54.6, 112.1, 120.8, 122.1, 123.4, 126.1, 126.6, 129.2, 129.3, 130.0, 130.8, 134.7,

134.9, 135.0, 144.8, 152.6, 161.8, 164.0, 165.0 ppm. UV/Vis ($CHCl_3$): $\lambda_{max}$ ($E_{max}$) = 396.0 (0.20), 513 (sh, 0.67), 544 (1.0), 594 (sh, 0.91), 616 nm (0.98). Fluoreszenz ($CHCl_3$): $\lambda_{max}$ = 740.2 nm. Fluoreszenzquantenausbeute ($CHCl_3$, $\lambda_{exc}$ = 465 nm, $E_{465\ nm}$ = 0.0476 $cm^{-1}$, Referenz: (1a) mit $\Phi$ = 1.00): 0.72. HRMS ($C_{59}H_{71}N_5O_4$): Ber. *m*/*z*: = 913.550; Gef. *m*/*z* = 913.549, $\Delta$ = -0.0010.

Beispiel 12: 2,10-Bis-(1-hexylheptyl)-6-(2,4-bismethoxyphenyl)-[1,3]diazepino-[4',5',6'-d'';e'';f'']phenanthra [2,1,10-*def*:7,8,9-*d'e'f'*]-2,5,9,11-tetrahydro-diisochinolin-1,3,9,11-tetraon. *N*,*N'*-Bis(1-hexylheptyl)perylen-3,4:9,10-tetracarbonsäure-3,4:9,10-bisimid (200 mg, 0.265 mmol) und $NaNH_2$ (200 mg, 5.13 mmol) wurden in 2,4-Bismethoxybenzonitril (10 g) bei 100°C gelöst und analog zu 2,10-Bis(1-hexylheptyl)-6-phenyl[1,3]diazepino[4',5',6':4,5]phenanthro [2,1,10-*def*::7,8,9-*d'e'f'*]diisochinolin-1,3,9,11(2*H*,5*H*,9*H*,11*H*)-tetraon umgesetzt und aufgearbeitet und säulenchromatographisch gereinigt (Kieselgel, Chloroform) Ausb. 80 mg (32.4 %) metallisch glänzender, violetter Farbstoff der Formel (8). Schmp. >300°C. $R_f$ (Kieselgel, Chloroform) = 0.38. UV/Vis ($CHCl_3$): $\lambda_{max}$ ($E_{rel}$) = 396.9 (0.14), 464.3 (0.29), 491.4 (0.37), 560.3 (0.62), 603.5 (1.00). Fluoreszenz ($CHCl_3$): $\lambda_{max}$ ($I_{rel}$) = 634.2 (1.00), 684.6 (0.66). Fluoreszenzquantenausb. ($CHCl_3$, $\lambda_{exc}$ = 560 nm, $E_{560\ nm}$ = 0.01088 $cm^{-1}$, Referenz: (1a) mit $\Phi$ = 1.00): 0.96. $^1$H-NMR (400 MHz, $CDCl_3$, 25°C): $\delta$ = 0.77-0.87 (m, 12 H, $CH_3$), 1.17-1.43 (m, 32 H, $CH_2$), 1.81-1.98 (m, 4 H, $\beta$-$CH_2$), 2.20-2.40 (m, 4 H, $\beta$-$CH_2$), 3.96 (s, 3 H, $OCH_3$), 4.15 (s, 3 H, $OCH_3$), 5.15-5.30 (m, 2 H, CH-N), 6.61 (d, 2 H, $^3J$ =1.8 Hz, $CH_{aryl}$), 6.80 (d, 2 H, $^3J$ =9.1 Hz, $CH_{aryl}$), 8.50-8.79 (m, 6 H, $H_{perylen}$), 10.60-10.72 (br, 1 H), 12.13-12.24 ppm (br, 1 H, N-H). $^{13}$C-NMR (100 MHz, $CDCl_3$, 25°C): $\delta$ = 14.3, 22.9, 27.3, 29.5, 32.0, 32.7, 54.8, 56.0, 56.5, 98.9, 106.8, 109.8, 121.2122.4, 123.6, 126.3, 126.9, 129.4, 130.2, 130.5, 133.2, 135.0, 135.4, 143.7, 159.9, 164.5, 165.4 ppm. HRMS ($C_{59}H_{70}N_4O_6$): Ber. *m*/*z* = 930.5295; Gef. *m*/*z* = 930.5289, $\Delta$ = -0.0006.

Beispiel 13: 2,10-Bis-(1-hexylheptyl)-6-(3,4-bismethoxyphenyl)-[1,3]diazepino-[4',5',6'-d'',e'';f'']phenanthra [2,1,10-*def*:7,8,9-*d'e'f'*]-2,5,9,11-tetrahydro-diisochinolin-1,3,9,11-tetraon. *N*,*N'*-Bis(1-hexylheptyl)perylen-3,4:9,10-tetracarbonsäure-3,4:9,10-bisimid (0.240 g, 0.317 mmol) und $NaNH_2$ (0.200 g, 5.13 mmol) wurden in 2,4-Bismethoxybenzonitril (10 g) bei 100 °C gelöst und analog zu 2,10-Bis(1-hexylheptyl)-6-phenyl[1,3]diazepino[4',5',6':4,5]phenanthro [2,1,10-*def*::7,8,9-*d'e'f'*]diisochinolin-1,3,9,11(2*H*,5*H*,9*H*,11*H*)-tetraon umgesetzt (4.5 h Reaktionszeit) und aufgearbeitet und säulenchromatographisch gereinigt (Kieselgel, Chloroform). Ausb.: 118 mg (39.9 %) metallisch glänzender, violetter Farbstoff der Formel (8), Schmp. >300°C. $R_f$(Kieselgel, Chloroform) = 0.38. $^1$H-NMR (600 MHz, $CDCl_3$, 25°C): $\delta$ = 0.78-0.85 (m, 12 H, $CH_3$), 1.17-1.44 (m, 32 H, $CH_2$), 1.83-1.98 (m, 4 H, $\beta$-$CH_2$), 2.21-2.34 (m, 4 H, $\beta$-$CH_2$), 4.04 (s, 3 H, $OCH_3$), 4.11 (s, 3 H, $OCH_3$), 5.15-5.28 (m, 2 H, CH-N), 7.07 (d, 2 H, $^3J$ =8.4 Hz, $CH_{aryl}$), 7.78-7.87 (m, 2 H, $CH_{aryl}$), 8.44-8.75 (m, 5 H, $H_{peryylen}$), 10.63-10.68 (br, 1 H), 11.35 ppm (s, 1 H, N-H). $^{13}$C-NMR (100 MHz, $CDCl_3$, 25°C): $\delta$ = 14.3, 14.3, 22.8, 22.8, 27.2, 27.3, 27.4, 32.5, 32.7, 32.8, 54.8, 54.9, 56.5, 56.5, 103.4, 104.2, 111.6, 121.0, 121.3, 121.5, 122.7, 123.6, 126.6, 126.8, 129.4, 130.6, 144.3, 150.0, 153.0, 157.7, 164.1, 165.3, 166.0 ppm. UV/Vis ($CHCl_3$): $\lambda_{max}$ ($E_{rel}$) = 500.9 (0.29), 549.4 (0.58), 593.7 (1.00). Fluoreszenz ($CHCl_3$): $\lambda_{max}$ ($I_{rel}$) = 614.8 (1.00), 665.7 (0.61). Fluoreszenzquantenausbeute ($CHCl_3$, $\lambda_{exc}$ = 549 nm, $E_{549\ nm}$ = 0.008146 $cm^{-1}$, Referenz: (1a) mit $\Phi$ = 1.00): 1.00. HRMS ($C_{59}H_{70}N_4O_6$): Ber. *m*/*z* = 930.5295; Gef. *m*/*z* = 930.5284, $\Delta$ = -0.0011. $C_{59}H_{70}N_4O_6$ (931.2): Ber. C 76.10, H 7.58, N 6.02; Gef. C 75.66, H 7.38, N 5.90.

Beispiel 14: Distickstofftetraoxidin Dichlormethan: Festes Blei(II)nitrat (100 g, 302 mmol) wird in einem Rundkolben mit dem Bunsenbrenner stark erhitzt und die entstehenden nitrosen Gase solange in einen Vorlagekolben mit Dichlormethan (1000 mL) geleitet, bis die Dichlormethan-Lösung gesättigt ist. Dieses Reagenz wird für den alternativen Syntheseweg gemäss den folgenden Beispielen verwendet.

Beispiel 15: 1-Nitro-*N*,*N'*-bis-(1-hexylheptyl)perylen-3,4:9,10-bis(dicarboximid). *N*,*N'*-Bis(1-hexylheptyl)perylen-3,4:9,10-tetracarbonsäure-3,4:9,10-bisimid (1a, 3.00 g, 3.97 mmol) wurde in Dichlormethan (200 mL) vorgelegt, mit Methansulfonsäure (2 mL, 30.8 mmol) versetzt (Katalysator im Überschuss), mit soviel einer gesättigten Lösung von Distickstofftetroxid in Dichlormethan (gemäss Beispiel 14) unter Rühren bei Raumtemperatur tropfenweise versetzt bis eine Kontrolle mittels Dünnschichtchromatographie (Kieselgel, Chloroform) eine vollständige Umsetzung ergibt (Farbumschlag der Lösung nach Dunkelrot); mit destilliertem Wasser gewaschen (100 mL), über Magnesiumsulfat getrocknet, mit dem Rotationsverdampfer eingedampft, in wenig Chloroform gelöst und säulenchromatographisch gereinigt (Kieselgel, Dichlormethan). Ausbeute 2.41 g (76 %) dunkelroter, metallisch-glänzender Feststoff, Schmp. 120 °C. $R_f$ (Kieselgel/$CHCl_3$): 0.80. IR (ATR): $\tilde{\nu}$ = 3048 w, 2955 s, 2927 s, 2857 s, 1703 s, 1661 s, 1596 s, 1537 s, 1457 m, 1427 m, 1405 s, 1337 s, 1251 s, 1209 w, 1179 m, 1112 w, 973 w, 920 w, 855 w, 812 m, 746 $cm^{-1}$ m. $^1$H-NMR (300 MHz, $CDCl_3$, 25°C) $\delta$ = 0.83 (t, 6 H, $^3J$= 7.0 Hz, $CH_3$), 1.21-1.38 (m, 16 H, $CH_2$), 1.85-1.91 (m, 2 H, $\beta$-$CH_2$), 2.22-2.28 (m, 2 H, $\beta$-$CH_2$), 5.10-5.20 (m, 2 H, $\alpha$-$CH_2$), 8.25 (d, $^3J$ = 8.1 Hz, 1 H, $H_{perylen}$), 8.59 (d, $^3J$ = 8.1 Hz, 1 H, $H_{perylen}$), 8.71-8.77 (m, 5 H, $H_{perylen}$). UV/Vis ($CHCl_3$): $\lambda_{max}$ ($E_{rel}$) = 490 (0.67), 523 nm (1.0).

Beispiel 16: 1-Amino-*N*,*N'*-bis-(1-hexylheptyl)perylen-3,4:9,10-bis(dicarboximid). 1-Nitro-*N*,*N'*-bis-(1-hexylheptyl)perylen-3,4:9,10-bis(dicarboximid) gemäss Beispiel 15 (2.41 g, 3.01 mmol) wurde in THF (100 mL) gelöst (dunkelrote Lösung), mit Eisen-Pulver (350 mg, 6.27 mmol) und konzentrierter Salzsäure (11 mL) versetzt, unter Rühren 30 Minuten unter Rückfluss erhitzt (nach 10 Minuten ein Farbumschlag von Dunkelrot nach Dunkelblau), abkühlen lassen, mit destill. Wasser (500 mL) gefällt, abgesaugt, in wenig Chloroform gelöst, säulenchromatographisch gereinigt (Kieselgel, Chloroform) und eingedampft. Ausb. 2.08 g (72.1 %). sehr feiner, amorphen Feststoff, Schmp. 92-94°C. $R_f$ (Kieselgel/$CHCl_3$):

0.30. IR (ATR): $\tilde{\nu}$ =3350 m, 3242 m, 3047 w, 2954 s, 2926 s, 2856 s, 1694 s, 1653 s, 1616 m, 1590 s, 1573 m, 1510 m, 1463 m, 1429 s, 1397 m, 1373 m, 1339 s, 1311 m, 1269 m, 1252 m, 1178 m, 1122 w, 1084 m, 1062 w, 979 w, 846 w, 809 m, 750 w, 725 $cm^{-1}$ w. $^1$H-NMR (300 MHz, $CDCl_3$, 25˚C): $\delta$ = 0.83 (t, 6 H, $^3J$= 7.0 Hz, $CH_3$), 1.21-1.38 (m, 16 H, $CH_2$), 1.85-1.91 (m, 2 H, $\beta$-$CH_2$), 2.22-2.28 (m, 2 H, $\beta$-$CH_2$), 5.10-5.20 (m, 4 H, $\alpha$-$CH_2$, $NH_2$), 8.17 (s, 1 H, $H_{Perylen}$), 8.49-8.55 (m, 3 H, $H_{Perylen}$), 8.64 (s, 2 H, $H_{Perylen}$), 8.87 ppm (d, $^3J$ = 8.1 Hz, 1 H, $H_{Perylen}$). UV/Vis ($CHCl_3$): $\lambda_{max}$ ($E_{rel}$) = 420 (0.32), nm 571 (1.00). Fluoreszenz ($CHCl_3$): $\lambda_{max}$ = 684 nm.

Beispiel 17: 1-Benzamidyl-*N*,*N*'-bis(1-hexylheptyl)perylen-3,4:9,10-tetracarbonsäurebisimid. 1-Amino-*N*,*N*'-bis-(1-hexylheptyl)perylen-3,4:9,10-bis(dicarboximid) gemäss Beispiel 16 (240 mg, 0.31 mmol) wurde in Dioxan-(60 mL) gelöst, tropfenweise mit Benzoylchlorid (1.00 g, 7.10 mmol) in Dioxan (10 mL) versetzt, 7 h bei 100 ˚C unter Rückfluß gerührt (nach 2 h wird noch einmal die gleiche Menge an Benzoylchlorid zugegeben (Überwachung des Reaktionsendes mittels DC), abfiltriert. Säulenchromatographisch gereinigt (Kieselgel, Chloroform, roter Vorlauf aus dem Ausgangsmaterial, pinkfarbene Hauptfraktion), mit dem Rotationsverdampfer eingedampft, in wenig Chloroform aufgenommen und mit Methanol gefällt, abgesaugt und im Trockenschrank bei 110 ˚C getrocknet. Ausb. 125 mg (46 %) pinker Farbstoff der Formel (7), Schmp. >300 ˚C. $R_f$ (Kieselgel, $CH_2Cl_2$) = 0.53. IR (ATR): $\tilde{\nu}$ = 3212.5 w, 2952.1 m, 2921.4 s, 2853.3 m, 1697.0 s, 1654.9 s, 1591.4 m, 1527.1 w, 1503.1 w, 1482.2 w, 1466.4 m, 1455.4 m, 1407.9 m, 1362.2 w, 1326.9 s, 1267.5 m, 1246.2 m, 1175.6 m, 1106.9 w, 1025.0 w, 971.7 w, 940.7 w, 896.9 w, 845.5 w, 809.2 m, 745.5 w, 702.2 w, 686.8 w, 614.9 w $cm^{-1}$. $^1$H-NMR (600 MHz, $CDCl_3$, 25˚C): $\delta$ = 0.82 (t, $^3J_{H,H}$ = 7.0 Hz, 12 H, $CH_3$), 1.16-1.35 (m, 33 H, $CH_2$ und NH), 1.79-1.85 (m, 4 H, $\beta$-$CH_2$), 2.15-2.25 (m, 4 H, $\beta$-$CH_2$), 5.10-5.19 (m, 2 H, $\alpha$-CH), 7.59 (t, $^3J$ = 7.7 Hz, 2 H, Phenyl), 8.04 (d, $^3J$ = 7.3 Hz, 1 H, Phenyl), 8.45 (s, 2H, Phenyl), 8.52 (d, $^3J$ = 7.9 Hz, 1 H, $H_{Perylen}$), 8.60-8.64 (m, 2 H, Phenyl), 8.79 (s, 1 H, $CH_{Perylen}$), 8.97-8.99 ppm (m, 2 H, $CH_{Perylen}$). $^{13}$C-NMR (150 MHz, $CDCl_3$, 25˚C): $\delta$ = 14.0, 22.6, 26.9, 29.2, 31.7, 31.8, 32.3, 54.8, 54.9, 122.5, 123.7, 125.6, 127.0, 127.5, 128.0, 129.2, 132,9, 133.2, 134.3, 134.3, 135.2, 135.2, 165.8 ppm. UV/ViS ($CHCl_3$): $\lambda_{max}$ ($E_{rel}$) = 274 (0.51), 313 (0.29), 380 (0.12), 419 (0.12), 498 (0.71), 531 nm (1.00). Fluoreszenz ($CHCl_3$): $\lambda_{max}$ = 599 nm. Fluoreszenzquantenausb. ($CHCl_3$, $\lambda_{exc}$ = 500 nm, $E_{500\ nm}$ = 0.01835 $cm^{-1}$, Referenz: (1a) mit 1.00): 0.813. HRMS ($C_{57}H_{67}N_3O_5$): Ber. *m/z* = 873.508; Gef. *m/z* = 873.508., $\Delta$ = 0.0000. $C_{57}H_{67}N_3O_5$ (874.16): Ber. C 78.32, H 7.73, N 4.81; Gef. C 77.97, H 7.43, N 4.73.

Beispiel 18: 2,10-Bis-(1-hexylheptyl)-6-phenyl[1,3]diazepino[4',5',6'-*d''*,*e''*,*f''*]phenanthra[2,1,10-*def*: 7,8,9-*d'e'f'*]-2,5,9,11-tetrahydro-diisochinolin-1,3,9,11-tetraon. 1-Benzamidyl-*N*,*N*'-bis(1-hexylheptyl)perylen-3,4:9,10-tetracarbonsäure-3,4-anhydrid-9,10-imid (100 mg, 0.110 mmol) und $NaNH_2$ (100 mg, 2.56 mmol) wurden in Benzonitril (250 mL) suspendiert, auf 165˚C erhitzt (Blaufärbung), nach 3 h wieder abgekühlt, mit einem 1:1-Gemisch aus HCl-Lösung (2 N) und $CHCl_3$ (300 mL) ausgeschüttelt, vom $CHCl_3$ befreit, im Vakuum destillativ vom Benzonitril befreit, in wenig $CHCl_3$ aufgenommen, filtriert und säulenchromatographisch gereinigt (Kieselgel, $CHCl_3$). Ausb. 60 mg (57 %) metallisch glänzender, violetter Farbstoff.

Beispiel 19: Erschöpfende Nitrierung von Perylen-3,4,9,10-tetracarbonsäure-3,4:9,10-bits-(1-hexylheptylimid) - 1,6-Dinitroperylen-3,4,9,10-tetracarbonsäure-3,4:9,10-bis-(-hexylheptylimid) und 1,7-Dinitroperylen-3,4,9,10-tetracarbonsäure-3,4:9,10-bis-(1-hexylheptylimid). *N*,*N*'-Bis(1-hexylheptyl)perylen-3,4:9,10-tetracarbonsäure-3,4:9,10-bisimid ((1a), 3.19 g, 4.23 mmol) wurde in wenig Dichlormethan gelöst, mit Methansulfonsäure (2 mL) und Distickstofftetroxid-Lösung versetzt, unter Bestrahlung mit Licht (80 W Wolframfaden-Glühlampe) 6 h bei Raumtemperatur gerührt, durch Versetzen mit destill. Wasser (100 ml) gestoppt, mehrfach mit Chloroform (jeweils 100 ml) extrahiert, über Magnesiumsulfat getrocknet, eingeengt und chromatographiert (Kieselgel, Chloroform). Man erhält ein Gemisch aus 1,6-Dinitroperylen-3,4,9,10-tetracarbonsäure-3,4:9,10-bis-(1-hexylheptylimid) und 1,7-Dinitroperylen-3,4,9,10-tetracarbonsäure-3,4:9,10-bis-(1-hexylheptylimid) als tiefroten Feststoff. Eine Trennung dieser beiden Isomere gelang aufgrund identischer $R_f$-Werte nicht. Ausb. 2.72 g (76.1 %). $R_f$ (Kieselgel/- $CHCl_3$): 0.7. IR (ATR):$\tilde{\nu}$ = 3047 w, 2978 m, 2927 s, 2857 m, 1705 s, 1664 s, 1599 s, 1542 s, 1427 w, 1407 m, 1335 s, 1251 m, 812 m, 743 $cm^{-1}$ w. $^1$H-NMR (300 MHz, $CDCl_3$, 25˚C): $\delta$ = 0.83 (t, 6 H, $^3$J= 7.0 Hz, $CH_3$), 1.21-1.38 (m, 16 H, $CH_2$), 1.85-1.91 (m, 2 H, $\beta$-$CH_2$), 2.22-2.28 (m, 2 H, $\beta$-$CH_2$), 5.10-5.20 (m, 2 H, $\alpha$-$CH_2$), 8.31 (d, $^3J$ = 8.0 Hz, 1 H, $CH_{Perylen}$), 8.33 (d, $^3J$ = 8.0 Hz, 1 H, $CH_{Perylen}$), 8.66 - 8.70 (m, 2 H, $CH_{Perylen}$), 8.83 (s, 2 H, $H_{Perylen}$). UV/Vis ($CHCl_3$): $\lambda_{max}$ ($E_{rel}$) = 450 (0.26), 491 (0.73), 520 nm (1.00).

Beispiel 20*:* Isomerengemisch 1,6-Diaminoperylen-3,4,9,10-tetracarbonsäure-3,4:9,10-bis-(1-hexylheptylimid) und 1,7-Diaminoperylen-3,4,9,10-tetracarbonsäure-3,4:9,10-bis-(1-hexylheptylimid). Das Isomerengemisch 1,6-Dinitroperylen-3,4,9,10-tetracarbonsäure-3,4:9,10-bis-(1-hexylheptylimid) und 1,7-Dinitroperylen-3,4,9,10-tetracarbonsäure-3,4:9,10-bis-(1-hexylheptylimid) aus der Nitrierung von *N*,*N*'-Bis(1-hexylheptyl)perylen-3,4:9,10-tetracarbonsäure-3,4:9,10-bisimid (1.00 g, 1.19 mmol) wurde in siedendem Ethanol (150 mL) unter Rückfluss gelöst, mit Eisenpulver (500 mg, 8.93 mmol) und Salzsäure (konz., 5.00 mL) versetzt, 30 min gerührt, durch Zugabe von destill. Wasser (500 mL) gestoppt, eine Stunde weiter bei Raumtemperatur gerührt, abfiltriert und chromatographiert (Kieselgel, Dichlormethan). Ausb. 814 mg (87.6 %), Schmp. 146 - 148 ˚C. $R_f$ (Kieselgel/$CHCl_3$):0.20. IR (ATR): $\tilde{\nu}$ = 3441 s br., 3360 s, 3250 m, 2954 m, 2926 s, 2857 m, 1691 s, 1646 s, 1588 s, 1552 w, 1530 w, 1515 w, 1455 m, 1422 m, 1393 m, 1339 s, 1272 m, 1246 w, 1185 w, 1107 w, 984 w, 873 m, 806 m, 778 w, 755 m, 723 w, 574 $cm^{-1}$ w. UV/Vis ($CHCl_3$): $\lambda_{max}$ ($E_{rel}$) = 384 nm (0.19), 402 (0.25), 432 (0.16), 506 sh (0.32), 581 sh (0.90), 611 (1.0).

Beispiel 21: 1,6-Bis-benzamidyl-*N*,*N*'-bis(1-hexylheptyl)perylen-3,4:9,10-tetra-carbonsäurebisimid und 1,7-Bis-benzamidyl-*N*,*N'*-bis(1-hexylheptyl)perylen-3,4:9,10-tetracarbonsäurebisimid. Eine Lösung aus dem Isomerengemisch 1,6-Diaminoperylen-3,4,9,10-tetracarbonsäure-3,4:9,10-bis-(1-hexylheptylimid) und 1,7-Diaminoperylen-3,4,9,10-tetracarbonsäure-3,4:9,10-bis-(1-hexylheptylimid) (1.00 g, 1.27 mmol) in 1,4-Dioxan (100 mL) wurde mit Benzoylchlorid (2.00 g, 14.2 mmol) in 1,4-Dioxan (10 mL) tropfenweise versetzt, insgesamt 7 h bei 100 ˚C unter Rückfluss gerührt (nach jeweils 2 h wurden Benzoylchlorid (2.00 g) zugegeben und das Ende der Reaktion wird mittels DC überwacht), eingeengt durch Abdestillieren vom Benzoylchlorid befreit, säulenchromatographisch (Kieselgel, Chloroform, pinkfarbenes Produkt nach einigen Vorfraktionen) gereinigt, mit dem Rotationsverdampfer eingedampft und im Feinvakuum bei 200˚C von einer farblosen Flüssigkeit befreit. Ausb. 450 mg (35 %) metallisch glänzender Farbstoff, Schmp. >300˚C. $R_f$ (Kieselgel, Chloroform) = 0.05. IR (ATR): $\tilde{\nu}$ = -3222.2 m, 2952.2 m, 2922.0 s, 2854.0 m, 1699.4 s, 1654.9 s, 1592.0 s, 1502.4 w, 1478.9 w, 1455.4 w, 1409.3 w, 1322.4 s, 1267.3 m, 1243.3 m, 1180.4 w, 1108.0 w, 1026.1 w, 895.7 w, 862.1 m, 809.1 w, 749.4 m, 704.0 m, 585.4 w $cm^{-1}$. $^1$H-NMR (600 MHz, $CDCl_3$, 25˚C): $\delta$ = 0.82-0.86 (m, 12 H, $CH_3$), 1.11-1.26 (m, 32 H, $CH_2$), 1.80-2.10 (m, 8 H, $\beta$-$CH_2$), 4.90-5.02 (m, 2 H, CH-N), 7.48-7.66 (m, 5 H, $CH_{aryl}$), 7.87-7.92 (m, 3 H, $CH_{aryl}$), 8.18-8.22 (m, 2 H, CHaryl), 8.58-8.80 ppm (m, 6 H, $H_{pery}$). $^{13}$C-NMR (150 MHz, $CDCl_3$, 25˚C): $\delta$ = 14.1, 14.1, 14.1, 22.6, 22.6, 22.6, 27.0, 27.0, 29.3, 29.3, 29.4, 31.7, 31.7, 31.7, 32.1, 32.3, 54.8, 55.2, 124.8, 125.1, 126.1, 126.1, 127.4, 127.7, 127.9, 128.3, 128.3, 128.3, 128.3, 128.8, 128.8, 128.8, 128.8, 129.2, 129.4, 130.3, 131.0, 132.7, 133.7, 135.4, 162.4, 163.0, 164.1, 165.9 ppm. UV/Vis ($CHCl_3$): $\lambda_{max}$ ($E_{rel}$) = 387 (0.36), 544 nm (1.0). Fluoreszenz ($CHCl_3$): $\lambda_{max}$ ($I_{rel}$) = 615 nm. Fluoreszenzquantenausb. ($CHCl_3$, $\lambda_{exc}$ = 521 nm, $E_{521}$ nm = 0.03251 $cm^{-1}$, Referenz: (1a) mit 1.00): 0.53. MS (EI): *m*/*z* (%) = 995.6 [$M^+$+ 2 H] (6), 994.6 [$M^+$+ H] (16), 993.6 [$M^+$] (22), 874.2 (9).

Beispiel 22: 1,6-Bis-benzamidyl-*N*,*N'*-bis(1-hexylheptyl)perylen-3,4:9,10-tetracarbonsäurebisimid und 1,7-Bis-benzamidyl-*N*,*N'*-bis(1-hexylheptyl)perylen-3,4:9,10-tetracarbonsäurebisimid (100 mg, 0.10 mmol) als Isomerengemisch und $NaNH_2$ (100 mg, 2.56 mmol) wurden in Benzonitril (250 mL) suspendiert, auf 165˚C erhitzt (Blaufärbung), nach 3 h abgekühlt und mit einem 1:1-Gemisch aus wäßriger HCl (2 N) und $CHCl_3$ (300 mL) ausgeschüttelt. Die organische Phase wurde eingedampft, im Feinvakuum vom Benzonitril befreit, in wenig $CHCl_3$ aufgenommen, filtriert und säulenchromatographisch (Kieselgel, $CHCl_3$) gereinigt. Der eluierte Farbstoff wurde in wenig Dichlormethan aufgenommen und mit Methanol gefällt. Ausb. 37 mg (35 %) schwarzer Farbstoff der Formel (9) und / oder (10).

Beispiel 23: 2,10-Bis(1-hexylheptyl)-(*N*-benzyl)-6-phenyl[1,3]diazepino[4',5',6':4,5]-phenanthro[2,1,10-*def*::7,8,9-*d'e'f'*]diisochinolin-1,3,9,11(*2H*,*5H*,*9H*,*11H*)-tetraon. 2,10-Bis(1-hexylheptyl)-6-phenyl[1,3]diazepino[4',5',6':4,5]phenanthro[2,1,10- *def*:7,8,9-*d'e'f'*]diisochinolin-1,3,9,11(*2H*,*5H*,*9H*,*11H*)-tetraon (85 mg, 0.098 mmol) wurde in DMF (5 mL) gelöst, auf 100˚C erhitzt, mit wasserfreiem Kaliumcarbonat (100 mg, 0.724 mmol) versetzt (Blaufärbung der vorher pinken Lösung), tropfenweise mit Benzylbromid (400 mg, 2.34 mmol) versetzt (Violettfärbung), 3 h bei 100 ˚C gerührt, durch Zugabe von 2 N HCl-Lösung gestoppt, abgesaugt, 16 h bei 110 ˚C getrocknet und säulenchromatographisch gereinigt (Kieselgel, Chloroform/*iso*-Hexan 3:1). Ausb. 26 mg (27.7 %) dunkel-violetter Feststoff der Formel (7), Schmp. >250˚C. $R_f$(Kieselgel/Chloroform) = 0.81. IR (ATR): $\tilde{\nu}$ = 3061.3 vw, 2953.6 m, 2922.4 vs, 2854.4 s, 2359.2 w, 2341.1 w, 1685.5 vs, 1641.8 vs, 1590.8 s, 1573.8 m, 1529.9 w, 1483.8 w, 1455.2, w, 1424.0 m, 1406.6 vw, 1378.0 w, 1357.6 w, 1330.2 vs, 1251.6 m, 1222.4 w, 1180.7 w, 1108.5 w, 1075.2 w, 1027.9 w, 873.3 w, 845.7 w, 809.0 m, 774.1 w, 751.3 w, 720.6 w, 699.0 m, 673.7 w $cm^{-1}$. $^1$H-NMR (150 MHz, $CDCl_3$, 25 ˚C): $\delta$ = 0.83 (t, $^3J$=6.9 Hz, 12 H, $CH_3$), 1.18-1.40 (m, 32 H, $CH_2$), 1.85-1.94 (m, 4 H, $CH_2$), 2.15 (s, 2 H, $CH_2$), 2.23-2.32 (m, 2 H, $CH_2$), 5.06-5.24 (m, 2 H, CH-N), 6.19 (s, 1 H, *N*-$CH_2$), 6.25 (s, 1 H, *N*-$CH_2$), 6.54-6.64 (br, 2 H, $CH_{aromat,benzyl),}$ 6.95-7.04 (br, 2 H, $CH_{aromat,benzyl}$), 7.67 (s, 3 H, $CH_{Phenyl}$), 8.06 (s, 2 H, $CH_{Phenyl}$), 8.58-8.82 (m, 5 H, $CH_{perylen}$), 10.84 ppm (s, 1 H, $CH_{perylen}$). $^{13}$C-NMR (150 MHz, $CDCl_3$, 25 ˚C): $\delta$ = 14.3, 14.3, 22.8, 22.8, 27.2, 27.3, 29.5, 29.6, 32.1, 32.0, 32.7, 54.9, 54.9, 55.1, 75.8, 121.5, 122.8, 125.6, 126.1, 126.5, 127.0, 128.8, 129.3, 129.4, 129.9, 130.6, 131.5, 135.0, 145.0, 164.0, 165.1 ppm. UV/Vis ($CHCl_3$): $\lambda_{max}$ ($E_{rel}$) = 395 (0.11), 435 (0.11), 500 (0.17), 537 (0.52), 582 (1.00). Fluoreszenz ($CHCl_3$): $\lambda_{max}$ ($I_{rel}$) = 593 (1.00), 647 nm (0.44). Fluoreszenzquantenausb. ($\lambda_{exc}$ = 490 nm, $E_{490\ nm}$ = 0.13671/1 cm, $CHCl_3$, Referenz (1a) mit $\Phi$ = 1.00): $\Phi$ = 1.00. MS (DEI$^+$, 70 eV): *m*/*z* (%): 964.4 (6.3), 963.3 (24.9), 962.3 (69.8), 961.3 (100.0) [$M^+$], 871.1 [$M^+$ -Benzyl], 780.7 (5.0), 779.7(11.4), 778.7 (11.3) [$M^+$-$C_{13}H_{26}$], 598.2 (6.1), 597.2 (18.8), 596.2 (28.6), 595.2 (14.9) [$M^+$- 2 $C_{13}H_{26}$].

Beispiel 24: 2,10-Bis(1-hexylheptyl)-(*N*-4-tert-butylbenzyl)-6-phenyl[1,3]diazepino-[4',5',6':4,5]phenanthro [2,1,10-*def*::7,8,9-*d'e'f'*]diisochinolin-1,3,9,11(*2H*,*5H*,*9H*,-*11H*)-tetraon. 2,10-Bis(1-hexylheptyl)-6-phenyl[1,3]diazepino [4',5',6':4,5]-phenanthro[2,1,10-*def*::7,8,9-*d'e'f'*]diisochinolin-1,3,9,11(*2H,5H,9H,11H*)-tetraon (85 mg, 0.098 mmol) wurde in DMF (5 mL) mit Kaliumcarbonat (100 mg, 0.724 mmol) und 4-*tert*-Butylbenzylbromid (400 mg, 1.76 mmol) wie bei 2,10-Bis(1-hexylheptyl)-(*N*-benzyl)-6-phenyl[1,3]diazepino[4',5',6':4,5]-phenanthro[2,1,10- *def*.:7,8,9-*d'e'f'*]diisochinolin-1,3,9,11(*2H,5H,9H,11H*)-tetraon umgesetzt und aufgearbeitet und säulenchromatographisch gereinigt (Kieselgel, Chloroform/*iso*-Hexan 2:1). Ausb. 64 mg (64.5 %) schwarz-violetter Feststoff der Formel (7), Schmp. > 250˚C. $R_f$(Kieselgel/Chloroform:*iso*-Hexan 2:1):0.40. IR (ATR): $\tilde{\nu}$ = 3058.1 vw, 2953.6 m, 2922.6 vs, 2854.5 s, 2360.5 w, 2340.9 w, 1687.1 vs, 1643.3 vs, 1591.3 s, 1573.9 m, 1529.7 w, 1483.8 w, 1463.0, w, 1424.0 m, 1407.4 vw, 1377.6 w, 1357.6 w, 1330.1 vs, 1251.9 m, 1223.0 w, 1179.4 w, 1107.2 w, 1025.1 w, 929.4 vw, 873.3 w, 844.9 w, 809.8 m, 774.1 w, 750.7 w, 719.5 w, 700.8 m, 673.0 w $cm^{-1}$. $^1$H-NMR (600 MHz, $CDCl_3$, 25˚C): $\delta$ = 0.81 (t, $^3J$=7.0 Hz, 12 H, $CH_3$), 1.07 (s, 9 H, *tert*-Butyl-$CH_3$), 1.16-1.40 (m, 32 H, $CH_2$), 1.83-1.97 (m, 4 H, $CH_2$), 2.12-2.31 (m, 4 H, $CH_2$), 5.13-5.23 (m, 2 H, CH-N),

6.14 (s, 1 H, *N*-$CH_2$), 6.19 (s, 1 H, *N*-$CH_2$), 6.46-6-61 (br, 2 H, $CH_{aromat,benzyl}$), 7.00 (d, $^3J$=8.3 Hz, 2 H, $CH_{aromat,benzyl}$), 7.65 (s, 3H, $CH_{Phenyl}$), 8.06 (s, 2H, $CH_{Phenyl}$), 8.47-8.79 (m, 5H, $CH_{perylen}$), 10.80 ppm (s, 1H, $CH_{perylen}$). $^{13}$C-NMR (150 MHz, $CDCl_3$, 25 ˚C): δ = 14.3, 14.3, 22.8, 22.9, 27.2, 27.5, 29.5, 31.3, 32.0, 32.1, 32.7, 34.6, 54.9, 76.6, 121.3, 122.7, 123.8, 125.5, 125.7, 126.1, 126.7, 126.9, 129.2, 129.5, 130.0, 130.7, 131.4, 134.8, 139.9, 145.0, 164.0, 165.1 ppm. UV/Vis ($CHCl_3$): $\lambda_{max}$ ($E_{rel}$) = 395 (0.11), 436 (0.12), 501 (0.17), 538 (0.52), 582 (1.00). Fluoreszenz ($CHCl_3$): $\lambda_{max}$ ($I_{rel}$) = 595 (1.00), 648 nm (0.45). Fluoreszenzquantenausb. ($\lambda_{exc}$ = 490 nm, $E_{490\ nm}$ = 0.013671/1 cm, $CHCl_3$, Referenz (2a) mit Φ = 1.00): 1.00. MS (DEI$^+$, 70 eV): *m/z* (%): 1020.6 (7.5), 1019.6 (28.7), 1018.6 (73.0), 1017.6 (100.0) [$M^+$], 872.1 (9.0), 871.1 (15.4) [$M^+$ -Benzyl], 835.9 (8.6), 834.9 (9.0) [$M^+$-$C_{13}H_{26}$], 653.4 (6.7), 652.4 (11.2) [$M^+$- 2 $C_{13}H_{26}$], 651.4 (6.6). $C_{68}H_{80}N_4O_4$ (1017,39): Ber. C 80.28; H 7.93; N 5.51; Gef. C 79.90; H 7.38; N 5.39.

Beispiel 25: Bichromophor aus Monoaddukt und Perylenbisimid. 2,10-Bis(1-hexylheptyl)-6-phenyl[1,3]diazepino[4',5',6':4,5]phenanthro[2,1,10-*def*::7,8,9-*d'e'f*]-diisochinolin-1,3,9,11(*2H,5H,9H,11H*)-tetraon (100 mg, 0.115 mmol) wurde in DMF (5 mL) mit Kaliumcarbonat (100 mg, 0.724 mmol) und Perylen-benzylbromid (400 mg, 0.529 mmol) wie bei 2,10-Bis(1-hexylheptyl)-(*N*-benzyl)-6-phenyl[1,3]-diazepino[4',5',6':4,5]-phenanthro[2,1,10-*def*::7,8,9-*d'e'f*]diisochinolin-1,3,9,11-(*2H,5H,9H,11H*)-tetraon umgesetzt und aufgearbeitet und säulenchromatographisch gereinigt (Kieselgel, Chloroform). Ausb. 96 mg (54.1 %) der Formel (7), Schmp. > 250˚C. $R_f$(Kieselgel/Chloroform): 0.40. UV/Vis ($CHCl_3$): $\lambda_{max}$ ($E_{rel}$) = 391 (0.11), 431 (0.14), 460 (0.25), 492 (0.56), 528 (1.00), 582 (0.73). Fluoreszenz ($CHCl_3$): $\lambda_{max}$ ($I_{rel}$ = 594 (1.00), 645 (0.45), 709 nm (0.10). Fluoreszenzquantenausb. ($CHCl_3$, $\lambda_{exc}$ = 582 nm, $E_{582}$ nm = 0.02399 cm$^{-1}$, Referenz: (1a) mit 1.00): 1.00. Fluoreszenzquantenausb. ($CHCl_3$, $\lambda_{exc}$ = 528 nm, $E_{528}$ nm = 0.03272 cm$^{-1}$, Referenz: (1a) mit 1.00): 0.80. MS (FAB$^+$): *m/z*(%): 1544.4 (0.1) [$M^+$- H], 871.5 (0.2) [$M^+$- $C_{45}H_{62}N_2O_4$], 675.4 (0.7) [$M^+$- $C_{57}H_{66}N_2O_4$], 460.3, 391.4, 307.3.

Beispiel 26: 2,10-Bis(1-hexylheptyl)-(*N*-butyl)-6-phenyl[1,3]diazepino[4',5',6':4,5]-phenanthro[2,1,10-*def*::7,8,9-d'e'f]diisochinolin-1,3,9,11(*2H,5H,9H,11H*)-tetraon. 2,10-Bis(1-hexylheptyl)-6-phenyl[1,3]diazepino[4',5',6':4,5] phenanthro[2,1,10-*def*::7,8,9-*d'e'f'*]diisochinolin-1,3,9,11(*2H,5H,9H,11H*)-tetraon (100 mg, 0.115 mmol), 1-Iodbutan (635 mg, 3.45 mmol) und Kaliumcarbonat (120 mg, 0.868 mmol) wurden in DMF (5 mL) wie bei 2,10-Bis(1-hexylheptyl)-(*N*-benzyl)-6-phenyl-[1,3]diazepino[4',5',6':4,5]-phenanthro[2,1,10-*def*::7,8,9-*d'e'f*]diisochinolin-1,3,9,11(*2H,5H, 9H,11H*)-tetraon umgesetzt und aufgearbeitet und säulenchromatographisch gereinigt (Kieselgel, Chloroform). Ausb. 48 mg (45 %) dunkelrot bis schwarzer Feststoff der Formel (7), Schmp. > 250˚C. $R_f$(Kieselgel/Chloroform): 0.76. IR (ATR): $\tilde{\nu}$ = 3062.8 w, 2954.3 m, 2922.6 s, 2855.1 s, 1686.5 s, 1644.5 s, 1591.3 m, 1574.4 m, 1529.3 w, 1484.2 w, 1424.0 m, 1408.1 w, 1378.6 w, 1330.3 s, 1252.4 m, 1221.3 w, 1178.6 w, 1101.3 w, 1025.7 w, 874.5 w, 841.9 w, 808.0 m, 772.1 w, 750.0 w, 587.6 w cm$^{-1}$. $^1$H-NMR (600 MHz, $CDCl_3$, 25 ˚C): δ = 0.56 (t, $^3J$ = 12.0 Hz, 3 H, $CH_3$), 0.80-0.82 (m, 12 H, $CH_3$), 1.22-1.31 (m, 32 H, $CH_2$), 1.84-1.91 (m, 4 H, β-CH), 2.29-2.24 (m, 4 H, β-CH), 4.97 (t, $^3J$ = 12.0 Hz, 2 H, *N*-$CH_2$), 5.16-5.24 (m, 2 H, *N*-CH), 7.67-7.68 (m, 3 H, $CH_{aryl}$), 7.99-8.01 (m, 2 H, $CH_{aryl}$), 8.71-8.74 (m, 5 H, $CH_{perylen}$), 10.82 ppm (d, $^3J$=12.0 Hz, 1 H, $CH_{perylen}$). UV/Vis ($CHCl_3$): $\lambda_{max}$ ($E_{rel}$) = 376.6 (0.79), 395.2 (0.09), 438.8 (0.11), 502.4 (0.16), 539.2 (0.52), 583.8 nm (1.00). Fluoreszenz ($CHCl_3$): $\lambda_{max}$ ($I_{rel}$) = 596.2 (1.00), 647.5 nm (0.45). Fluoreszenzquantenausb. ($CHCl_3$, $\lambda_{exc}$ = 539 nm, $E_{539\ nm}$ = 0.01647 cm$^{-1}$, Referenz: (1a) mit Φ = 1.00): 1.00. HRMS ($C_{68}H_{80}N_4O_4$): Ber. *m/z* = 926.5710, Gef. *m/z* = 926.5721, Δ = 0.0011.

Beispiel 27: 2,10-Bis(1-hexylheptyl)-(*N*-pentyl)-6-phenyl[1,3]diazepino[4',5',6':4,5]-phenanthro[2,1,10-*def*::7,8,9-*d'e'f*]diisochinolin-1,3,9,11(*2H,5H, 9H,11H*)-tetraon. 2,10-Bis(1-hexylheptyl)-6-phenyl[1,3]diazepino[4',5',6':4,5] phenanthro[2,1,10-*def*::7,8,9-*d'e'f'*]diisochinolin-1,3,9,11(*2H,5H,9H,11H*)-tetraon (100 mg, 0.115 mmol), 1-Iodpentan (683 mg, 3.45 mmol) und Kaliumcarbonat (120 mg, 0.868 mmol) wurden in DMF (5 mL) wie bei 2,10-Bis(1-hexylheptyl)-(*N*-benzyl)-6-phenyl[1,3]diazepino[4',5',6':4,5]-phenanthro[2,1,10-*def*::7,8,9-*d'e'f'*]diisochinolin-1,3,9,11(*2H,5H, 9H,11H*)-tetraon umgesetzt und aufgearbeitet, zur Entfernung von nicht regiertem 1-Iodpentan säulenchromatographisch (Kieselgel, Chloroform) gereinigt, in Toluol gelöst und mit einem Überschuss an DBU versetzt und säulenchromatographisch (Aluminiumoxid basisch, Toluol, das deprotonierte Ausgangsmaterial wird fest adsorbiert) gereinigt. Ausb. 75 mg (69 %) dunkelrot bis schwarzer Feststoff der Formel (7).

Beispiel 28: 2,10-Bis(1-hexylheptyl)-(*N*-pentyl)-6-phenyl[1,3]diazepino[4',5',6':4,5]-phenanthro[2,1,10-*def*::7,8,9-*d'e'f*]diisochinolin-1,3,9,11 (*2H,5H,9H,11H*)-tetraon (Variante zu Bsp. 27). 2,10-Bis(1-hexylheptyl)-6-phenyl[1,3] diazepino[4',5',6':4,5]-phenanthro[2,1,10-*def*::7,8,9-*d'e'f'*]diisochinolin-1,3,9,11 (*2H,5H, 9H,11H*)-tetraon (100 mg, 0.115 mmol), 1-Iodpentan (683 mg, 3.45 mmol) und Kaliumcarbonat (120 mg, 0.868 mmol) wurden in DMPU (5 mL) suspendiert, 6 h auf 100˚C erhitzt, noch lauwarm mit wäßriger HCl (2 N, 50 mL, ausgefallener Farbstoff als rote Suspension) versetzte, abgesaugt, getrocknet, in Chloroform aufgenommen und zur Entfernung von nicht regiertem 1-Iodpentan säulenchromatographisch (Kieselgel, Chloroform) gereinigt. Ausb. 90 mg (83 %), Schmp. > 250˚C. $R_f$ (Kieselgel/Chloroform): 0.59. IR (ATR): $\tilde{\nu}$ = 3061.2 w, 2954.1 m, 2922.7 s, 2854.7 s, 1686.7 s, 1644.5 s, 1591.1 m, 1529.6 w, 1484.3 w, 1454.7 w, 1424.7 w, 1408.1 w, 1378.5 w, 1331.0 s, 1251.8 m, 1222.8 w, 1179.2 w, 1101.9 w, 1025.9 w, 929.9 w, 874.0 w, 842.2 w, 808.4 w, 771.9 w 749.8 w cm$^{-1}$. $^1$H-NMR (600 MHz, $CDCl_3$, 25˚C): δ = 0.57 (t, $^3J$=7.3 Hz, 3 H, C$H_3$), 0.66-0.75 (br, 2 H, $CH_2$), 0.78-0.83 (m, 12 H, C$H_3$), 0.88-0.96 (m, 2 H, C$H_2$), 1.15-1.43 (m, 32 H, C$H_2$), 1.82-1.95 (m, 4 H, β - CH), 2.21-2.40 (m, 4 H, β-C*H*), 4.93 (t, $^3J$=6.8 Hz, 2 H, *N*-C$H_2$), 5.15-5.30 (m, 2 H, *N*-C*H*), 7.66-7.67 (m, 3 H, C$H_{aryl}$), 7.98-7.99 (m, 2 H, C$H_{aryl}$), 8.66-8.73 (m, 5 H, C$H_{perylen}$), 10.77 ppm (d, $^3J$ = 6 Hz, 1 H, C$H_{perylen}$). $^{13}$C-NMR

(150 MHz, $CDCl_3$, 25˚C): δ = 13.9, 14.3, 22.2, 22.8, 22.9, 27.2, 27.4, 28.4, 28.7, 29.5, 29.6, 32.0, 32.0, 32.7, 49.4, 54.8, 121.5, 122.8, 123.9, 127.0, 127.3, 129.3, 130.2, 130.5, 131.3, 131.6, 135.1, 145.1, 163.9 164.1, 165.2 ppm. UV/ViS ($CHCl_3$):$\lambda_{max}$ ($E_{rel}$) = 377.2 (0.09), 396.0 (0.10), 440.2 (0.11), 502.8 (0.16), 539.8 (0.52), 584.2 nm (1.00). Fluoreszenz ($CHCl_3$): $\lambda_{max}$ ($I_{rel}$) = 596.6 (1.00), 650.1 nm (0.45). Fluoreszenzquantenausb. ($CHCl_3$, $\lambda_{exc}$ = 540 nm, $E_{540}$ nm = 0.01462 $cm^{-1}$, Referenz: (1a) mit 1.00): 1.00, HRMS ($C_{62}H_{78}N_4O_4$): Ber. *m*/*z* = 941.5900, Gef. *m*/*z* = 941.5924, Δ = 0.0024. $C_{62}H_{78}N_4O_4$ (961.3): Ber. C 79.11; H 5.95; N 8.14; Gef. C 78.99; H 5.91; N 8.51.

Beispiel 29: 2,10-Bis(1-hexylheptyl)-(*N*-hexyl)-6-phenyl[1,3]diazepino[4',5',6':4,5]-phenanthro[2,1,10-*def*::7,8,9-*d'e'f'*]diisochinolin-1,3,9,11(*2H,5H,9H,11H*)-tetraon. 2,10-Bis(1-hexylheptyl)-6-phenyl[1,3]diazepino[4',5',6':4,5]phenanthro[2,1,10-*def*::-7,8,9-*d'e'f'*]diisochinolin-1,3,9,11(*2H,5H,9H,11H*)-tetraon (100 mg, 0.115 mmol), 1-Bromhexan (570 mg, 3.45 mmol) und Kaliumcarbonat (300 mg, 2.17 mmol) wurden in DMPU (5 mL) suspendiert, 3 h auf 100˚C erhitzt, noch lauwarm mit wässriger HCl (2 N, 50 mL) versetzt, dreimal mit Chloroform (je 50 mL) extrahiert, über Magnesiumsulfat getrocknet, eingedampft, in wenig Chloroform aufgenommen und zur Entfernung von nicht regiertem 1-Bromhexan säulenchromatographisch (Kieselgel, Chloroform/ *iso*-Hexan 3:1) gereinigt. Ausb. 86 mg (78%) der Formel (7), Schmp. > 250˚C. $R_f$(Kieselgel, Chloroform): 0.59. $^1$H-NMR (600 MHz, $CDCl_3$, 25 ˚C): δ = 0.60 (t, $^3J$=7.3 Hz, 3 H, $CH_3$), 0.67-0.75 (br, 2 H, $CH_2$), 0.76-0.82 (m, 12 H, $CH_3$), 0.83-0.90 (m, 2 H, $CH_2$), 0.92-01.10 (m, 2 H, $CH_2$), 1.16-1.44 (m, 34 H, $CH_2$), 1.82-1.96 (m, 4 H, β -C*H*), 2.20-2.40 (m, 4 H, β-C*H*), 4.93 (t, $^3J$=6.7 Hz, 2 H, *N*-$CH_2$), 5.15-5.31 (m, 2 H, *N-CH*), 7.61-7.70 (m, 3 H, $CH_{aryl}$), 7.96-8.01 (m, 2 H, $CH_{aryl}$), 8.60-8.82 (m, 5 H, $CH_{perylen}$), 10.76 ppm (d, $^3J$ = 8.04 Hz, 1 H, $CH_{perylen}$). $^{13}$C-NMR (150 MHz, $CDCl_3$, 25˚C): δ = 13.9, 14.3, 22.5, 22.8, 22.9, 25.9, 27.2, 27.4, 28.9, 29.5, 29.6, 31.2, 32.0, 32.1, 32.7, 49.4, 54.8, 121.4, 122.8, 123.8, 127.0, 127.3, 129.3, 130.2, 130.5, 131.3, 131.6, 145.1, 163.9 ppm. UV/Vis ($CHCl_3$): $\lambda_{max}$ ($E_{rel}$) = 377.1 (0.11), 394.7 (0.12), 500.9 (0.18), 539.0 (0.53), 583.7 nm (1.00). Fluoreszenz ($CHCl_3$): $\lambda_{max}$ ($I_{rel}$) = 596.8 (1.00), 650.1 nm (0.45). Fluoreszenzquantenausb. ($CHCl_3$, $\lambda_{exc}$ = 539 nm, $E_{539}$ nm = 0.01671 $cm^{-1}$, Referenz: (1a) mit 1.00): 1.00.

Beispiel 30: 2,10-Bis(1-hexylheptyl)-(*N*-allyl)-6-phenyl[1,3]diazepino[4',5',6':4,5]-phenanthro[2,1,10-*def*::7,8,9-*d'e'f'*]diisochinolin-1,3,9,11 (*2H,5H,9H,11H*)-tetraon. 2,10-Bis(1-hexylheptyl)-6-phenyl[1,3]diazepino[4',5',6':4,5]phenanthro[2,1,10-*def*::-7,8,9-*d'e'f'*]diisochinolin-1,3,9,11(*2H,5H,9H,11H*)-tetraon (100 mg, 0.115 mmol), Allylbromid (624 mg, 3.45 mmol) und Kaliumcarbonat (300 mg, 2.17 mmol) wurden in DMPU (5 mL) suspendiert, 24 h auf 65˚C erhitzt, noch lauwarm mit wäßriger HCl (2 N, 50 mL) versetzt, abgesaugt, getrocknet, in wenig Chloroform aufgenommen und zur Entfernung von nicht regiertem Allylbromid säulenchromatographisch (Kieselgel, Chloroform/*iso*-Hexan 2:1) gereinigt. Ausb. 87 mg (83 %) der Formel (7), Schmp. > 250˚C. $R_f$(Kieselgel, Chloroform): 0.52. $^1$H-NMR (600 MHz, $CDCl_3$, 25˚C): δ = 0.82 (t, $^3J$=7.0 Hz, 12 H, $CH_3$), 1.16-1.40 (m, 32 H, $CH_2$), 1.82-1.92 (m, 4 H, β -CH), 2.20-2.34 (m, 4 H, β-C*H*), 4.44-4.64 (br, 1 H, $CH_{allyl}$), 4.81-4.88 ( br, 1 H, $CH_{allyl}$), 5.15-5.22 (m, 2 H, *N*-$CH_2$), 5.42-5.60 (br, 3 H, *N-CH* + $CH_{allyl}$), 7.64-7.68 (m, 3 H, $CH_{aryl}$), 7.95-8.00 (m, 2 H, $CH_{aryl}$), 8.60-8.79 (m, 5 H, $CH_{perylen}$), 10.75 ppm (d, $^3J$ = 8.1 Hz, 1 H, $CH_{perylen}$). $^{13}$C-NMR (150 MHz, $CDCl_3$, 25˚C): δ = 14.3, 22.8, 22.8, 27.2, 27.3, 29.5, 29.5, 32.0, 32.1, 32.7, 51.3, 54.9, 55.3, 121.5, 122.8, 123.9, 125.6, 127.0, 127.3, 129.3, 129.9, 130.4, 130.4, 131.5, 132.5, 135.0, 139.6, 144.8, 163.4, 164.1, 165.1 ppm. UV/Vis ($CHCl_3$): $\lambda_{max}$ (ε) = 376.0 (8067), 395.4 (8978), 500.3 (14530), 536.9 (43870), 581.3 nm (82740). Fluoreszenz ($CHCl_3$): $\lambda_{max}$ ($I_{rel}$) = 594.6 (1.00), 644.2 nm (0.32). Fluoreszenzquantenausb. ($CHCl_3$, $\lambda_{exc}$ = 537.5 nm, $E_{537.5}$ nm = 0.01405 $cm^{-1}$, Referenz: (1a) mit Φ = 1.00): 1.00. HRMS ($C_{60}H_{70}N_4O_4$): Ber. *m*/*z* = 910.5397, Gef. *m*/*z* = 910.5379, Δ=-0.0018. $C_{60}H_{70}N_4O_4$ (911.2): Ber. C 79.09, H 7.74, N 6.15; Gef. C 78.87, H 7.71, N 5.83.

Beispiel 31: 2,10-Bis(1-hexylheptyl)-(*N*-propargyl)-6-phenyl[1,3]diazepino[4',5',6':-4,5]-phenanthro[2,1,10-*def*::7,8,9-*d'e'f'*]diisochinolin-1,3,9,11(*2H,5H, 9H,11H*)-tetraon. 2,10-Bis(1-hexylheptyl)-6-phenyl[1,3]diazepino[4',5',6':4,5]phenanthro[2,1,10-*def*::7,8,9-*d'e'f'*]diisochinolin-1,3,9,11(*2H,5H,9H,11H*)-tetraon (300 mg, 0.344 mmol), Propargylbromid (1.53 g, 10.3 mmol, 80 proz. in Toluol) und Kaliumcarbonat (900 mg, 6.517 mmol) wurden in DMPU (15 mL) suspendiert, 24 h auf 85˚C erhitzt, eingedampft, mit wässriger HCl (2 N, 150 mL) versetzt, abgesaugt, getrocknet, in wenig Chloroform aufgenommen und zur Entfernung von nicht regiertem Allylbromid säulenchromatographisch (Kieselgel, Chloroform/*iso*-Hexan 3:1) gereinigt. Ausb. 212 mg (67.7%) der Formel (7), Schmp. > 250˚C. $R_f$ (Kieselgel, Chloroform): 0.45. $^1$H-NMR (600 MHz, $CDCl_3$, 25˚C): δ = 0.82 (t, $^3J$=6.8 Hz, 12 H, $CH_3$), 1.15-1.44 (m, 32 H, $CH_2$), 1.82-1.95 (m, 4 H, β-C*H*), 1.99 (s, 1H, C≡C*H*), 2.21-2.36 (m, 4 H, β-C*H*), 5.14-5.30 (m, 2H, *N*-$CH_2$), 5.67 (s, 2H, $CH_{propargyl}$ ), 7.65-7.75 (m, 3 H, $CH_{aryl}$), 7.99-8.03 (m, 2 H, $CH_{aryl}$), 8.53-8.78 (m, 5 H, $CH_{perylen}$), 10.68 ppm (d, $^3J$ = 8.0 Hz, 1 H, $CH_{perylen}$). $^{13}$C-NMR (150 MHz, $CDCl_3$, 25 ˚C): δ = 14.3, 14.3, 22.7, 22.8, 22.9, 27.2, 27.4, 29.5, 29.6, 29.9, 32.0, 32.1, 32.7, 32.8, 39.7, 54.9, 55.4, 121.6, 122.5, 122.8, 123.1, 124.0, 125.6, 126.9, 127.3, 129.1, 129.3, 129.5, 130.5, 131.4, 131.8, 132.6, 134.9, 139.1, 144.7, 162.9, 164.0, 165.1 ppm. UV/Vis ($CHCl_3$): $\lambda_{max}$ ($E_{rel}$) = 375.2 (0.10), 395.3 (0.11), 434.6 (0.11), 498.4 (0.18), 534.8 (0.54), 579.0 nm (1.00). Fluoreszenz ($CHCl_3$): $\lambda_{max}$ ($I_{rel}$) = 591.6 (1.00), 643.0 nm (0.47). Fluoreszenzquantenausb. ($CHCl_3$, $\lambda_{exc}$ = 543 nm, $E_{537.5\ nm}$ = 0.008991 $cm^{-1}$, Referenz: (1a) mit 1.00): 0.17. HRMS ($C_{60}H_{68}N_4O_4$): Ber. *m*/*z* 908.5241, Gef. *m*/*z* 908.5216, Δ = - 0.0025. $C_{60}H_{68}N_4O_4$ (909.2): Ber. C 79.26, H 7.54, N 6.16; Gef. C 79.13, H 7.62, N 6.14.

Beispiel 32: 2,9-Bis(1-hexylheptyl)-bis-[1,3]diazepino[4',5',6':1,12;4",5", 6":6,7]-perylo[3,4-*cd*:9,10-*c'd'*]dipyridin-1,3,9,11(*2H,5H,10H,13H*)-tetraon. 2,9-Bis(1-hexylheptyl)-bis-[1,3]diazepino[4',5',6':1,12;4",5",6":6,7]perylo[3,4-*cd*:9,10-*c'd'*]dipyridin-1,3,9,11(*2H,5H,10H,13H*)-tetraon gemäss Beispiel 22 (90 mg, 0.091 mmol), *p-tert*-Butylbenzylbromid

(800 mg, 3.52 mmol), Kaliumcarbonat (600 mg, 4.34 mmol) wurden in 10 mL DMPU suspendiert, 6 h auf 100˚C erhitzt, noch warm mit wässriger HCl-Lösung (2 N, 50 mL) versetzt, dreimal mit Chloroform ausgeschüttelt, über Magnesiumsulfat getrocknet, eingedampft, und säulenchromatographisch (Kieselgel, Chloroform/*iso*-Hexan 3:1) gereinigt. Ausb. 72 mg (62%) schwarzer Feststoff der Formel (9) und (10), Schmp. >250˚C. $R_f$ (Kieselgel, Chloroform; *iso*-Hexan 3:1) = 0.62. $^1$H-NMR (600 MHz, $CDCl_3$, 25 ˚C, *cis-Produkt*): $\delta$ = 0.82 (q, $^3J$ = 7.04 Hz, 12 H, $CH_3$), 1.05 (s, 18 H, $CH_{3,tert\text{-}Butyl}$), 1.17-1.40 (m, 32 H, $CH_2$), 1.82-1.91 (m, 2 H, $CH_2$), 1.97-2.12 (m, 4 H, $CH_2$), 2.23-2.33 (m, 2 H, $CH_2$), 5.10-5.26 (m, 2 H, CH-N), 6.12 (s, 6 H, *cis-N*-$CH_2$), 6.44 (d, $^3J$ = 7.7 Hz, 2 H, *cis*-$CH_{aromatisch}$), 6.90-6.96 (br, 2 H, $CH_{aromat}$), 7.57-7.67 (br, 6 H, $CH_{aromat}$), 7.94-8.07 (br, 4 H, $CH_{aromat}$), 8.73-8.83 (br, 2 H, $CH_{perylen}$), 10.84 ppm (d, $^3J$ = 7.64 Hz, 2 H, $CH_{perylen}$). $^1$H-NMR (600 MHz, $CDCl_3$, 25 ˚C, *trans-Produkt*): $\delta$ = 0.82 (q, $^3J$ = 7.04 Hz, 12 H, $CH_3$), 1.05 (s, 18 H, $CH_{3,tert\text{-}Butyl}$), 1.17-1.40 (m, 32 H, $CH_2$), 1.82-1.91 (m, 2 H, $CH_2$), 1.97-2.12 (m, 4 H, $CH_2$), 2.23-2.33 (m, 2 H, $CH_2$), 5.10-5.26 (m, 2 H, CH-N), 6.06 (s, 6 H, *trans-N*-$CH_2$), 6.37 (d, $^3J$ = 7.7 Hz, 2 H, *trans*-$CH_{aromatisch}$), 6.90-6.96 (br, 2 H, $CH_{aromat}$), 7.57-7.67 (br, 6 H, $CH_{aromat}$), 7.94-8.07 (br, 4 H, $CH_{aromat}$), 8.73-8.83 (br, 2 H, $CH_{perylen}$), 10.84 ppm (d, $^3J$ = 7.64 Hz, 2 H, $CH_{perylen}$). $^{13}$C-NMR (150 MHz, $CDCl_3$, 25˚C): $\delta$ = 14.3, 14.3, 22.8, 23.0, 27.2, 27.8, 29.5, 29.7, 31.3, 31.3, 32.0, 32.1, 32.7, 33.0, 34.5, 51.7, 51.8,, 54.7, 55.8, 76.9, 108.4, 108.8, 121.9, 125.3, 125.6, 125.8, 126.1, 126.3, 127.5, 128.8, 129.3, 130.2, 130.3, 130.5, 131.2, 131.4, 131.9, 133.9, 134.0, 135.1, 139.7, 140.0, 143.7, 150.4, 150.6, 163.1, 163.7, 163.8, 164.1, 164.4 ppm. UV/ViS ($CHCl_3$): $\lambda_{max}$ ($E_{rel}$) = 383.6 (0.11), 404.2 (0.13), 465.4 (0.18), 488.8 (0.19), 535.0 (0.11), 577.4 (0.41), 629.8 (1.00). Fluoreszenz ($CHCl_3$): $\lambda_{max}$ ($I_{rel}$) = 642.3 (1.00), 701.9 nm (0.34). Fluoreszenzquantenausb.($CHCl_3$, $\lambda_{exc}$ = 577 nm, $E_{577\ nm}$ = 0.014317 $cm^{-1}$, Referenz: (1a) mit 1.00): 1.00. MS (DEI$^+$, 70 eV): *m*/*z* (%) = 1281.3 (16.4), 1280.3 (48.7), 1279.3 (96.0), 1278.3 (100.0) [$M^+$], 1134.3 (3.3), 1133.3 (8.4), 1132.3 (11.5) [$M^+$ -p-*tert*-Butylbenzyl], 1098.2 (2.7), 1097.2 (4.6), 1096.2 (4.2) [$M^+$ - $C_{13}H_{26}$]. $C_{86}H_{98}N_6O_4$ (1279.7): Ber. C 80.71, H 7.72, N 6.57; Gef. C 80.35, H 7.84 ,N 6.46.

**Patentansprüche**

1. Verbindung der Formel

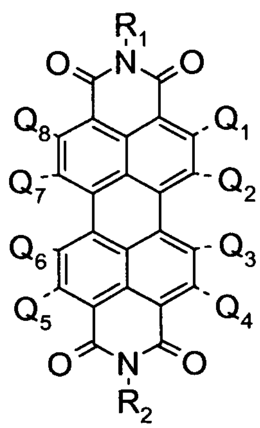

(X),

worin ein Paar $Q_1$ und $Q_2$ oder ein Paar $Q_2$ und $Q_3$, sowie gegebenenfalls von 0 bis 3 Paare $Q_3$ und $Q_4$, $Q_5$ und $Q_6$, $Q_6$ und $Q_7$, und/oder $Q_7$ und $Q_8$ je paarweise zusammen einen heterozyklischen Ring

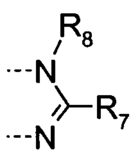

oder

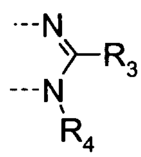

bilden, die restlichen, keinen heterozyklischen Ring bildenden $Q_1$, $Q_4$, $Q_5$ und $Q_8$ Wasserstoff sind, und die restlichen, keinen heterozyklischen Ring bildenden $Q_3$, $Q_6$ und $Q_7$ unabhängig voneinander $R_5$ oder $R_6$ sind, bevorzugt

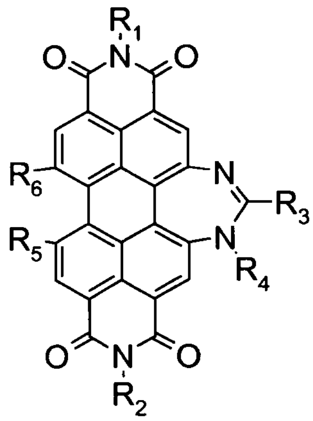
(5),
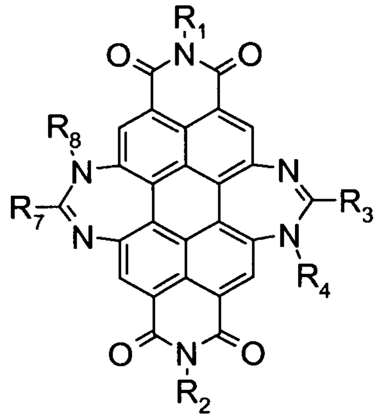
(6),

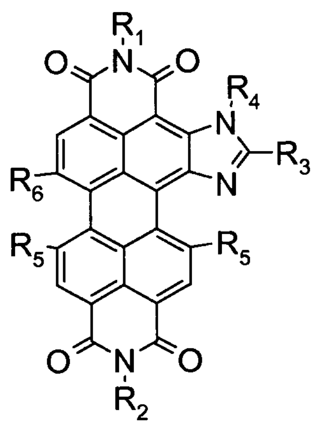
(7),
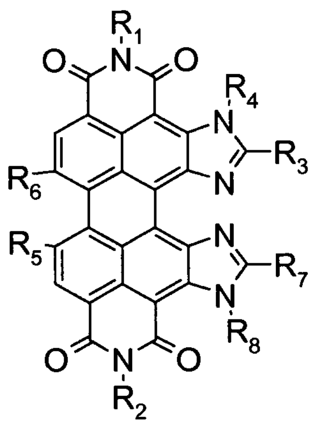
(8),
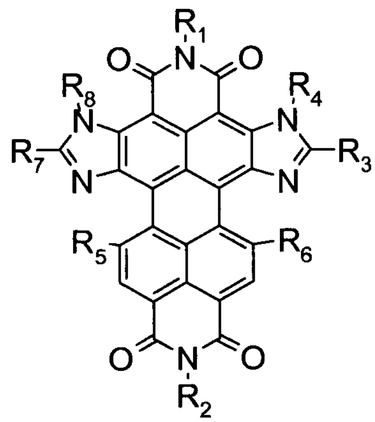
(9),

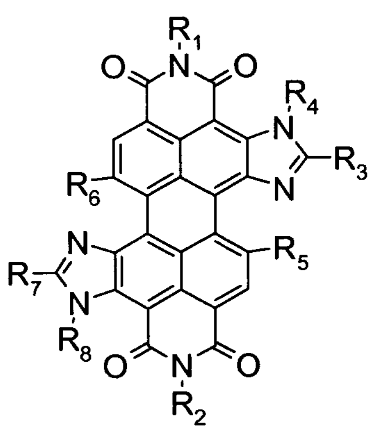
(10),
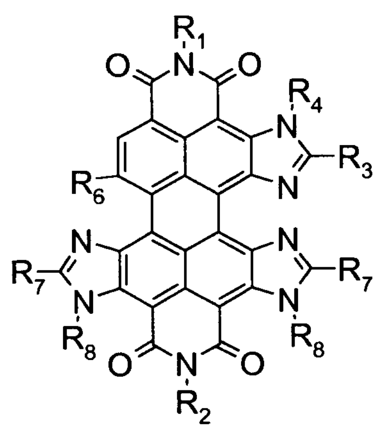
(11),

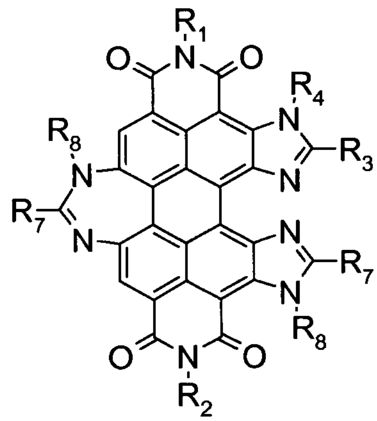

(12)

oder

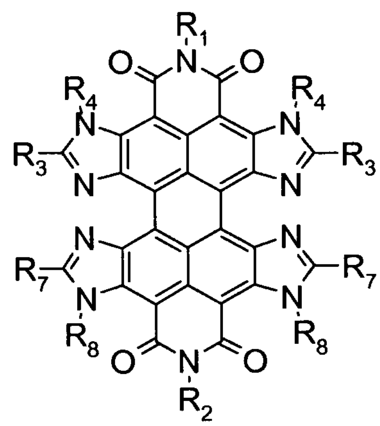

(13),

worin

$R_1$ bis $R_8$ unabhängig voneinander für H, F, Cl, Br, J oder {linear $C_1$-$C_{37}$Alkyl}(-$R_9$)$_m$ stehen, m eine Zahl von 0 bis 12 ist,

$R_9$, und bei mehreren $R_9$ jedes $R_9$ unabhängig von allen anderen $R_9$, für H, F, Cl, Br, J, CN oder {linear $C_1$-$C_{18}$Alkyl} (-$R_{10}$)$_n$ steht, n eine Zahl von 0 bis 6 ist,

$R_{10}$, und bei mehreren $R_{10}$ jedes $R_{10}$ unabhängig von allen anderen $R_{10}$, für H, F, Cl, Br, J, CN oder {linear $C_1$-$C_{18}$Alkyl} steht,

wobei in jedem {linear $C_1$-$C_{37}$Alkyl}, gegebenenfalls unabhängig von allen anderen {linear $C_1$-$C_{37}$Alkyl}, keine oder von 1 bis 10 -$CH_2$- Einheiten durch $R_{11}$ ersetzt sein können, und / oder in {linear $C_1$-$C_{18}$Alkyl}, und bei mehreren {linear $C_1$-$C_{18}$Alkyl} in jedem {linear $C_1$-$C_{18}$Alkyl} unabhängig von allen anderen {linear $C_1$-$C_{18}$Alkyl}, keine oder von 1 bis 6 -$CH_2$- Einheiten durch $R_{12}$ ersetzt sein können, und

$R_{11}$ und $R_{12}$ unabhängig voneinander, und bei mehreren $R_{11}$ und $R_{12}$ jedes $R_{11}$ oder $R_{12}$ unabhängig von allen anderen $R_{11}$ und $R_{12}$, für -CO-, -O-, -S-, -Se-, -Te-, cis- oder trans- -CH=CH-, cis- oder trans- -N=CH-, -C≡C-, 1,2-, 1,3- oder 1,4-Phenylen, 2,3-, 2,4-, 2,5-, 2,6-, 3,4- oder 3,5-Pyridindiyl, 2,3-, 2,4-, 2,5- oder 3,4-Thiophendiyl, 1,2-, 1,3-, 1,4-, 1,5-, 1,6-, 1,7-, 1,8-, 2,3-, 2,6- oder 2,7-Naphthylen, worin 0, 1 oder 2 =CH- durch =N- ersetzt sein können, oder 1,2-, 1,3-, 1,4-, 1,5-, 1,6-, 1,7-, 1,8-, 1,9-, 1,10-, 2,3-, 2,6-, 2,7-, 2,9-, 2,10- oder 9,10-disubstituierte Anthracylen, worin 0, 1 oder 2 =CH- durch =N- ersetzt sein können, stehen,

oder zwei beliebige Substituenten $R_9$, $R_{10}$, $R_{11}$ und/oder $R_{12}$ bilden ein- oder mehrfach paarweise zusammen eine Direktbindung, so dass Ringe entstehen, bevorzugt Cyclohexan- oder Benzolringe,

wobei fakultativ $R_4$ durch eine Direktbindung mit $R_4$ eines zweiten Moleküls, und / oder $R_8$ durch eine Direktbindung mit $R_8$ eines zweiten Moleküls gebunden sein können, so dass Dimere, Trimere, Tetramere oder höhere Oligomere entstehen.

2. Verbindung gemäss Anspruch 1, worin {linear $C_1$-$C_{37}$Alkyl}(-$R_9$)$_m$ ein unsubstituiertes oder substituiertes Kohlenwasserstoffrest bedeutet, welcher von 1 bis 60, bevorzugt von 1 bis 37, besonders bevorzugt von 1 bis 18 Kohlenstoffatome enthält, wobei dieser Kohlenwasserstoffrest bei $R_3$ und/oder $R_8$ bevorzugt durch dreifache Substitution von $-CH_2-$ mit -CH=CH- und einer Direktbindung zwischen 2 Substituenten $R_9$ ein Phenylradikal bildet.

**3.** Verfahren zur Herstellung einer Verbindung gemäss Anspruch 1 oder 2, **dadurch gekennzeichnet, dass** ein Perylentetracarbonsäurebisimid mit einem aromatischen Nitril in Gegenwart einer starken Base, zweckmässig in Gegenwart von Sauerstoff, umgesetzt wird.

**4.** Verfahren gemäss Anspruch 3, worin das Arylnitril, bezogen auf das Perylentetracarbonsäurebisimid, äquimolekular oder im Überschuss eingesetzt wird, und die Reaktionstemperatur von 80 bis 300˚C, bevorzugt von 100 bis 200˚C, insbesondere zirka 160˚C beträgt.

**5.** Verfahren zur Herstellung einer Verbindung gemäss Anspruch 1 oder 2, **dadurch gekennzeichnet, dass** ein Perylentetracarbonsäurebisimid der Formel (X'), worin als einzige Abweichung zur Formel (X) $Q_4$, $Q_4$ und $Q_6$, oder $Q_4$ und $Q_7$ Arylamido-Gruppen bedeuten, worin Aryl aus Gruppen $R_3$ oder $R_8$ ausgewählt ist, in Gegenwart einer starken Base, zweckmässig mit Natriumamid, umgesetzt wird.

**6.** Verfahren gemäss Anspruch 3, 4 oder 5, worin die Umsetzung in Gegenwart eines inerten Lösungsmittels stattfindet.

**7.** Verfahren zur Herstellung einer Verbindung gemäss Anspruch 1 oder 2, **dadurch gekennzeichnet, dass** eine Verbindung gemäss Anspruch 1 oder 2, worin $R_4$, $R_8$ oder $R_4$ und $R_8$ gleich H sind, an mindestens einem $R_4$ oder $R_8$ alkyliert, bevorzugt methyliert werden, bevorzugt in einem dipolaren aprotischen Lösungsmittel.

**8.** Zusammensetzung, enthaltend einer Verbindung gemäss Anspruch 1 oder 2 und eine schwache Base, bevorzugt ein primäres, sekundäres oder tertiäres Amin.

**9.** Verfahren zur Erhöhung des Stokes-Shifts einer Verbindung gemäss Anspruch 1 oder 2, bevorzugt durch den ESPT-Mechanismus, **dadurch gekennzeichnet, dass** eine schwache Base, bevorzugt ein primäres, sekundäres oder tertiäres Amin, der Verbindung gemäss Anspruch 1 oder 2 zugesetzt wird.

**10.** Verwendung einer Verbindung gemäss Anspruch 1 oder 2 als Farbmittel, bevorzugt als Pigment, besonders bevorzugt in Leim- und verwandten Farben, Papierfarben, Druckfarben, Tinten, Tuschen, Anstrichfarben oder Lacken, als Rheologieverbesserer, als Fluoreszenzfarbmittel, in optischen Aufzeichnungsmaterialien, in OLEDS (organischen Leuchtdioden), in photovoltaischen Zellen, zur Massenfärbung von Polymeren, zur Färbung von Naturstoffen, als Beizenfarbmittel, in elektrophotographischen Systemen, für Sicherheits- und Erkennungs-Markierungen, als Komponente von Farbmittelzusammensetzungen, in nichtlinearen optischen Elementen, in Lasern, zur Frequenzumwandlung von Licht, in Anzeigeelementen, als Ausgangsmaterial für supraleitende organische Materialien, für Feststoff-Fluoreszenz-Markierungen, für dekorative oder künstlerische Zwecke, oder als Tracer in der Biochemie, Medizin, Technik oder Naturwissenschaft.

**11.** Verwendung gemäss Anspruch 10, worin die Verbindung gemäss Anspruch 1 oder 2 in einem Fluoreszenz-Solarkollektor, in einem Display, in einer Kaltlichtquelle, in einem Chemilumineszenz-Leuchtstab, in einer integrierten Halbleiterschaltung, in einem Lumineszenznachweisverfahren oder in einem Photoleiter verwendet wird.

**12.** Verfahren zur Anregung von Fluoreszenz im Bereich von 500 bis 1000 nm, **dadurch gekennzeichnet, dass** eine Verbindung gemäss Anspruch 1 oder 2 mit einer elektromagnetischen Strahlung der Wellenlänge von 250 bis 600 nm, bevorzugt sichtbares Licht der Wellenlänge von 400 bis 600 nm, bestrahlt wird.

**13.** Verfahren gemäss Anspruch 12, worin die Fluoreszenz zur Erzeugung von Strom oder Wärme, oder zur Vollziehung einer chemischen Reaktion verwendet wird.

**14.** Verfahren zur Detektion von Fluoreszenz im Bereich von 500 bis 1000 nm, **dadurch gekennzeichnet, dass** die Fluoreszenz durch Bestrahlung einer Verbindung gemäss Anspruch 1 oder 2 mit einer elektromagnetischen Strahlung der Wellenlänge von 250 bis 600 nm, bevorzugt sichtbares Licht der Wellenlänge von 400 bis 600 nm, angeregt wird.

**15.** Verfahren gemäss Anspruch 14, worin die detektierte Fluoreszenz aufgefangen wird und in ein analoges oder digitales Signal oder in Energie umgewandelt wird.

## Claims

**1.** A compound of the formula

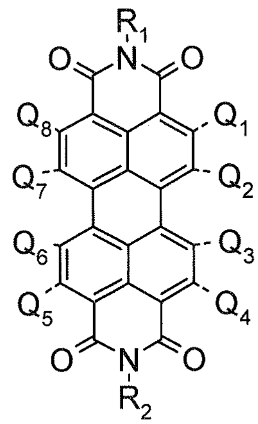

(X)

in which one pair $Q_1$ and $Q_2$ or one pair $Q_2$ and $Q_3$, and optionally from 0 to 3 pairs $Q_3$ and $Q_4$, $Q_5$ and $Q_6$, $Q_6$ and $Q_7$ and/or $Q_7$ and $Q_8$, in each case in pairs, together form a heterocyclic ring

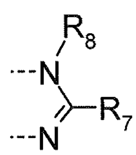

or

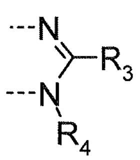

,

the remaining $Q_1$, $Q_4$, $Q_5$ and $Q_8$ which do not form a heterocyclic ring are each hydrogen, and the remaining $Q_3$, $Q_6$ and $Q_7$ which do not form a heterocyclic ring are each independently $R_5$ or $R_6$,
preferably

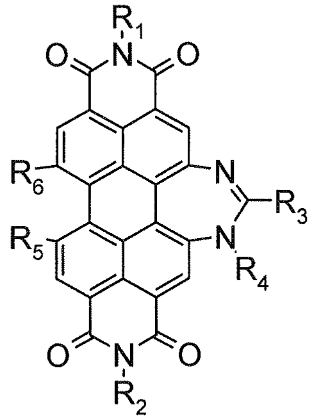

(5),

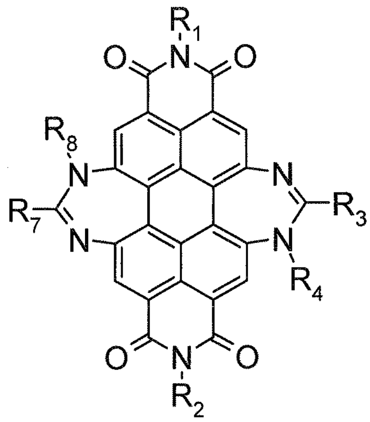

(6),

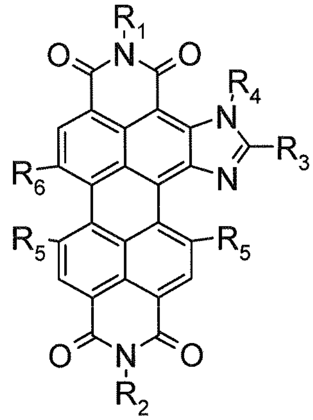

(7),

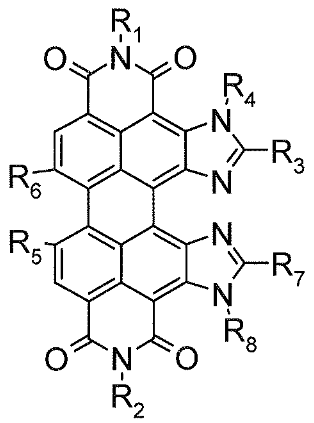

(8),

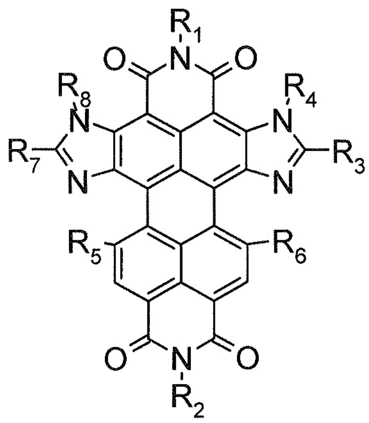

(9),

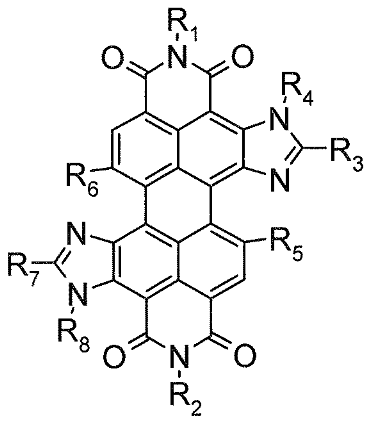

(10),

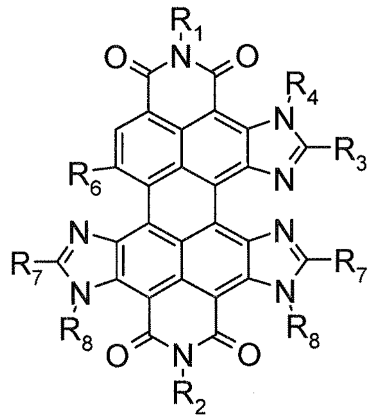

(11),

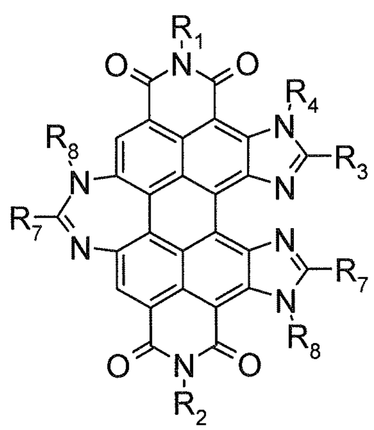

(12) or

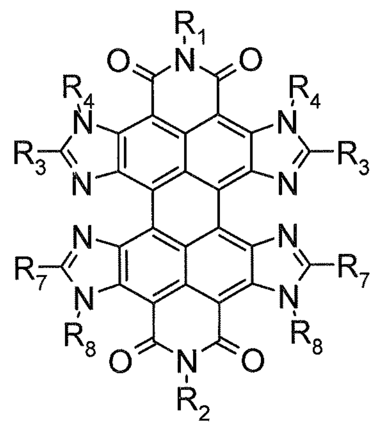

(13)

in which

$R_1$ to $R_8$ are each independently H, F, Cl, Br, I or {linear C1-C37alkyl }$(-R_9)_m$, m is from 0 to 12,

$R_9$, and in the case of multiple $R_9$ each $R_9$ independently of all other $R_9$, is H, F, Cl, Br, I, CN or { linear $C_1$-$C_{18}$alkyl} $(-R_{10})_n$, n is from 0 to 6,

$R_{10}$, and in the case of multiple $R_{10}$ each $R_{10}$ independently of all other $R_{10}$, is H, F, Cl, Br, I, CN or { linear $C_1$-$C_{18}$alkyl } ,

where no or from 1 to 10 $-CH_2-$ units in each {linear $C_1$-$C_{37}$alkyl }, optionally independently of all other { linear $C_1$-$C_{37}$alkyl }, may be replaced by $R_{11}$, and/or no or from 1 to 6 $-CH_2-$ units in { linear $C_1$-$C_{18}$alkyl }, and in the case of multiple { linear $C_1$-$C_{18}$alkyl } in each { linear $C_1$-$C_{18}$alkyl } independently of all other { linear $C_1$-$C_{18}$alkyl }, may be replaced by $R_{12}$, and

$R_{11}$ and $R_{12}$ are each independently, and in the case of multiple $R_{11}$ and $R_{12}$ each $R_{11}$ or $R_{12}$ independently of all other $R_{11}$ and $R_{12}$, is -CO-, -O-, -S-, -Se-, -Te-, cis or trans -CH=CH-, cis or trans -N=CH-, -C≡C-, 1,2-, 1,3- or 1,4-phenylene, 2,3-, 2,4-, 2,5-, 2,6-, 3,4- or 3,5-pyridinediyl, 2,3-, 2,4-, 2,5- or 3,4-thiophenediyl, 1, 2-, 1, 3-, 1, 4-, 1, 5-, 1,6-, 1, 7-, 1, 8-, 2, 3-, 2,6- or 2,7-naphthylene, in which 0, 1 or 2 =CH- may be replaced by =N-, or 1,2-, 1, 3-, 1,4-, 1,5-, 1,6-, 1,7- , 1, 8-, 1, 9-, 1, 10-, 2, 3-, 2,6-, 2, 7-, 2, 9-, 2,10- or 9,10-disubstituted anthracylene in which 0, 1 or 2 =CH- may be replaced by =N-,

or any two $R_9$, $R_{10}$, $R_{11}$ and/or $R_{12}$ substituents together form, in a single pair or multiple pairs, a direct bond, so as to form rings, preferably cyclohexane or benzene rings,

where $R_4$ may optionally be bonded by a direct bond to $R_4$ of a second molecule, and/or $R_8$ may be bonded by a direct bond to $R_8$ of a second molecule, so as to form dimers, trimers, tetramers or higher oligomers.

**2.** A compound according to claim 1, in which { linear $C_1$-$C_{37}$alkyl} $(-R_9)_m$ is an unsubstituted or substituted hydrocarbon radical which comprises from 1 to 60, preferably from 1 to 37 and more preferably from 1 to 18 carbon atoms, where this hydrocarbon radical in the case of $R_3$ and/or $R_8$ preferably forms a phenyl radical by trisubstitution of $-CH_2-$ with -CH=CH- and a direct bond between 2 $R_9$ substituents.

3. A process for preparing a compound according to claim 1 or 2, which comprises reacting a perylenetetracarboximide with an aromatic nitrile in the presence of a strong base, appropriately in the presence of oxygen.

4. The process according to claim 3, in which the aryl nitrile, based on the perylenetetracarboximide, is used in an equimolecular amount or in excess, and the reaction temperature is from 80 to 300˚C, preferably from 100 to 200˚C, especially approximately 160˚C.

5. A process for preparing a compound according to claim 1 or 2, which comprises reacting a perylenetetracarboximide of the formula (X') in which, as the sole difference from the formula (X), $Q_4$, $Q_4$ and $Q_6$, or $Q_4$ and $Q_7$ are each arylamido groups in which aryl is selected from $R_3$ or $R_8$ groups, in the presence of a strong base, appropriately with sodium amide.

6. The process according to claim 3, 4 or 5, in which the reaction takes place in the presence of an inert solvent.

7. A process for preparing a compound according to claim 1 or 2, wherein a compound according to claim 1 or 2 in which $R_4$, $R_8$ or $R_4$ and $R_8$ are each H is alkylated, preferably methylated, at least one $R_4$ or $R_8$, preferably in a dipolar aprotic solvent.

8. A composition comprising a compound according to claim 1 or 2 and a weak base, preferably a primary, secondary or tertiary amine.

9. A process for increasing the Stokes shift of a compound according to claim 1 or 2, preferably by the ESPT mechanism, which comprises adding a weak base, preferably a primary, secondary or tertiary amine, to the compound according to claim 1 or 2.

10. The use of a compound according to claim 1 or 2 as a colorant, preferably as a pigment, more preferably in distempers and related colors, paper inks, printing inks, solventborne and waterborne inks, paints or coating materials, as rheology improvers, as fluorescent colorants, in optical recording materials, in OLEDs (organic light-emitting diodes), in photovoltaic cells, for bulk coloring of polymers, for coloring of natural substances, as a mordant dye, in electrophotographic systems, for security and identification markings, as a component of colorant compositions, in nonlinear optical elements, in lasers, for frequency conversion of light, in display elements, as a starting material for superconductive organic materials, for solid fluorescent markings, for decorative or artistic purposes, or as a tracer in biochemistry, medicine, technology or natural science.

11. The use according to claim 10, in which the compound according to claim 1 or 2 is used in a fluorescent solar collector, in a display, in a cold light source, in a chemiluminescent glow stick, in an integrated semiconductor circuit, in a luminescence detection method or in a photoconductor.

12. A process for inducing fluorescence in the range from 500 to 1000 nm, which comprises irradiating a compound according to claim 1 or 2 with electromagnetic radiation of wavelength from 250 to 600 nm, preferably visible light of wavelength from 400 to 600 nm.

13. The process according to claim 12, in which the fluorescence is used to generate power or heat, or to conduct a chemical reaction.

14. A process for detecting fluorescence in the range from 500 to 1000 nm, which comprises inducing the fluorescence by irradiating a compound according to claim 1 or 2 with electromagnetic radiation of wavelength from 250 to 600 nm, preferably visible light of wavelength from 400 to 600 nm.

15. The process according to claim 14, in which the detected fluorescence is collected and converted to an analog or digital signal or to energy.

**Revendications**

1. Composés de la formule

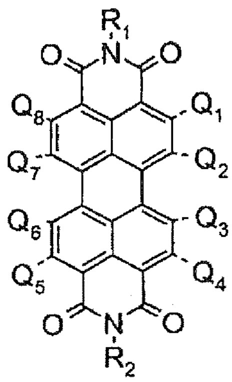 (X),

dans lesquels une paire $Q_1$ et $Q_2$ ou une paire $Q_2$ et $Q_3$, ainsi qu'éventuellement de 0 à 3 paires $Q_3$ et $Q_4$, $Q_5$ et $Q_6$, $Q_6$ et $Q_7$ et/ou $Q_7$ et $Q_8$ forment chacune ensemble par paires un anneau hétérocyclique

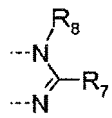

ou

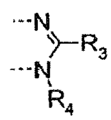 ,

les $Q_1$, $Q_4$, $Q_5$ et $Q_8$ restants, qui ne forment pas d'anneau hétérocyclique, sont l'hydrogène, et les $Q_3$, $Q_6$ et $Q_7$ restants, qui ne forment pas d'anneau hétérocyclique sont $R_5$ et $R_6$ indépendamment l'un de l'autre, de préférence

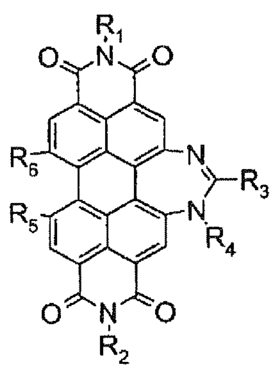 (5), 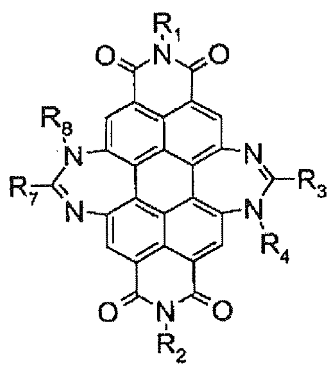 (6),

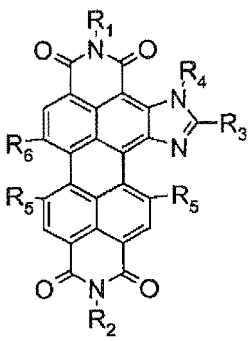 (7), 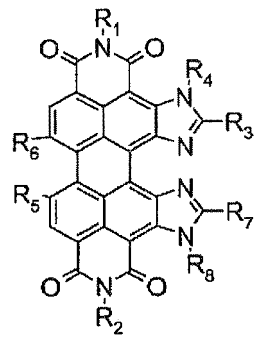 (8), 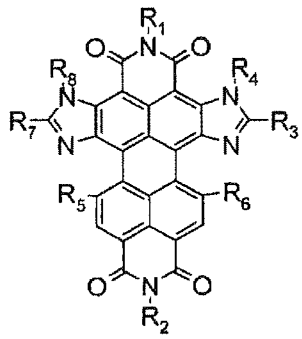 (9),

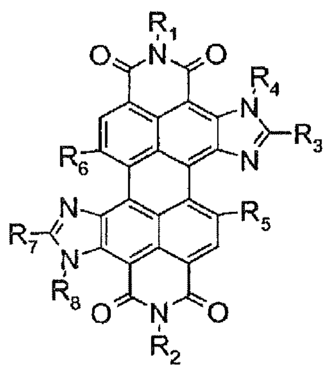 (10), 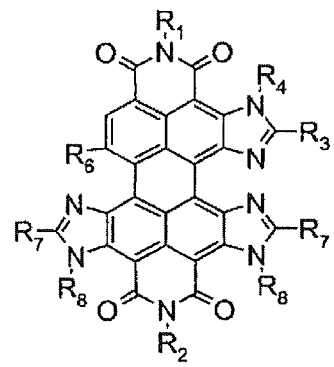 (11),

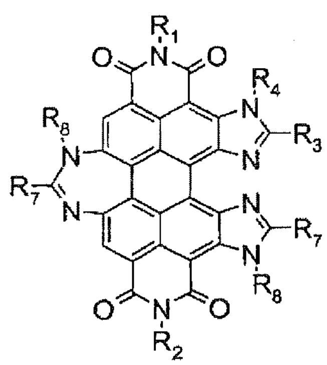 (12) ou 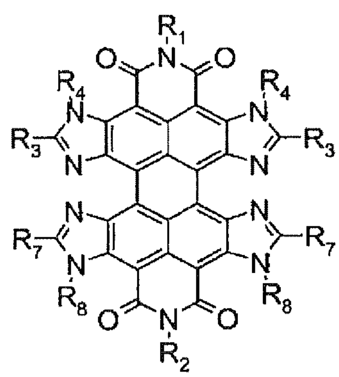 (13),

dans lesquels

$R_1$ à $R_8$ sont, indépendamment l'un de l'autre, H, F, Cl, Br, I ou {alkyle linéaire en $C_1$-$C_{37}$} $(-R_9)_m$, m étant un nombre de 0 à 12,

$R_9$, et s'il y a plusieurs $R_9$, chaque $R_9$ est, indépendamment de tous les autres $R_9$, H, F, Cl, Br, I, CN ou {alkyle linéaire en $C_1$-$C_{18}$ } $(-R_{10})_n$, n étant un nombre de 0 à 6,

$R_{10}$, et s'il y a plusieurs $R_{10}$, chaque $R_{10}$ est, indépendamment de tous les autres $R_{10}$, H, F, Cl, Br, I, CN ou {alkyle linéaire en $C_1$-$C_{18}$},

dans lesquelles, dans chaque {alkyle linéaire en $C_1$-$C_{37}$}, le cas échéant indépendamment de tous les autres {alkyles linéaires en $C_1$-$C_{37}$}, aucune ou de 1 à 10 unités $-CH_2-$ peut/peuvent être remplacée(s) par $R_{11}$, et/ou dans {alkyle linéaire en $C_1$-$C_{18}$}, et s'il y a plusieurs {alkyles linéaires en $C_1$-$C_{18}$}, dans chaque {alkyle linéaire en $C_1$-$C_{18}$} indépendamment de tous les autres {alkyles linéaires en $C_1$-$C_{18}$}, aucune ou de 1 à 6 unités $-CH_2-$ peut/peuvent être remplacée(s) par $R_{12}$,

$R_{11}$ et $R_{12}$ indépendamment l' un de l' autre, et s'il y a plusieurs $R_{11}$ et $R_{12}$, chaque $R_{11}$ ou $R_{12}$ est, indépendamment de tous les autres $R_{11}$ et $R_{12}$, -CO-, -O-, -S-, -Se-, -Te-, cis- ou trans- -CH=CH-, cis- ou trans--N=CH-, -C≡C-, 1,2-, 1,3- ou 1,4-phénylène, 2,3-, 2,4-, 2,5-, 2,6-, 3,4- ou 3,5-pyridinediyle, 2,3-, 2,4-, 2,5- ou 3,4-thiophènediyle, 1,2-,

1,3-, 1,4-, 1,5-, 1,6-, 1,7-, 1,8-, 2,3-, 2,6- ou 2,7-naphtylène, dans lesquels 0, 1 ou 2 =CH- peut/peuvent être remplacé (s) par =N-, ou 1,2-, 1,3-, 1,4-, 1,5-, 1,6-, 1,7-, 1,8-, 1,9-, 1,10-, 2,3-, 2,6-, 2,7-, 2,9-, 2,10- ou 9,10-anthracylène disubstitué, dans lesquels 0, 1 ou 2 =CH- peut/peuvent être remplacé(s) par =N-,
ou deux substituants quelconques $R_9$, $R_{10}$, $R_{11}$ et/ou $R_{12}$ forment ensemble par paire une liaison directe simple ou multiple, de façon à produire des anneaux, de préférence des anneaux de cyclohexane ou de benzène, dans lesquelles, de manière facultative, $R_4$ peut être lié par une liaison directe avec $R_4$ d'une deuxième molécule, et/ou $R_8$ peut être lié par une liaison directe avec $R_8$ d'une deuxième molécule, de façon à former des dimères, des trimères, des tétramères ou des oligomères plus élevés.

**2.** Composé selon la revendication 1, dans lequel {alkyle linéaire en $C_1C_{37}$ } $(-R_9)_m$ est un radical hydrocarboné non substitué ou substitué, qui contient de 1 à 60, de préférence de 1 à 37, et de préférence encore de 1 à 18 atomes de carbone, dans lequel ce radical hydrocarboné forme, pour $R_3$ et/ou $R_8$, un radical phényle de préférence par triple substitution de $-CH_2-$ avec -CH=CH- et une liaison directe entre 2 substituants $R_9$.

**3.** Procédé pour la fabrication d'un composé selon la revendication 1 ou 2, **caractérisé en ce que** l'on convertit un bisimide de l'acide pérylène tétracarboxylique avec un nitrile aromatique en présence d'une base forte, avantageusement en présence d'oxygène.

**4.** Procédé selon la revendication 3, dans lequel l'arylnitrile, rapporté au bisimide de l'acide pérylène tétracarboxylique, est utilisé de façon équimoléculaire ou en excès, et la température de réaction vaut de 80 à 300°C, de préférence de 100 à 200°C, en particulier environ 160°C.

**5.** Procédé pour la fabrication d'un composé selon la revendication 1 ou 2, **caractérisé en ce que** l'on convertit un bisimide de l'acide pérylène tétracarboxylique de formule (X'), dans laquelle le seul changement par rapport à la formule (X) est que $Q_4$, $Q_4$ et $Q_6$, ou $Q_4$ et $Q_7$ sont des groupes arylamido, dans lesquels l'aryle est choisi dans des groupes $R_3$ ou $R_8$, en présence d'une base forte, avantageusement avec de l'amidure de sodium.

**6.** Procédé selon la revendication 3, 4 ou 5, dans lequel la conversion a lieu en présence d'un solvant inerte.

**7.** Procédé pour la fabrication d'un composé selon la revendication 1 ou 2, **caractérisé en ce qu'**un composé selon la revendication 1 ou 2, dans lequel $R_4$, $R_8$ ou $R_4$ et $R_8$ sont également H, est alkylé, de préférence méthylé, sur au moins un $R_4$ ou $R_8$, de préférence dans un solvant aprotique dipolaire.

**8.** Composition, contenant un composé selon la revendication 1 ou 2 et une base faible, de préférence une amine primaire, secondaire ou tertiaire.

**9.** Procédé pour augmenter la dérive de Stokes d'un composé selon la revendication 1 ou 2, de préférence par le mécanisme ESPT, **caractérisé en ce que** l'on ajoute au composé selon la revendication 1 ou 2 une base faible, de préférence une amine primaire, secondaire ou tertiaire.

**10.** Utilisation d'un composé selon la revendication 1 ou 2 comme colorant, de préférence comme pigment, de préférence encore dans des peintures à la colle et apparentées, des couleurs pour papier, des encres d'imprimerie, des encres, des encres de Chine, des peintures ou des laques, comme améliorants de rhéologie, comme colorants de fluorescence, dans des matériaux d'enregistrement optique, dans des OLEDS (diodes électroluminescentes organiques), dans des cellules photovoltaïques, pour la coloration dans la masse de polymères, pour la coloration de substances naturelles, comme colorants à mordant, dans des systèmes électrophotographiques, pour des marquages de sécurité et de reconnaissance, comme composants de compositions de colorants, dans des éléments optiques non linéaires, dans des lasers, pour la conversion de fréquence de la lumière, dans des éléments d'affichage, comme matière première pour des matériaux organiques supraconducteurs, pour des marquages par fluorescence de matières solides, pour des applications décoratives et artistiques, ou comme traceurs en biochimie, médecine, technique ou sciences naturelles.

**11.** Utilisation selon la revendication 10, dans laquelle le composé selon la revendication 1 ou 2 est utilisé dans un collecteur solaire à fluorescence, dans un écran d'affichage, dans une source de lumière froide, dans une barre lumineuse à chimiluminescence, dans un circuit semi-conducteur intégré, dans un procédé de détection à luminescence ou dans un photoconducteur.

**12.** Procédé pour l'excitation de la fluorescence dans la plage de 500 à 1000 nm, **caractérisé en ce qu'**un composé

selon la revendication 1 ou 2 est exposé à un rayonnement électromagnétique d'une longueur d'onde de 250 à 600 nm, de préférence de lumière visible d'une longueur d'onde de 400 à 600 nm.

**13.** Procédé selon la revendication 12, dans lequel la fluorescence est utilisée pour produire du courant ou de la chaleur, ou pour accomplir une réaction chimique.

**14.** Procédé pour la détection de la fluorescence dans la plage de 500 à 1000 nm, **caractérisé en ce que** la fluorescence est excitée par exposition d'un composé selon la revendication 1 ou 2 à un rayonnement électromagnétique d'une longueur d'onde de 250 à 600 nm, de préférence de lumière visible d'une longueur d'onde de 400 à 600 nm.

**15.** Procédé selon la revendication 14, dans lequel la fluorescence détectée est captée et transformée en un signal analogique ou numérique ou en énergie.

Abb. 1

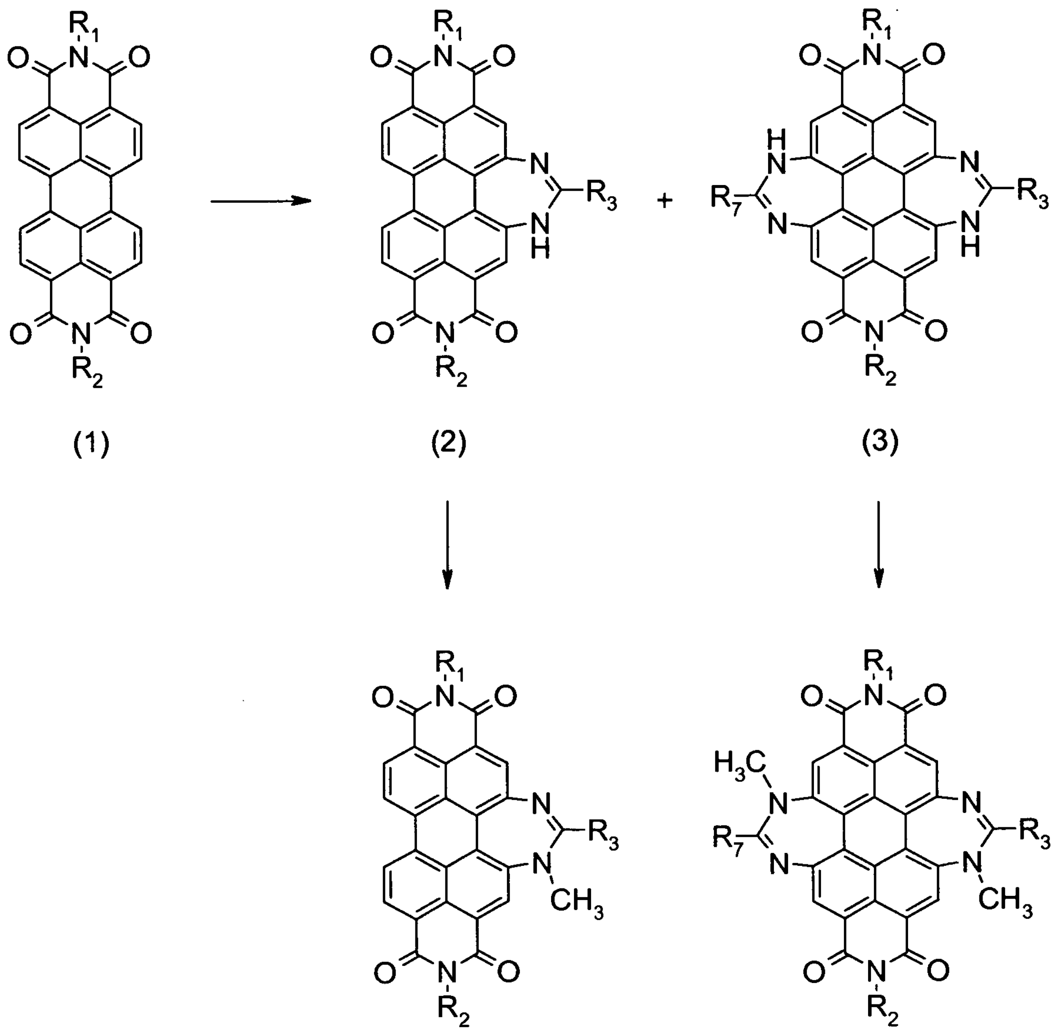

(1) (2) (3)

(4) (5)

| (1) | $R_1 = R_2$ |
|---|---|
| a | $CH(n\text{-}C_6H_{13})_2$ |
| b | $CH(iso\text{-}C_3H_7)_2$ |

| (2) - (5) | $R_1 = R_2$ | $R_3 = R_7$ |
|---|---|---|
| a | $CH(n\text{-}C_6H_{13})_2$ | $C_6H_5$ |
| b | $CH(iso\text{-}C_3H_7)_2$ | $C_6H_5$ |
| c | $CH(n\text{-}C_6H_{13})_2$ | 4-Br-$C_6H_4$ |
| d | $CH(n\text{-}C_6H_{13})_2$ | 4-$CH_3O$-$C_6H_4$ |
| e | $CH(n\text{-}C_6H_{13})_2$ | 2-Naphthyl |

Abb. 2

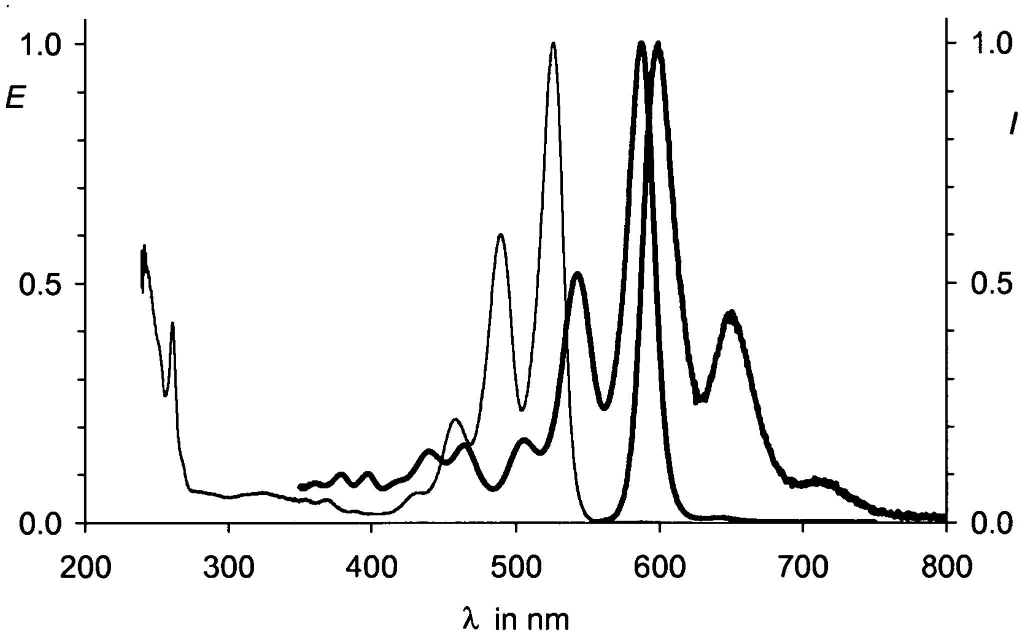
1.0
E
0.5
0.0
1.0
I
0.5
0.0
200
300
400
500
600
700
800
λ in nm

Abb. 3

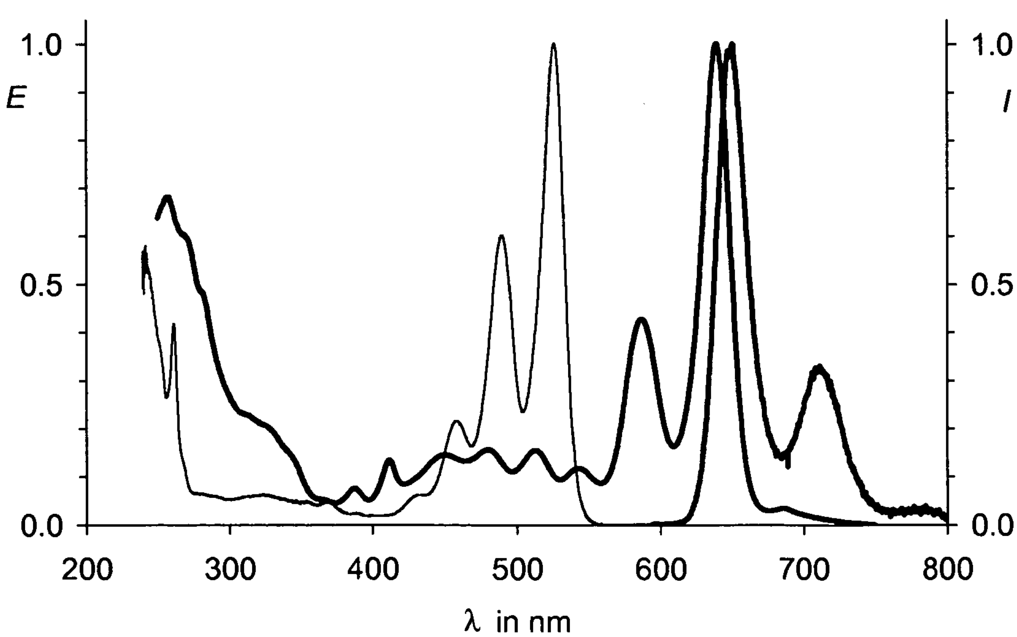
1.0
E
0.5
0.0
1.0
I
0.5
0.0
200
300
400
500
600
700
800
λ in nm

Abb. 4

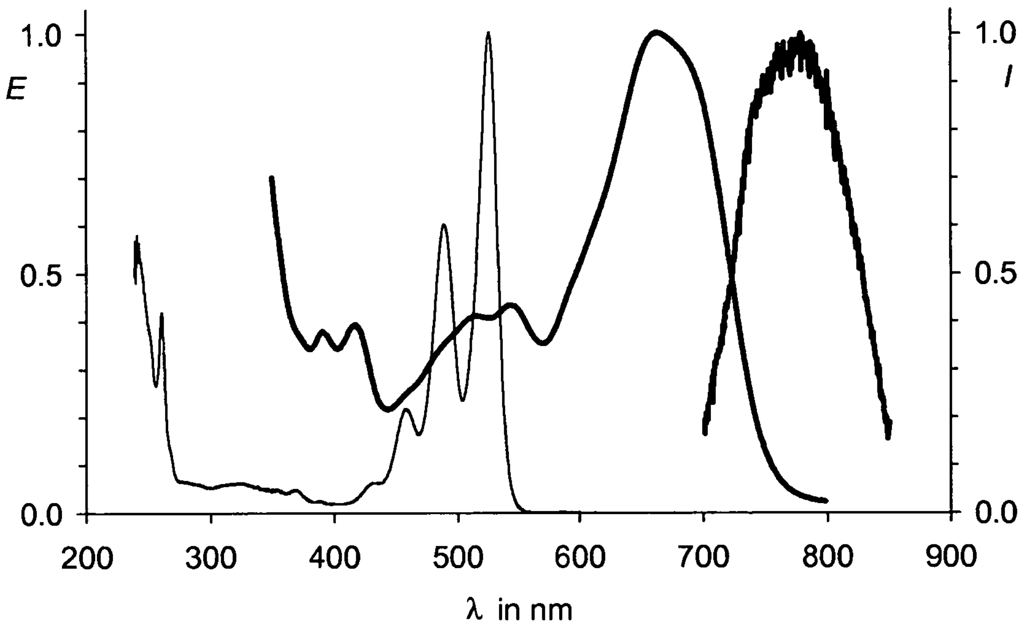
1.0
E
0.5
0.0
1.0
I
0.5
0.0
200
300
400
500
600
700
800
900
λ in nm

Abb. 5

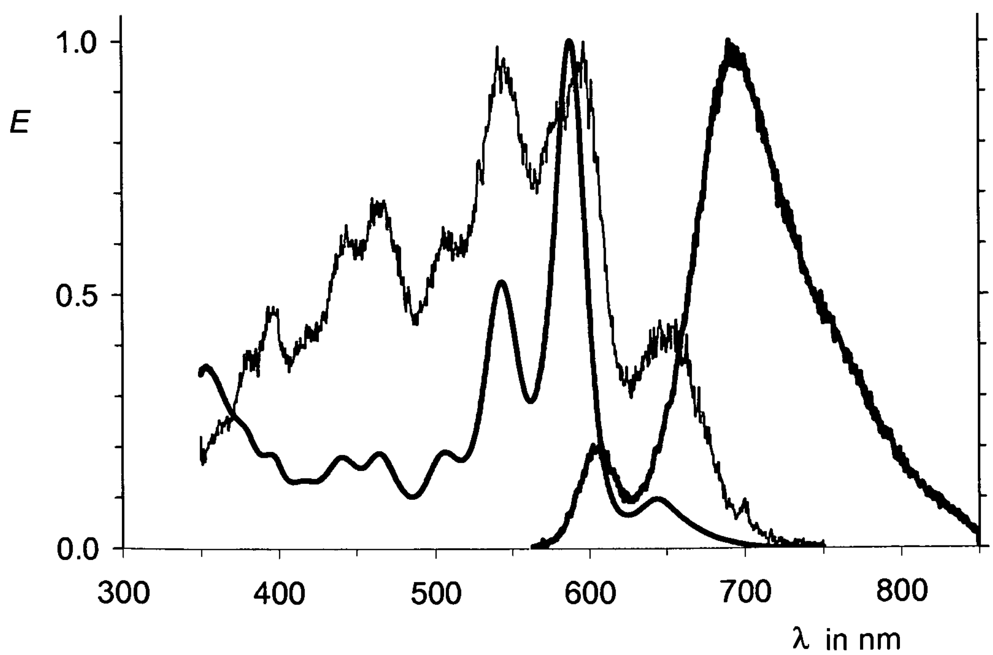
1.0
E
0.5
0.0
1.0
I
0.5
0.0
300
400
500
600
700
800
λ in nm

Abb. 6

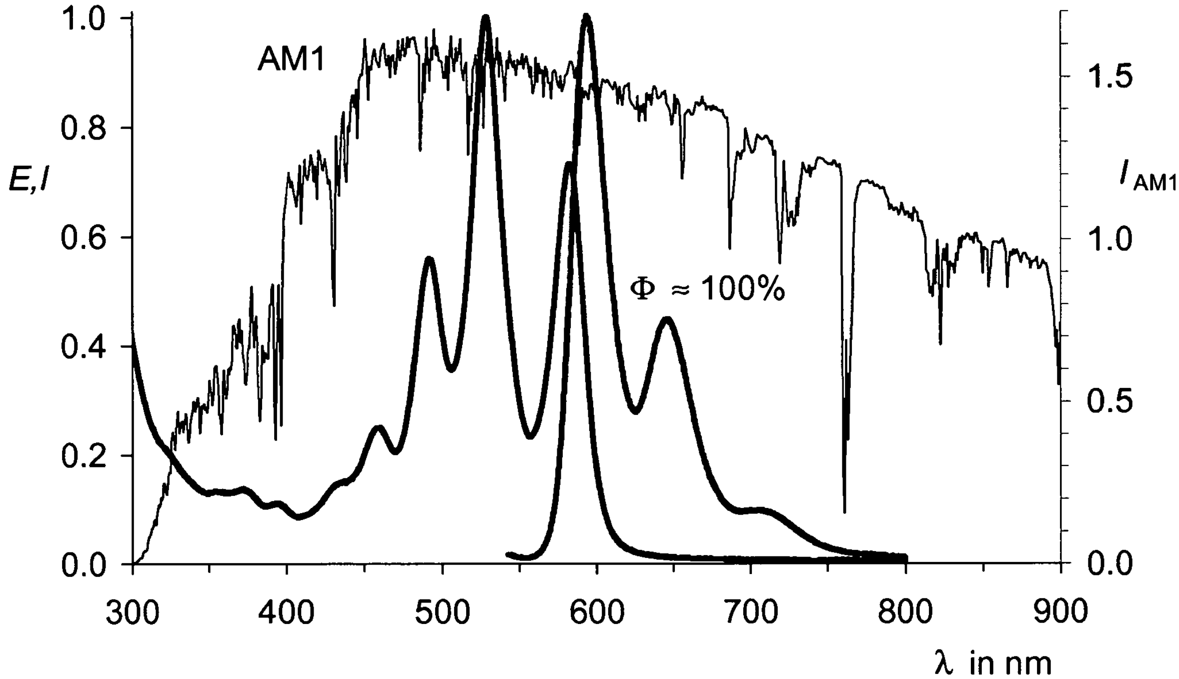
1.0
0.8
0.6
0.4
0.2
0.0
E,I
AM1
Φ ≈ 100%
1.5
1.0
0.5
0.0
$I_{AM1}$
300
400
500
600
700
800
900
λ in nm

Abb. 7

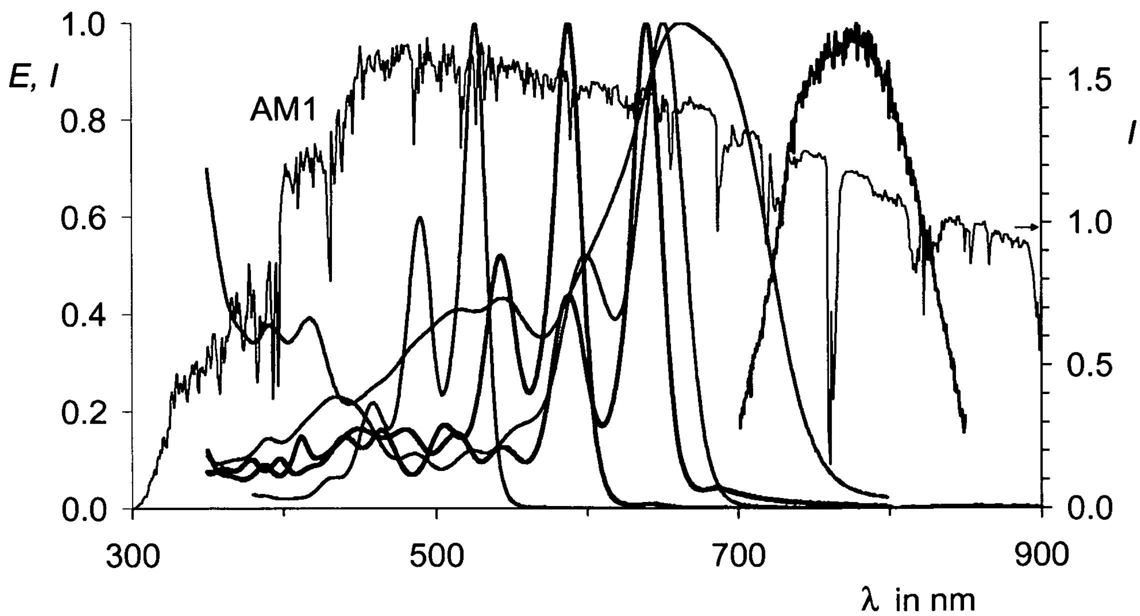
1.0
0.8
0.6
0.4
0.2
0.0
E, I
AM1
1.5
1.0
0.5
0.0
I
300
500
700
900
λ in nm

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- *Nachr. Chem. Tech. Lab.,* 1980, vol. 28, 716-718
- *Helv. Chim. Acta,* 2005, vol. 88, 1309-1343
- *Heterocycles,* 1995, vol. 40, 477-500
- *J. Prakt. Chem.,* 1997, vol. 339, 597-602
- *Liebigs Ann. Chem.,* 1995, 481-486
- *Chem. Ber.,* 1983, vol. 116, 3524-3528
- *Pure Appl. Chem.,* 1996, vol. 68, 1441-1442
- *Forschungsber. - Bundesminist. Forsch. Technol., Technol. Forsch. Entwickl.,* 1984, 84-164
- *Chem. Abstr.,* 1985, vol. 102, 150903
- *Vestn. Khar'kov. Politekh. Inst.,* 1969, vol. 41, 21-26
- *Chem. Abstr.,* 1971, vol. 75, 7375
- *Tetrahedron Lett.,* 1999, vol. 40, 7047-7050
- *Eur. J. Org. Chem.,* 2000, 365-380
- *Prakt. Chem.,* 1926, vol. 112, 1-36
- *Dyes Pigm.,* 2003, vol. 59, 109-116
- *Chem. Ber.,* 1988, vol. 121, 225-230
- *Angew. Chem.,* 2006, vol. 118, 4555-4561
- *Angew. Chem. Int. Ed. Engl.,* 2006, vol. 45, 4444-4447
- *Angew. Chem.,* 2007, vol. 119, 8510-8514